(19) Europäisches Patentamt
European Patent Office
Office européen des brevets

(11) EP 3 456 829 A1

# (12) EUROPEAN PATENT APPLICATION

(43) Date of publication:
**20.03.2019 Bulletin 2019/12**

(51) Int Cl.:
***C12N 15/82*** *(2006.01)*

(21) Application number: **17191524.2**

(22) Date of filing: **18.09.2017**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **Nomad Bioscience GmbH**
**80333 München (DE)**

(72) Inventors:
- **TORTI, Stefano**
  **06114 Halle (Saale) (DE)**
- **SCHLESIER, René**
  **06110 Halle (Saale) (DE)**
- **GIRITCH, Anatoli**
  **06108 Halle (Saale) (DE)**
- **GLEBA, Yuri**
  **10117 Berlin (DE)**

(74) Representative: **Blodig, Wolfgang**
**Wächtershäuser & Hartz**
**Patentanwaltspartnerschaft mbB**
**Weinstrasse 8**
**80333 München (DE)**

## (54) METHOD OF IMPROVING POTEXVIRAL VECTOR STABILITY

(57) The invention provides a method of producing a potexviral vector for expressing a protein of interest in a plant, comprising
producing a second heterologous nucleic acid comprising a second ORF encoding said protein and having, in the second ORF, an increased GC-content compared to a first ORF encoding said protein in a first heterologous nucleic acid, and
providing said potexviral vector comprising the following segments: (i) a nucleic acid sequence segment encoding a potexviral RNA-dependent RNA polymerase, (ii) a nucleic acid sequence comprising or encoding a potexviral triple-gene block, and (iii) said second heterologous nucleic acid or a portion thereof comprising said second ORF.

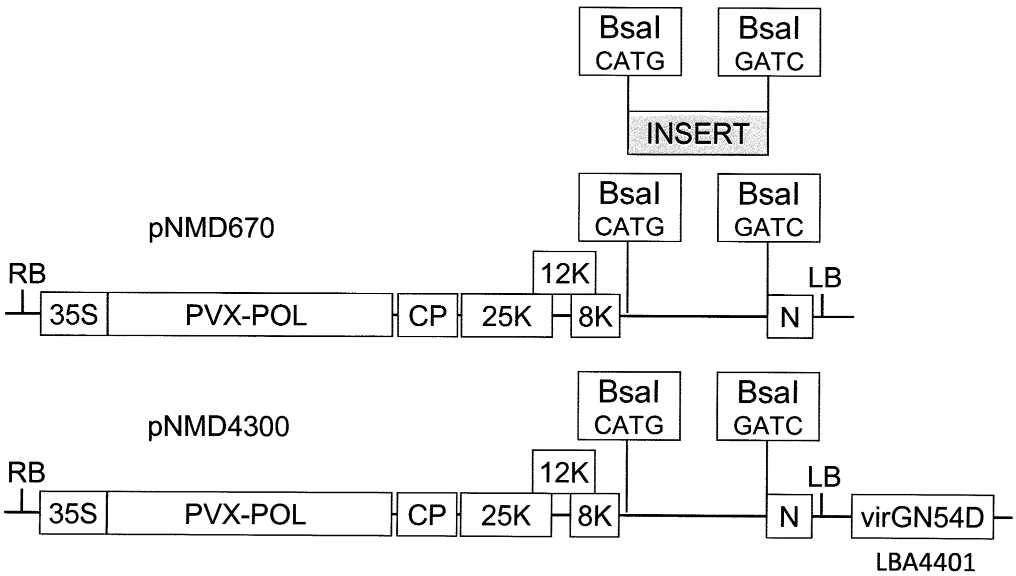


Fig. 1



Printed by Jouve, 75001 PARIS (FR)

## Description

### Field of the Invention

The present invention relates to a method of producing a potexviral vector for expressing a protein of interest in a plant. The invention also relates to methods of improving the capability for long-distance movement in a plant of a potexviral replicon. The invention also provides a process of expressing a protein of interest in a plant or in plant tissue. Further, nucleic acids for the methods and processes are provided.

### Background of the invention

High-yield expression of heterologous proteins in plants can be achieved using viral vectors. Viral vector systems were predominantly developed for transient expression followed by infection (Donson et al., 1991, Proc Natl Acad Sci U S A, 88:7204-7208; Chapman, Kavanagh & Baulcombe, 1992, Plant J., 2:549-557) or transfection (Marillonnet et al., 2005, Nat Biotechnol., 23:718-723; Santi et al., 2006, Proc Natl Acad Sci U S A. 103:861-866; WO2005/049839) of a plant host. The best-established and commercially viable systems are based on plus-sense single-stranded RNA viruses, preferably on Tobacco Mosaic Virus (TMV)-derived vectors. Another group of RNA virus-based vectors derived from potexvirus such as PVX (Potato Virus X) can also provide high yield of recombinant proteins (Chapman, Kavanagh & Baulcombe, 1992, Plant J., 2:549-557; Baulcombe, Chapman & Santa Cruz, 1995, Plant J., 7:1045-1053; Zhou et al., 2006, Appl. Microbiol. Biotechnol., 72 (4): 756-762; Zelada et al., 2006, Tuberculosis, 86:263-267). Also potexviruses are plant RNA viruses with a plus-sense single-stranded genome.

In the first generation of systemic viral vectors, a large proportion of plant resources was wasted for the production of viral coat protein that is necessary for systemic movement of a viral replicon. For TMV-derived vectors this problem was solved by removing the coat protein gene and by using agro-infiltration for efficient systemic delivery of replicons, thus significantly boosting the yield of recombinant proteins of interest (WO2005/049839; Marillonnet et al., (2005), Nat. Biotechnol., 23:718-723). However, unlike for TMV-derived replicons, for potexvirus-derived replicons viral coat protein is preferred not only for systemic, but also for short distance (cell-to-cell) movement. WO 2008/028661 describes a way to increase the expression yield of a protein of interest expressed in a plant or in plant tissue from a potexviral vector by a vector design wherein the sequences as defined in item (ii) of claim 1 are positioned after (downstream in 5 to 3' direction) the RNA-dependent RNA polymerase coding sequence (RdRp or RdRP) of item (i) and precede said heterologous nucleic acid of item (iii). In the special case of potexviral vectors, this vector design leads to a cell-to-cell movement capability of the RNA replicon and, at the same time, to higher expression levels of the heterologous nucleic acid compared to potexviral vectors where a heterologous nucleic acid was placed upstream of the potexviral coat protein gene.

Viral vectors used for expressing a foreign gene in plants typically contain, apart from the ORF encoding the foreign protein to be expressed, remaining viral ORFs that allow the vector to replicate and to spread in plant tissue or entire plants, such as by cell to cell movement and/or long distance movement in a plant. When expressing a sequence of interest in a plant, the replicated and spreading viral vector is desired to be stable such as not to change the nucleic acid sequence of interest to be expressed.

### Summary of the Invention

The inventors have observed that when low leaves of young plants that were infiltrated with an agrobacterial suspension carrying vectors encoding potexviral replicons containing an ORF to be expressed, spread over plant organs, but are sometimes not stable and lose the nucleic acid sequence of interest over time. On the other hand, some potexviral vectors containing a heterologous nucleic acid sequence such as that encoding AtFT or sGFP are unusually stable.

It is therefore an object of the present invention to provide a potexviral vector for expressing a heterologous nucleic acid or heterologous protein of interest, that is stable, notably in long-distance movement of the vector in plants.

The inventors have studied this problem in detail to find a solution. Accordingly, the present invention that provides:

(1) A method of producing a potexviral vector for expressing a protein of interest in a plant, comprising
producing a second heterologous nucleic acid sequence comprising a second ORF encoding said protein of interest and having, in the second ORF, an increased GC-content compared to a first ORF encoding said protein in a first heterologous nucleic acid sequence,and
providing said potexviral vector comprising the following segments: (i) a nucleic acid sequence encoding a potexviral RNA-dependent RNA polymerase, (ii) a nucleic acid sequence comprising or encoding a potexviral triple-gene block, and (iii) said second heterologous nucleic acid sequence or a portion thereof comprising said second ORF.

(2) A method of improving the capability for long-distance movement in a plant of a potexviral replicon encoding a protein of interest to be expressed in said plant, comprising
producing a second heterologous nucleic acid sequence comprising a second ORF encoding said protein of interest and having, in the second ORF, an increased GC-content compared to a first ORF encoding said protein in a first heterologous nucleic acid sequence,and
providing said potexviral replicon, or a potexviral vector comprising or encoding said potexviral replicon, said potexviral replicon comprising the following segments: (i) a nucleic acid sequence encoding a potexviral RNA-dependent RNA polymerase, (ii) a nucleic acid sequence comprising a potexviral triple-gene block, and (iii) said second heterologous nucleic acid sequence or a portion thereof comprising said second ORF.
(3) The method according to (1) or (2), wherein said step of providing a potexviral vector or potexviral replicon comprises inserting said second heterologous nucleic acid sequence, or a portion thereof comprising said second ORF, into a nucleic acid comprising (i) a nucleic acid sequence encoding a potexviral RNA-dependent RNA polymerase and (ii) a nucleic acid sequence encoding a potexviral triple-gene block to produce the potexviral vector or the potexviral replicon comprising the second heterologous nucleic acid sequence or a portion thereof comprising said second ORF.
(4) A process of expressing a protein of interest in a plant or in plant tissue, comprising producing a potexviral vector according to the method of (1) and providing the produced potexviral vector to at least a part of said plant.
(5) The method or process according to any one of (1) to (4), wherein said plant is selected from *Nicotiana* species such as *Nicotiana benthamiana* and *Nicotiana tabacum,* tomato, potato, pepper, eggplant, soybean, *Petunia hybrida, Brassica napus, Brassica campestris, Brassica juncea,* cress, arugula, mustard, strawberry, spinach, *Chenopodium capitatum,* alfalfa, lettuce, sunflower, potato, cucumber, corn, wheat, and rice.
(6) The method or process according to any one of (1) to (5), wherein said (ii) nucleic acid sequence comprising or encoding a potexviral triple-gene block said further comprises a nucleic acid sequence encoding a potexviral coat protein or a nucleic acid sequence encoding a tobamoviral movement protein.
(7) A method of improving the capability for long-distance movement in a plant of a potexviral replicon encoding a protein of interest to be expressed in said plant, comprising
increasing the GC-content of a first ORF encoding said protein in a first heterologous nucleic acid sequence, thereby obtaining a second heterologous nucleic acid sequence comprising a second ORF, said second ORF encoding said protein and having an increased GC-content, and
inserting said second heterologous nucleic acid sequence, or a portion thereof containing said second ORF, into a nucleic acid comprising (i) a nucleic acid sequence encoding a potexviral RNA-dependent RNA polymerase and (ii) a nucleic acid sequence comprising or encoding a potexviral triple-gene block to produce a potexviral vector comprising or encoding said potexviral replicon, said potexviral vector comprising the second heterologous nucleic acid sequence or a portion thereof comprising said second ORF.
(8) A potexviral vector obtained or obtainable by the method of (1).
(9) A nucleic acid comprising the following segments: (i) a nucleic acid sequence encoding a potexviral RNA-dependent RNA polymerase, (ii) nucleic acid sequence comprising or encoding a potexviral triple-gene block, and (iii) a heterologous nucleic acid sequence comprising an ORF encoding a protein of interest, wherein
said ORF consists of at least 200 and at most 400 nucleotides and has a GC-content of at least 50%; or
said ORF consists of at least 401 and at most 800 nucleotides has a GC-content of at least 55%; and/or
said ORF consists of at least 801 nucleotides and has a GC-content of at least 58%.
(10) A nucleic acid comprising the following segments: (i) a nucleic acid sequence encoding a potexviral RNA-dependent RNA polymerase, (ii) nucleic acid sequence comprising or encoding a potexviral triple-gene block, and (iii) a heterologous nucleic acid sequence comprising an ORF encoding a protein of interest, wherein
said ORF consists of at least 100 and at most 500 nucleotides and has a GC-content of at least 50%; or
said ORF consists of at least 501 and at most 1000 nucleotides has a GC-content of at least 55%; and/or
said ORF consists of at least 1001 nucleotides and has a GC-content of at least 58%.
(11) The nucleic acid according to (9) or (10), said nucleic acid further comprising a nucleic acid sequence encoding a potexviral coat protein or a nucleic acid sequence encoding a tobamoviral movement protein.
(12) A combination or kit comprising a first and a second nucleic acid, said first nucleic acid comprising segments (i) and (ii) as defined in (9), (10) or (11), said second nucleic acid comprising segment (iii) as defined in (9) or (10).
(13) The combination or kit according to (12), wherein said first nucleic acid has, downstream of segment (ii) a first site-specific recombination site recognizable by a site-specific recombinase, and said second nucleic acid has, upstream of segment (iii), a second site-specific recombination site recognizable by said site-specific recombinase for allowing site-specific recombination between said first and said second site-specific recombination site and formation of a nucleic acid according to (9), (10), or (11), or a potexviral vector according to (8).
(14) A process of expressing a heterologous nucleic acid sequence of interest in a plant or in plant tissue, comprising providing the plant or plant tissue with a nucleic acid of (9) or (10), with a potexviral vector according to (8), or with a combination or kit of nucleic acids according to (12) or (13), for expressing said heterologous nucleic acid sequence of interest.

(15) Use of a heterologous nucleic acid as defined in (9) to (11), a potexviral vector according to (8), or a combination or kit according to (12) or (13) for expressing a protein encoded by said heterologous nucleic acid and for achieving improved long-distance movement of a potexviral vector in a plant.

The inventors have surprisingly found that potexviral replicons carrying a heterologous nucleic acid encoding a protein of interest for expression in a plant or plant tissue have an improved capability for long-distance movement in a plant, if the GC content of the heterologous nucleic acid, notably of the ORF encoding the protein of interest, is increased. Thereby, the expression yield of a protein of interest in the plant or plant tissue is improved and costs for purification of the protein of interest decrease. In one embodiment, the protein of interest provides the plant with an agronomic trait.

Brief Description of the drawings

**Figure 1** shows schematically Potato Virus X (PVX)-based entry vectors pNMD4300 and pNMD670 for cloning of inserts of interest. The nucleotide sequences of these vectors are given as SEQ ID NO: 24 and 23, respectively. RB and LB indicate the right and left borders of T-DNA of binary vectors. P35S: cauliflower mosaic virus 35S promoter; PVX-pol: RNA-dependent RNA polymerase from PVX; CP: coat protein ORF; 25K, 12K and 8K together indicate the 25 kDa, 12 kDa and 8 kDa triple gene block modules from PVX; N: 3'-untranslated region from PVX. INSERT stands for DNA insert of interest; BsaI stand for BsaI restriction sites with corresponding nucleotide overhangs shown below. virGN54D is a virG gene with N54D mutation from LBA4404 strain of *Agrobacterium tumefaciens.*

**Figure 2** shows RT-PCR analysis of foreign insert stability in PVX viral vectors.

36 days old tomato *Solanum lycopersicum* 'Balcony Red' plants were transfected by syringe infiltration of agrobacterial cultures carrying PVX vectors. The infiltration was performed into two cotyledons leaves. Total RNA was isolated from systemic leaves of PVX infected plants 26 days post infiltration using NucleoSpin® RNA Plant kit (Macherey-Nagel). RNA was reverse transcribed using PrimeScript™ RT Reagent Kit (Takara Clontech); resulting cDNA was used as a template for PCR with oligos specific for either PVX (UPPER PANEL) or tobacco Elongation Factor EF1$\alpha$ used as a RNA loading control (LOWER PANEL). PCR fragments of expected size are shown with arrows. Positions of missing expected PCR products on the gel are shown with a dashed line.

RT-PCR products were resolved in 1% agarose gels. MWL: Molecular Weight Ladder; GFP: RT-PCR product for plant infected with PVX vector carrying GFP insertion; GUS, AtFT, CaDREB, SlSUN, SlLOG1, SlDREB1, SlGR, SlOVATE, SlWOOLLY: RT-PCR products for plants infected with PVX vectors with insertions of GUS, AtFT, CaDREB, SlSUN, SlLOG1, SlDREB1, SlGR, SlOVATE, SlWOOLLY genes, respectively; V: plant infected with empty PVX entry vector without foreign insertion. Sizes of expected PCR fragments are given in brackets.

**Figure 3** shows the relation between Insert Length and Stability. Latest day post infiltration when the full-length insert was detected (Y-axis) was plotted against the length of corresponding foreign insert (X axis). For analysis, values for GFP, GUS, AtFT, CaDREB, SlSUN, SlLOG1, SlDREB1, SlGR, SlOVATE and SlWOOLLY inserts (Table 1) were used.

**Figure 4** shows the relation between GC content and Stability of the insert. Latest day post infiltration when the full-length insert was detected (Y-axis) was plotted against the GC content (%) of corresponding foreign insert (X axis). For analysis, values for GFP, GUS, AtFT, CaDREB, SlSUN, SlLOG1, SlDREB1, SlGR, SlOVATE and SlWOOLLY inserts (Table 1) were used. GC content of inserts was determined using ENDMEMO on-line DNA/RNA GC Content Calculator (www.endmemo.com/bio/gc.php).

**Figure 5** shows the relation between GC content to Length Ratio and Stability of the insert. Latest day post infiltration when the full-length insert was detected (Y-axis) was plotted against the GC content to Length Ratio of corresponding foreign insert (X axis). For analysis, values for GFP, GUS, AtFT, CaDREB, SlSUN, SlLOG1, SlDREB1, SlGR, SlOVATE and SlWOOLLY inserts (Table 1) were used. The ratio between GC content and Length of insert was calculated using the formula: Ratio GC Content/Length = (GC content (%) / Length (bp) x 100.

**Figure 6** shows RT-PCR analysis of PVX vector stability for construct containing *SlANT1* insertions with different codon usage 21 days post infiltration (dpi). Systemic leaves of three independent tomato 'Balcony Red' plants were analyzed as described in Example 2.

Native (35.2%; 4.3): native *SlANT1* coding sequence with 35.2% GC content and 4.3 Ratio GC Content/Length (pNMD721 construct).

Tobacco (39.5%; 4.8): *SlANT1* coding sequence optimized for *Nicotiana tabacum* codon usage (39.5% GC content and 4.8 Ratio GC Content/Length; pNMD29561).

Arabidopsis (41.0%; 5.0): *SlANT1* coding sequence optimized for *Arabidopsis thaliana* codon usage (41.0% GC content and 5.0 Ratio GC Content/Length; pNMD29541).

Human (48.0%, 5.8): *SlANT1* coding sequence optimized for *Homo sapiens* codon usage (48.0% GC content and 5.8 Ratio GC Content/Length; pNMD29531).

Rice (48.4%; 5.9): *SlANT1* coding sequence optimized for *Homo sapiens* codon usage (48.4% GC content and 5.9 Ratio GC Content/Length; pNMD29551).

V: empty entry PVX vector pNMD4300. PL: plasmid; 1, 2, and 3: Plants 1, 2, and 3, respectively. Plasmid amplified PCR fragment serves as a positive size control.

**Figure 7** shows RT-PCR analysis of PVX vector stability for construct containing *SlANT1* insertions with different codon usage 52 days post infiltration. Systemic leaves of three independent tomato 'Balcony Red' plants were analyzed as described in Example 2.

Native (35.2%; 4.3): native *SlANT1* coding sequence with 35.2% GC content and 4.3 Ratio GC Content/Length (pNMD721 construct).

Tobacco (39.5%; 4.8): *SlANT1* coding sequence optimized for *Nicotiana tabacum* codon usage (39.5% GC content and 4.8 Ratio GC Content/Length; pNMD29561).

PVX (44.7%; 5.4): *SlANT1* coding sequence optimized for PVX codon usage (44.7% GC content and 5.4 Ratio GC Content/Length; pNMD30881).

Barley (51.0%; 6.2): *SlANT1* coding sequence optimized for *Hordeum vulgare* codon usage (51.0% GC content and 6.2 Ratio GC Content/Length; pNMD30722).

Bifido (56.1%; 6.8): *SlANT1* coding sequence optimized for *Bifidobacterium* codon usage (56.1% GC content and 6.8 Ratio GC Content/Length; pNMD30891).

V: empty entry PVX vector pNMD4300. PL: plasmid; 1, 2, and 3: Plants 1, 2, and 3, respectively. Plasmid amplified PCR fragment serves as a positive size control.

**Figure 8** shows RT-PCR analysis of PVX vector stability for construct containing native and codon-optimized sequences of *SlLOG1* and *SlOVATE* genes.

(A) Analysis of vectors with *SlLOG1* insertions.

Plant material from systemic leaves of tomato 'Balcony Red' plants was analyzed 34 days post infiltration. 1: plant transfected with pNMD27533 construct containing native *SlLOG1* sequence (41.9% GC content and 6.2 Ratio GC Content/Length). 2: plant transfected with pNMD31084 construct containing *SlLOG1* sequence optimized for *Oryza sativa* codon usage (53.2% GC content and 7.8 Ratio GC Content/Length). Expected size of PCR fragment for intact insertion is 870 bp, shown with arrow.

(B) Analysis of vectors with *SlLOG1* insertions. Upper panel: plant material analyzed 27 days post infiltration; Lower panel: plant material analyzed 82 days post infiltration.

Native (41.0%; 3.9): native *SlOVATE* coding sequence with 41.0% GC content and 4.6 Ratio GC Content/Length (pNMD27931 construct).

Rice (48.8%; 4.6): *SlOVATE* coding sequence optimized for *Oryza sativa* codon usage (48.8% GC content and 4.6 Ratio GC Content/Length; pNMD29551).

V: empty entry PVX vector pNMD4300. PL: plasmid; 1 and 2: Plants 1 and 2, respectively. Plasmid amplified PCR fragment serves as a positive size control.

**Figure 9** shows Table 1: PVX vector insertions and their stability (Example 2).

**Figure 10** shows Table 3: Native and codon-optimized sequences of SlLOG1 and SlOVATE genes (Example 4).

## Detailed Description of the Invention

Herein, the potexviral replicon is a nucleic acid that is replicated in plant cells and capable of cell-to-cell and long distance movement in a plant and in plant tissue. The potexviral replicon makes use of the replication and, preferably, protein expression system of potexviruses in plants or plant cells. The potexviral replicon may be built on a natural potexvirus e.g. by comprising genetic components from a potexvirus, or by using genetic components suitably altered compared to those of a potexvirus. The potexviral replicon is or comprises an RNA. The potexviral vector of the invention is the vehicle used for providing cells of a plant or of plant tissue with the potexviral replicon. The potexviral replicon may itself be used as the potexviral vector of the invention. However, the potexviral vector may comprise or encode the potexviral replicon. The potexviral vector as well as the nucleic acid mentioned below may be DNA or RNA. If it is RNA, it is or comprises the potexviral replicon; if it is DNA, it encodes the potexviral replicon. If the potexviral vector or said nucleic acid are DNA, segments (i) to (iii) are generally also DNA. If said potexviral vector or said nucleic acid are RNA, segments (i) to (iii) are generally also RNA.

The potexviral replicon is an RNA comprising at least the following segments (i) to (iii), preferably in this order in 5'- to 3'-direction:

(i) a nucleic acid sequence encoding a potexviral RNA-dependent RNA polymerase (RdRp);
(ii) a nucleic acid sequence comprising:

(a) a potexvirus triple gene block and
(b) optionally a sequence encoding a potexviral coat protein; or a sequence encoding a tobamoviral movement protein; and

(iii) a heterologous nucleic acid sequence comprising an ORF encoding a protein of interest.

The potexviral vector of the invention is a nucleic acid comprising or encoding the potexviral replicon. Accordingly, the potexviral vector comprises, preferably in this order in 5'- to 3'-direction, the following segments (i) to (iii):

(i) a nucleic acid sequence encoding a potexviral RNA-dependent RNA polymerase (RdRp);
(ii) a nucleic acid sequence:

(a) comprising or encoding a potexvirus triple gene block and
(b) optionally comprising a sequence encoding a potexviral coat protein; or comprising a sequence encoding a tobamoviral movement protein; and

(iii) a heterologous nucleic sequence comprising an ORF encoding a protein of interest.

The "nucleic acid" of the above potexviral vector and the "RNA" of the above potexviral replicon are also collectively referred to as "nucleic acid of the invention". The heterologous nucleic sequence of item (iii) is also referred to herein as "second heterologous nucleic (sequence)" and said ORF is also referred to herein as "second ORF". These elements and their production are further described below. Herein, an ORF (open reading frame) is the coding nucleic acid sequence of the protein of interest. The ORF consists of the base triplets from and including the start codon to the stop codon, and may include introns. The ORF encodes the protein of interest from its N-terminus to its C-terminus.

The potexviral replicon can replicate in plant cells due to the presence of the potexviral elements or segments of items (i) and (ii) and optionally further genetic elements of the potexviral replicon. These further genetic elements may also be contained in or encoded in the potexviral vector.

In the methods of the invention, the second heterologous nucleic acid sequence of item (iii) above is produced. The second heterologous nucleic acid sequence generally encodes the same protein as the first heterologous nucleic acid sequence. The second heterologous nucleic acid sequence differs from the first heterologous nucleic acid sequence in that the ORF of the former has a higher GC content than the ORF of the latter. Higher GC content means that the sum of G and C (guanine and cytosine) bases is higher. Thus, "GC content" herein means a G+C content. The GC content is determined by counting the number of G and C bases in a given nucleic acid. The second heterologous nucleic acid sequence may consist of the ORF or coding sequence of the protein of interest. The coding sequence of the protein of interest is herein also referred to as ORF (open reading frame). Alternatively, the second heterologous nucleic acid sequence may comprise the coding sequence (ORF) of the protein of interest and one or more further nucleotides or nucleotide stretches such as restriction endonuclease site(s) for engineering the potexviral vector or genetic elements for expressing the protein of interest from the potexviral replicon in plants or plant cells. If the second heterologous nucleic acid sequence comprises additional nucleotides or sequence stretches, the GC content defined herein is that of the segment that consists of the coding sequence (ORF) of the protein of interest. Preferably, the second heterologous nucleic acid sequence has a higher GC content than the first heterologous nucleic acid sequence.

The first heterologous nucleic acid sequence also comprises an ORF that encodes the protein of interest. The first heterologous nucleic acid sequence may be a physical entity. However, for the invention, it is sufficient that the higher CG content of the ORF of the second heterologous nucleic acid can be determined by counting GC bases. Therefore, it is not necessary that the first heterologous nucleic acid and its ORF is/are a physical entity; it is sufficient that the first heterologous nucleic acid is a virtual nucleic acid, e.g. represented by the commonly used characters C, G, A, and T/U written on a sheet of paper or written in a computer-readable electronic file. As is generally known, these characters stand for cytosine, guanine, adenine and thymine/uracil nucleotides, respectively, in a nucleic acid sequence.

The method employed for producing the second heterologous nucleic acid is not limited, provided the GC-content of the ORF encoding the protein of interest is higher than that of the ORF encoding the protein of interest of a first heterologous nucleic acid. Methods of producing a nucleic acid are part of the general knowledge in molecular biology. The second heterologous nucleic acid may, for example, be produced by automated DNA synthesis. The second heterologous nucleic acid may, alternatively, be produced by modifying the first heterologous nucleic acid by replacing nucleotides such that the GC content of the ORF encoding the protein of interest increases. Nucleotides of the first heterologous nucleic acid other than of the ORF may, if desired, also be changed in the production of the second heterologous nucleic acid.

Using the produced second heterologous nucleic acid, the potexviral replicon or the potexviral vector may be provided. The methods applicable in this step are generally known methods of molecular biology, and the invention is not limited with regard to the specific method used. Generally, it is preferred and more common to make the necessary nucleic acid modifications on the DNA level. Therefore, it is preferred that the second heterologous nucleic acid sequence is DNA and that the potexviral vector encoding the potexviral replicon is produced. For example, the second heterologous nucleic acid sequence may be inserted into a nucleic acid comprising a nucleic acid comprising the segments (i) and (ii) above to produce the potexviral vector of the invention. The step of inserting the second heterologous nucleic acid sequence may be a usual a sub-cloning step wherein parts or nucleotides of the second heterologous nucleic acid, e.g. nucleotides of an endonuclease restriction site, may get lost, i.e. may not be present in the product. Thus, it is possible that not the entire second heterologous nucleic acid sequence ends up in the potexviral vector. In any event, at least a portion comprising the ORF of the protein of interest of the second heterologous nucleic acid is inserted into the product which is the potexviral vector.

The second heterologous nucleic acid sequence may, apart from said ORF, further comprise genetic elements for expressing the protein of interest in plants or plants cells from the potexviral replicon, such as a ribosome binding site, a 5'-untranslated region and/or a 3'-untranslated region.

In the following, embodiments of the nucleic acid are described.

The nucleic acid of the invention comprises the following segments: (i) a nucleic acid sequence encoding a potexviral RNA-dependent RNA polymerase, (ii) a nucleic acid sequence comprising or encoding a potexviral triple-gene block, and (iii) a heterologous nucleic acid sequence comprising an ORF encoding the protein of interest.

In one embodiment, said ORF consists of at least 100 and at most 500 nucleotides and has a GC-content of at least 50%; or said ORF consists of at least 501 and at most 1000 nucleotides has a GC-content of at least 55%; and/or said ORF consists of at least 1001 nucleotides and has a GC-content of at least 58%.

In another embodiment, said ORF consists of at least 100 and at most 500 nucleotides and has a GC-content of at least 50%; or said ORF consists of at least 501 and at most 1000 nucleotides has a GC-content of at least 55%; and/or said ORF consists of at least 1001 nucleotides and has a GC-content of at least 58%.

In a further embodiment, said ORF has a GC-content of at least 50% within a segment of said heterologous nucleic acid sequence, said segment consisting of at least 200 and at most 400 nucleotides, preferably at least 100 and at most 500 nucleotides; or said ORF has a GC-content of at least 55% within a segment of said heterologous nucleic acid sequence, said segment consisting of from 401 to 800 nucleotides, preferably from 501 to 1000 nucleotides; and/or said ORF has a GC-content of at least 58% within a segment of said heterologous nucleic acid sequence, said segment consisting of 801 or more, preferably 1001 or more nucleotides. Preferably, said ORF has a GC-content of at least 52% within a segment of said heterologous nucleic acid sequence, said segment consisting of at least 200 and at most 400 nucleotides, preferably at least 100 and at most 500 nucleotides; or said ORF has a GC-content of at least 57% within a segment of said heterologous nucleic acid sequence, said segment consisting of from 401 to 800 nucleotides, preferably from 501 to 1000 nucleotides; and/or said ORF has a GC-content of at least 60% within a segment of said heterologous nucleic acid sequence, said segment consisting of 801 or more, preferably 1001 or more nucleotides. In another embodiment of the nucleic acid, said ORF has a GC-content of at least 50% within a segment of said heterologous nucleic acid sequence, said segment consisting of at least 100 and at most 500 nucleotides; or said ORF has a GC-content of at least 55% within a segment of said heterologous nucleic acid sequence, said segment consisting of from 501 to 1000 nucleotides; and/or said ORF has a GC-content of at least 58% within a segment of said heterologous nucleic acid sequence, said segment consisting of 1001 or more nucleotides; preferably, said ORF has a GC-content of at least 52% within a segment of said heterologous nucleic acid sequence, said segment consisting of at least 100 and at most 500 nucleotides; or said ORF has a GC-content of at least 57% within a segment of said heterologous nucleic acid sequence, said segment consisting of from 501 to 1000 nucleotides; and/or said ORF has a GC-content of at least 60% within a segment of said heterologous nucleic acid sequence, said segment consisting of 1001 or more nucleotides.

Potexviral vectors or nucleic acids comprising a heterologous nucleic acid encoding a green fluorescent protein may be excluded from the potexviral vectors or nucleic acid of the invention, respectively.

Said potexviral vector or nucleic acid of the invention may be obtainable by inserting the second heterologous nucleic acid sequence into a nucleic acid construct encoding a potexvirus, whereby said heterologous nucleic acid sequence may be inserted downstream of a sequence encoding the triple gene block and/or downstream of a sequence encoding the coat protein of said potexvirus. However, modifications may be made to the genetic components of a natural potexvirus, such as to the RdRP gene, the triple gene block, the coat protein gene, or to the 5' or 3' non-translated regions of a potexvirus, examples for which are described below.

The potexviral vector of the invention comprises, generally in the order from the 5' end to the 3' end, said segments (i) to (iii) of the invention. Further genetic elements may be present on said replicon for replication of the potexviral replicon in plant cells and/or or for expression of the protein of interest. For being a replicon, i.e. for autonomous replication in a plant cell, the potexviral replicon encodes an RdRp. The potexviral replicon may further have potexviral 5'- and/or 3'-untranslated regions and promoter-sequences in the 5'- or 3'-untranslated regions of said potexviral replicon for binding the potexviral RdRp and for replicating the potexviral replicon. Said potexviral replicon further may have sub-genomic promoters in segments of item (ii) and (iii) for generating sub-genomic RNAs for the expression of proteins encoded by the segments of items (ii) and (iii). If said potexviral vector or the nucleic acid is DNA, it will typically have a transcription promoter at its 5'-end for allowing production by transcription of said potexviral replicon in plant cells. An example of a transcription promoter allowing transcription of said RNA replicon from a DNA nucleic acid *in planta* is the 35S promoter that is widely used in plant biotechnology. The 35S promoter is an example of a constitutive promoter. Constitutive transcription promoters are preferably used in the potexviral vector, notably where the potexviral vector is used for transient transfection and transient expression on the protein of interest in a plant or in plant cells. If the potexviral vector is stably integrated in chromosomal DNA of a plant or in cells of a plant, the transcription promoter may be a regulated promoter such that formation of the potexviral replicon and expression of the protein of interest ca be started at a desired point in time. An example of regulated promoters is the ethanol-inducible promoter described, for example, in WO 2007/137788 A1.

Segment (i) encodes a potexviral RdRp. The encoded potexviral RdRp may be the RdRp of a potexvirus, such as potato virus X, or it may be a function-conservative variant of an RdRp of a potexvirus. Thus, the term "potexviral" is not restricted to sequences that are exactly present in a potexvirus; the terms "potexvirus" or "of a potexvirus" mean that the designated element or segment is taken from a potexvirus. The RdRp may be considered a function-conservative variant of the RdRp of a potexvirus if said sequence of segment (i) encodes a protein having a sequence identity of at least 36 % to a protein encoded by SEQ ID NO: 37. In another embodiment, said sequence identity is at least 45 %, in a further embodiment at least 55%, in another embodiment at least 65% and in an even further embodiment at least 75% to a protein encoded by SEQ ID NO: 37. These sequence identities may be present over the entire sequence of SEQ ID NO: 37. Alternatively, these sequence identities may be present within a protein sequence segment of at least 300 amino acid residues, within a protein sequence segment of at least 500 amino acid residues, within a protein sequence segment of at least 900 amino acid residues, or within a protein sequence segment of at least 1400 amino acid residues.

Herein, the determination of sequence identities and similarities is done using Align Sequences Protein BLAST (BLASTP 2.6.1+) (Stephen F. Altschul, Thomas L. Madden, Alejandro A. Schäffer, Jinghui Zhang, Zheng Zhang, Webb Miller, and David J. Lipman (1997), "Gapped BLAST and PSI-BLAST: a new generation of protein database search programs", Nucleic Acids Res. 25:3389-3402).

In one example, said sequence identity between an RdRP encoded by SEQ ID NO: 37 and a function-conservative variant of a potexvirus RdRp is at least 45% in a protein sequence segment of at least 900 amino acid residues. In another example, said sequence identity between a protein encoded by SEQ ID NO: 37 and a function-conservative variant of a potexvirus RdRp is at least 55% in a protein sequence segment of at least 900 amino acid residues.

Alternatively, the RdRp used in the potexviral replicon may be considered a function-conservative variant of a RdRp of a potexvirus if said sequence of item (i) encodes a protein having a sequence similarity of at least 50 % to a protein encoded by SEQ ID NO: 37. In another embodiment, said sequence similarity is at least 60 %, in a further embodiment at least 70%, and in another embodiment at least 80 % to a protein encoded by SEQ ID NO: 37. These sequence similarities may be present over the entire sequence of SEQ ID NO: 37. Alternatively, these sequence similarities may be present within a protein sequence segment of at least 300 amino acid residues, at least 500 amino acid residues, at least 900 amino acid residues, or at least 1400 amino acid residues. Amino acid sequence similarities may be determined using BLASTX defined above.

In one example, the sequence similarity between a protein encoded by SEQ ID NO: 37 and a function-conservative variant of a potexvirus RdRp is at least 70% in a protein sequence segment of at least 900 amino acid residues. In another example, said sequence similarity between a protein encoded by SEQ ID NO: 37 and a function-conservative variant of a potexvirus RdRp is at least 80% in a protein sequence segment of at least 900 amino acid residues.

Alternatively, the RdRp used in said potexviral replicon may be considered a function-conservative variant of a RdRp of a potexvirus if said sequence of item (i) has a sequence identity of at least 55 %, of at least 60%, or of at least 70% to SEQ ID NO: 37. Said sequence identities may be present within SEQ ID NO: 37, or within a sequence segment of at least 900 nucleotides, within a sequence segment of at least 1500 nucleotides, within a sequence segment of at least 2000 nucleotides, or within a sequence segment of at least 4200 nucleotides of SEQ ID NO: 37. Nucleotide sequence identities may be determined using the BLAST given above.

The potexviral replicon comprises the nucleic acid segment of item (ii) for allowing cell-to-cell movement of said potexviral replicon in a plant or in plant tissue. Cell-to-cell movement of the potexviral replicon is important for achieving expression of the segment of item (iii) in as many cells of said plant or said tissue as possible. The nucleic acid sequence of item (ii) comprises or encodes a potexviral triple gene block (abbreviated "TGB" herein; a review on the TGB is found in J. Gen. Virol. (2003) 84,1351-1366). The potexviral triple gene block encodes three proteins necessary to provide the capability of cell-to-cell movement to a potexvirus. The term "potexviral triple gene block" includes variants of the TGB of a potexvirus, provided the variants can provide, optionally with other necessary components, the potexviral replicon of the invention with the capability of cell-to-cell movement in a plant or in plant tissue.

Examples of a potexviral TGB are TGBs of a potexvirus. An example of a potexviral TGB is the TGB of potato virus X (referred to as "PVX TGB" herein). The PVX TGB consists of three genes encoding three proteins designated 25K, 12K, and 8K according to their approximate molecular weight. The gene sequences encoding the PVX 25K, the PVX 12 K protein, and the PVX 8K protein are given in SEQ ID NO: 29, SEQ ID NO: 31, and SEQ ID NO: 33, respectively. Protein sequences of the PVX 25 K protein, the PVX 12K protein, and the PVX 8K protein are given in SEQ ID NO: 30, SEQ ID NO: 32, and SEQ ID NO: 34, respectively.

In one embodiment, said variant of a potexvirus TGB is a block of three genes, said block encoding three proteins one of which having a sequence identity of at least 33% to the PVX 25K protein, one having a sequence identity of at least 36 % to the PVX 12K protein and one having a sequence identity of at least 30 % to the PVX 8K protein. In another embodiment, said function-conservative variant of a potexvirus TGB encodes three proteins one of which having a sequence identity of at least 40% to the PVX 25K protein, one having a sequence identity of at least 40% to the PVX 12K protein, and one having a sequence identity of at least 40 % to the PVX 8K protein. In a further embodiment, said function-conservative variant of a potexvirus TGB encodes three proteins one of which having a sequence identity of at least 50% to the PVX 25K protein, one having a sequence identity of at least 50 % to the PVX 12K protein and one having a sequence identity of at least 50 % to the PVX 8K protein. In a further embodiment, the corresponding sequence identity values are at least 60% for each protein. In a further embodiment, the corresponding sequence identity values are at least 70%, preferably at least 80%, for each protein.

In another embodiment, a function-conservative variant of a potexvirus TGB encodes three proteins as follows: a first protein comprising a protein sequence segment of at least 200 amino acid residues, said segment having a sequence identity of at least 40% to a sequence segment of the PVX 25K protein; a second protein comprising a protein sequence segment of at least 100 amino acid residues, said sequence segment having a sequence identity of at least 40 % to a sequence segment of the PVX 12K protein; and a third protein comprising a protein sequence segment of at least 55 amino acid residues, said sequence segment having a sequence identity of at least 40 % to a sequence segment of the PVX 8K protein. In a further embodiment, the corresponding sequence identity values are at least 50% for each protein. In a further embodiment, the corresponding sequence identity values are at least 60% for each of said first, second, and third protein.

Said nucleic acid sequence of item (ii) preferably comprises a further sequence encoding a protein for cell-to-cell movement and long distance movement of said potexviral replicon such as a potexvirus coat protein or a function-conservative variant thereof. A variant of said potexvirus coat protein is considered a function-conservative variant of said coat protein if it is capable of providing said potexviral replicon, together with other necessary components such as the TGB, with the capability of cell-to-cell movement and long distance movement in a plant or in plant tissue. In one embodiment where said potexviral replicon comprises a potexviral coat protein, said potexviral replicon does not have an origin of viral particle assembly for avoiding spread of said potexviral replicon from plant to plant in the form of an assembled plant virus. If said potexviral replicon comprises a potexviral coat protein gene and a potexviral TGB, it is possible that said TGB is located upstream of said coat protein gene or vice versa. Thus, said potexviral coat protein gene and said potexviral TGB may be present in any order in said nucleic acid sequence of item (ii).

The coding sequence of a PVX coat protein is given as SEQ ID NO: 35, and the amino acid sequence of the PVX coat protein is given as SEQ ID NO: 36. A protein can be considered a function-conservative variant of a potexvirus coat protein if it comprises a protein sequence segment of at least 200, alternatively at least 220, further alternatively 237 amino acid residues, said sequence segment having a sequence identity of at least 35% to a sequence segment of SEQ ID NO: 36. In another embodiment, a protein is considered a function-conservative variant of a potexvirus coat protein if it comprises a protein sequence segment of at least 200, alternatively at least 220, further alternatively 237 amino acid residues, said sequence segment having a sequence identity of at least 45% to a sequence segment of SEQ ID NO: 36. In alternative embodiments, the corresponding sequence identity values are at least 55%, preferably at least 65%, and more preferably at least 75%.

Alternatively, said nucleic acid sequence of item (ii) may comprise, optionally instead of said sequence encoding said potexviral coat protein or variant thereof, a sequence encoding a plant viral movement protein (MP). An example of a suitable MP is a tobamoviral MP such as an MP of tobacco mosaic virus or an MP of turnip vein clearing virus. Said sequence encoding a plant viral movement protein and said potexvirus TGB (or a function-conservative variant thereof) may be present in any order in said nucleic acid sequence of item (ii).

As described above, the heterologous nucleic acid sequence of item (iii) comprises at least the ORF of a protein of interest to be expressed in a plant or in plant tissue. Said sequence of item (iii) is heterologous in that it is heterologous to the potexvirus on which said potexviral replicon is based. In many cases, said sequence is also heterologous to said plant or said plant tissue in which it is to be expressed. For being expressible from said potexviral replicon in a plant or in plant tissue, the second heterologous nucleic acid of item (iii) typically comprises a sub-genomic promoter and other sequences required for expression such as ribosome binding site and/or an internal ribosome entry site (IRES). In a preferred embodiment, the second heterologous nucleic acid of item (iii) has one ORF that codes for one protein of interest.

The nucleic acid, the potexviral vector and/or the potexviral replicon of the invention may comprise a potexviral or, preferably, a potexvirus 5'-nontranslated region (5'-NTR) and a potexviral or, preferably, a potexvirus 3'-nontranslated region (3'-NTR).

The process of expressing a protein of interest in a plant or in plant tissue of the invention generally comprises providing a plant or plant tissue with said nucleic acid or potexviral vector of the invention. It is of course also possible to infect a plant or plant tissue with the potexviral replicon of the invention. In one embodiment, said process is a transient expression process, whereby incorporation of the nucleic acid or potexviral vector of the invention into chromosomal DNA of the plant host is not necessary and not selected for. Alternatively, the potexviral vector may be stably incorporated into chromosomal DNA to produce a transgenic plant. The production of transgenic plants is known to the skilled person and comprises, inter alia, transformation of plant cells or tissue, selection of transformed cells or tissue, and regeneration of transformed plants.

If said nucleic acid or said potexviral vector of the invention is RNA, it may be used for infecting a plant or plant tissue, preferably in combination with mechanical injury of infected plant tissue such as leaves. In another embodiment, said nucleic acid or potexviral vector of the invention is DNA. Said DNA may be introduced into cells of a plant or plant tissue, e.g. by particle bombardment or by *Agrobacterium-mediated* transformation. *Agrobacterium-mediated* transformation is the method of choice if several plants are to be provided with said nucleic acid or potexviral vector of the invention, e.g. for large scale protein production methods. Particularly efficient methods for *Agrobacterium-mediated* transformation or transfection are described in WO 2012/019660 and WO 2013/056829.

The process of expressing a protein of interest in a plant may be performed using the pro-vector approach (described in WO02088369 and by Marillonnet et al., 2004, Proc. Natl. Acad. Sci. USA, 101:6852-6857) by providing a plant or plant tissue with said kit or combination of nucleic acids of the invention. In this embodiment, the nucleic acid of the invention is produced by site-specific recombination between a first and a second nucleic acid in cells of said plant. Said first and a second nucleic acid act as the pro-vectors described in WO02088369 and by Marillonnet et al. (above) and are also referred to herein as pro-vectors. In one embodiment, a first nucleic acid (pro-vector) comprising or encoding segments of items (i) and (ii) and a second nucleic acid (pro-vector) comprising or encoding the segment of item (iii) is provided to a plant or plant tissue (e.g. by *Agrobacterium-mediated* transformation such as infiltration), wherein said first and said second pro-vector each has a recombination site for allowing assembly of a nucleic acid of the invention by site-specific recombination between said first and said second pro-vector. Preferable, said first nucleic acid has, downstream of segment (ii) a first site-specific recombination site recognizable by a site-specific recombinase, and said second nucleic acid has, upstream of segment (iii), a second site-specific recombination site recognizable by a, preferably the same, site-specific recombinase for allowing site-specific recombination between said first and said second site-specific recombination site and formation of a nucleic acid according to the invention.

Two or more vectors or said first and second nucleic acids may be provided to a plant or to plant tissue by providing mixtures of the vectors or mixtures of Agrobacterium strains, each strain containing one of said vectors or pro-vectors, to a plant or to plant tissue. The plant or plant tissue may further have or be provided with a site-specific recombinase recognizing the recombination sites of the first and second nucleic acids (pro-vectors). If the plant or plant tissue does not express the recombinase, a plant-expressible gene encoding the recombinase may be provided to the plant or plant tissue on one of said pro-vectors or on a separate vector. Examples of a usable site-specific recombinase are as described in WO02088369; an integrase as mentioned therein is also considered a site-specific recombinase. process of causing amplification and/or expression of one or more nucleic acid sequences of interest in a plant, plant tissue, plant cell or cell culture, characterized in that a plant cell is provided with at least two precursor vectors designed for undergoing processing by site-specific recombination in said plant cell, whereby due to the processing by site-specific recombination between said precursor vectors, said plant cell is endowed with at least one replicon capable of autonomous replication in said plant cell, whereby said replicon provide(s) for said amplification and/or expression.

Said protein of interest may be purified after production in said plant or plant tissue. Methods or purifying proteins from plants or plant cells are known in the art. In one method, a protein of interest may be directed to a plant apoplast and purified therefrom as described in WO 03/020938.

If one protein of interest has to be produced or expressed, a heterologous nucleic acid or ORF coding for said protein of interest may be included in said nucleic acid encoding said potexviral replicon. If two or more proteins of interest are to be produced in the same plant or in the same plant tissue, said plant or plant cells may be provided with another nucleic acid or potexviral vector comprising or encoding a further potexviral replicon. Said further potexviral replicon may then encode one or more further proteins of interest. In one embodiment, a first and a further nucleic acid of the invention may comprise or encode non-competing potexviral replicons as described in WO 2006/079546.

The process of expressing a protein of interest in a plant of the present invention is, with regard to the plant, not particularly limited. In one embodiment, dicotyledonous plants or tissue thereof are used. In another embodiment, *Nicotiana* species like *Nicotiana benthamiana* and *Nicotiana tabacum* are used; preferred plant species other than *Nicotiana* species are tomato, potato, pepper, eggplant, soybean, *Petunia hybrida, Brassica napus, Brassica campestris, Brassica juncea,* cress, arugula, mustard, strawberry, spinach, *Chenopodium capitatum,* alfalfa, lettuce, sunflower, potato, cucumber, corn, wheat and rice.

The most preferred plant viruses the potexviral replicons of the invention may be based on are Potexviruses such as potato virus X (PVX), papaya mosaic potexvirus or bamboo mosaic potexvirus.

The invention may also be used for improving the capability for long-distance movement in a plant of a potexviral RNA replicon encoding a protein to be expressed in said plant. In one embodiment, the method comprises the following steps:

a step of increasing the GC-content of a first ORF encoding said protein in a first heterologous nucleic acid sequence, thereby obtaining a second heterologous nucleic acid sequence comprising a second ORF, said second ORF encoding said protein and having an increased GC-content, and

a step of inserting said second heterologous nucleic acid sequence, or a portion thereof containing said second ORF, into a nucleic acid comprising (i) a nucleic acid sequence encoding a potexviral RNA-dependent RNA polymerase and (ii) a nucleic acid comprising or encoding a potexviral triple-gene block to produce a potexviral vector comprising or encoding said RNA replicon, said potexviral vector comprising the second heterologous nucleic acid or a portion thereof comprising said second ORF.

In another embodiment, the method comprises the following steps:

a step of producing a second heterologous nucleic acid sequence comprising a second ORF encoding said protein and having, in the second ORF, an increased GC-content compared to a first ORF encoding said protein in a first heterologous nucleic acid sequence,and

a step of providing said potexviral RNA replicon, or a potexviral vector comprising or encoding said potexviral RNA replicon, said potexviral RNA replicon comprising the following segments: (i) a nucleic acid sequence encoding a potexviral RNA-dependent RNA polymerase, (ii) a nucleic acid sequence comprising a potexviral triple-gene block, and (iii) said second heterologous nucleic acid or a portion thereof comprising said second ORF.

The above increasing, inserting, producing and providing steps may be performed similarly as described above. The methods of increasing the capability for long-distance movement in a plant of a potexviral replicon may be followed by providing the obtained potexviral replicon or potexviral vector to at least a part of said plant. An increase of the capability for long-distance movement in a plant may be followed experimentally, e.g. as described in the Examples. Generally, a plant may be provided with the potexviral vector on a selected leaf. After a predetermined period of time, e.g. after 5 days, after 7 days or after 9 days, tissue of systemic leaves may be investigated for the presence of the potexviral replicon encoded by the potexviral vector. RT-PCR may be used for testing any potexviral replicon in a systemic leaf for correctness and/or presence of all components of the potexviral replicon encoded by the potexviral vector. A systemic leaf is a leaf other than an inoculated leaf; a systemic leaf is a leaf where virus moves from a site of primary infection or transfection in inoculated leaf due to a long-distance systemic movement.

EXAMPLES

**Example 1: Plasmid constructs**

PVX-based assembled viral vectors pNMD670 and pNMD4300 (Fig. 1) were used for cloning of DNA inserts of interest. pNMD4300 is a modified version of pNMD670 construct which is described in WO 2012/019660. In contrast to pNMD670, pNMD4300 contains *virG* N54D mutant gene sequence from LBA4404 strain of *Agrobacterium tumefaciens* (GenBank Accession No CP007228, nucleotide positions 161000-161725) inserted into the plasmid backbone for increasing the efficiency of T-DNA transfer.

Nucleotide sequences of inserts of interest were either directly retrieved from GenBank or designed with modified GC content based on codon usage optimized for certain organisms. Sequences for cloning were either amplified using cDNA as a template or synthesized by Eurofins Genomics (Eurofins Genomics GmbH, Ebersberg, Germany). Codon usage modification was performed with Eurofins Genomics online tool based on codon usage patterns of organisms differing in average GC content (GENEius software). Inserts of interest were subcloned into pNMD4300 vector using Bsal restriction sites with CATG and GATC overhangs (Fig. 1). Flanking Bsal sites were added to sequences of interest either by PCR or during gene synthesis.

Sequences of gene inserts used for cloning are listed in Table1, Table 2 and Table 3.

**Example 2: PVX vector stability with inserts differing in Length, GC content and Ratio between GC content and the Length**

We subcloned AtFT, CaDREB-LP1, AmROS1, GmLOG1, SlLOG1, sGFP, SlGR, SlDREB1, SlOVATE, SlSUN, GUS and SlWoolly coding sequences (12 in total) into pNMD4300 cloning vector (Table 1). All of them except sGFP were native sequences from corresponding organisms. sGFP is a synthetic coding sequence for Green Fluorescent Protein from a jellyfish *Aequorea victoria* altered to conform to the favored codons of highly expressed human proteins which resulted in a substantial increase in expression efficiency (Haas et al 1996; Chiu et al 1996).

Gene inserts differed in their Length and GC content. The shortest insertion was AtFT (528b), and the longest one was SlWoolly (2193 bp) (Table 1). The GC content of inserts was determined using ENDMEMO on-line DNA/RNA GC Content Calculator (www.endmemo.com/bio/gc.php). The GC content of listed inserts was in in the range between 40.0% (SlSUN) and 61.4% (sGFP) (Table 1). We also calculated the Ratio between GC content and Length of inserts. It was done using the following formula:

$$\text{Ratio GC Content/Length} = (\text{GC content (\%)} / \text{Length (bp)} \times 100.$$

Multiplier x100 was used for convenience to avoid too small fractional numbers. According to this formula, the Ratio GC Content/Length varied between 2.0 (SlWoolly) and 8.6 (AtFT).

Cotyledons and the two first true leaves of 36 days old tomato *Solanum lycopersicum* 'Balcony Red' plants were syringe inoculated with agrobacterial cultures carrying PVX vectors listed in Table 1 (one independent plant per one construct). Plant material from infiltrated leaves was harvested using the cork borer at 9 dpi; the material from systemic leaves was harvested at 26, 27, 34 and 55 dpi. Total RNA isolated from harvested plant material reverse transcribed using PrimeScript™ RT Reagent Kit (Takara Clontech) and oligo dT primer. Resulting cDNA was used as a template for PCR with oligos specific for either PVX (8K-RT: tttgaagacatctcaacgcaatcatacttgtgc (SEQ ID NO: 25) and 3NTR-RT: tttgaagacttctcggttatgtagacgtagttatggtg (SEQ ID NO: 26)) or Elongation Factor EF1$\alpha$ from *N. benthamiana* (Genbank No. AY206004.1, oligos NbEF_for and NbEF_rev (*Dean et al.,* 2005) used as an RNA loading control. PCR products were resolved in 1% agarose gel. Fig. 2 illustrates the result of RT-PCR analysis for PVX vectors containing insertions of sGFP, GUS, AtFT, CaDREB-LP1, SlSUN, SlLOG1, SlDREB1, SlGR, SlOVATE and SlWoolly genes 26 days post infiltration. As one can see, at this time point PVX vectors with sGFP, AtFT, CaDREB1-LP1, SlSUN and SlGR remain pretty stable. In contrast, vectors with GUS, SlLOG1, SlDREB1 and SlWoolly have already lost their inserts.

The last day post infiltration when full-length insert was detected (even if additional shorter fragments resulting from partial insert elimination were present) was considered as a Last Time Point with Full Insert and used as criterion of vector stability (Table 1). We found the vectors with AtFT, CaDREB-LP1, AmROS1, and sGFP to be most stable: their inserts were detectable at 55 dpi. Vectors with SlLOG1, SlDREB1, GUS and SlWoolly genes were highly unstable: their inserts were not detectable in systemic leaves at all. Vectors with GmLOG1, SlGR, SlOVATE and SlSUN had moderate stability: their inserts were lost after 26-34 dpi.

We analyzed the relation between Length of tested inserts and their Stability. For this purpose, we plotted the Last Time Point with Full Insert (Y-axis) against the Length of Insert (X axis) (Fig. 3). We found that increasing the size of insert results in decreased vector stability, which is in accordance with former data from literature (e. g. Avesani et al., 2007).

We also analyzed the relation between GC content and Stability of inserts (Fig. 4). We did not find a clear trend for analyzed pool of sequences probably due to large difference in size between individual inserts (e.g. 4 times difference between SlWoolly and AtFT). We then analyzed the relation between the Ratio GC Content/Length and Insert Stability. In this case, a clear trend was observed: an increase of the Ratio GC Content/Length resulted in an increase of insert stability (Fig. 5).

**Example 3: Improving the stability of PVX with SlANT1 insert**

*Solanum lycopersicum* anthocyanin 1 (*SlANT1*) gene (AY348870.1) codes for MYB transcription factor anthocyanin 1 (SlANT1) (AAQ55181). ANT1 transcriptional factor activates the biosynthetic pathway leading to anthocyanin accumulation; plants overexpressing *ANT1* gene acquire intensive purple coloration due to anthocyanin accumulation (Mathews et al 2003).

We tried to overexpress *SlANT1* gene in tomato 'Balcony Red' plants using PVX-based viral vector. Native *SlANT1* coding sequence was subcloned into pNMD670 (without VirG) vector resulting in pNMD721 construct (Table 2). The pNMD721 construct was tested *in planta* using agrobacterial delivery via syringe infiltration of 28 days old plants. 21 dpi, relatively dense purple coloration was observed in infiltrated leaves. In contrast, few sparse colored spots were observed in systemic leaves. We analyzed systemic leaves of 3 independent plants transfected with this vector for the

integrity of *SlANT1* insert. RT-PCR analysis was performed as described in Example 2. It detected the loss of the insert by the PVX vector.

Table 2: *SlANT1* sequences with different codon usage (Example 3).

| SEQ ID NO: | Plasmid | Codon usage | Length, bp | GC content, % | Ratio GC content / Length |
|---|---|---|---|---|---|
| 13 | pNMD721 | *Solanum lycopersicum,* native (GenBank Accession No. AY348870.1) | 825 | 35.2 | 4.3 |
| 14 | pNMD29561 | *Nicotiana tabacum* | 825 | 39.5 | 4.8 |
| 15 | pNMD29541 | *Arabidopsis thaliana* | 825 | 41.0 | 5.0 |
| 16 | pNMD30881 | Potato Virus X | 825 | 44.7 | 5.4 |
| 17 | pNMD29531 | *Homo sapiens* | 825 | 48.0 | 5.8 |
| 18 | pNMD29551 | *Oryza sativa* | 825 | 48.4 | 5.9 |
| 19 | pNMD30722 | *Hordeum vulgare* | 825 | 51.0 | 6.2 |
| 20 | pNMD30891 | Bifidobacterium | 825 | 56.1 | 6.8 |

*SlANT1* sequence analysis revealed very low GC content (35.2%) and quite low Ratio GC content/Length (4.3). We designed 7 new sequence versions with increased GC content and, as result, Ratio between GC content and Length (Table 2). The design was performed using online codon optimization tool from Eurofins Genomics (GENEius software) based on codon usage of organisms with different average values of GC content in their genomes (data retrieved from Kazusa Codon Usage Database (www.kazusa.or.jp/codon/)). For this purpose, we selected codon usage patterns of *Nicotiana tabacum, Arabidopsis thaliana,* Potato Virus X, *Homo sapiens, Oryza sativa, Hordeum vulgare* and *Bifidobacterium* with average GC content 39.2%, 41.0%, 44.7%, 48.0%, 48.4%, 51.0%, and 56.1%, respectively. Additionally, poly dA (AAAAA and AAAAAAA) and poly dT (TTTTT) sequences as well as BsaI cleavage sites (GGTCTCNNNNN (SEQ ID NO: 27)) and predicted donor/acceptor splicing sites (AGGTRAG/GCAGGT (SEQ ID NO: 28)) were avoided inside sequences. Designed sequences were synthesized by Eurofins Genomics and subcloned into pNMD670 vector resulting in constructs listed in Table 2. All constructs were tested in tomato 'Balcony Red' using agrobacterial delivery via syringe infiltration (3 independent 28 days old plants per one construct). Systemic leaves of infected tomato plants were analyzed for PVX vector integrity at 21 and 52 dpi (Fig. 6 and 7).

At 21 dpi, complete loss of the insert with native sequence was found in 2 out of 3 plants. In one plant both intact and partially degraded vector sequences were detected (Fig. 6). For all other sequences (codon optimization for tobacco, Arabidopsis, human and rice), all tested plants contained intact vector sequence, although in some cases additional bands indicating partial loss of the insertion were also present (Fig. 6).

At 52 dpi, 2 plants for each construct were analyzed (Fig. 7). We found complete loss of the insert for native sequence in both plants. Vector degradation was also observed for tobacco and PVX-optimized sequences with lower GC content and Ratio between GC content and Length. In contrast, for sequences with higher GC content (barley and Bifidobacterium codon usage) one of two plants contained intact vectors with SlANT1 insertion (Fig. 7).

These data show that increasing the GC content of the foreign insert sequence and, correspondingly, the ratio between the GC content and Length allows improving the stability and increasing the lifetime of systemic PVX vector.

**Example 4: Improving the stability of PVX with SlLOG1 and SlOVATE inserts**

We also tried to improve the stability of PVX vectors with *SlLOG1 and SlOVATE* inserts. As it was shown in Example 2, *SlLOG1* insert with native sequence (pNMD27533) was not detectable in systemic leaves, indicating very high instability (Table 1). *SlOVATE* (pNMD27931) showed moderate stability; intact insert as well as products of degradation was still detectable in systemic leaves at 26 dpi; however, the intact insert was completely lost already at 27 dpi (Table 1).

*SlLOG1* native sequence is 678 bp in length; it has 41.9% GC content and 6.2 ratio between GC content and Length. *SlOVATE* is 1059 bp long; it has it has 41.0% GC content and 3.9 ratio between GC content and Length. We redesigned both sequences based on rice adapted codon used. Resulting sequences had increased GC content: 53.2% for *SlLOG1-rice and* 48.8% *for SlOVATE-rice.* Both sequences were synthesized by Eurofins MWG Operon and subcloned into pNMD4300 vector.

Resulting constructs (pNMD31084 for *SlLOG1-rice* and pNMD31611 for *SlOVATE-rice*) were tested in 24 and 25 days old tomato 'Balcony Red' plants as described in Example 2. At 34 dpi, RT-PCR analysis revealed the dramatic increase of *SlLOG1-rice* insert stability if compared with native sequence (Fig. 8, A). Significant increase of insert stability was also shown for codon-optimized *SlOVATE.* Rice codon usage adapted inserts remain intact at 27 dpi, whereas native sequence is completely lost (Fig. 8, A, Upper panel). Despite the presence of products of vector degradation, one can detect the intact insert of *SlOVATE-rice* (Fig. 8, A, Lower panel) even 82 dpi.

REFERENCES

1) Haas J., Park E.C., and Seed B. (1996) Codon usage limitation in the expression of HIV-1 envelope glycoprotein, Curr Biol 6(3): 315-24.

2) Chiu W., Niwa Y., Zeng W., Hirano T., Kobayashi H., and Sheen J. (1996) Engineered GFP as a vital reporter in plants, Curr Biol 6(3): 325-30.

3) Dean J.D., Goodwin P.H., Hsiang T. (2005) Induction of glutathione S-transferase genes of Nicotiana benthamiana following infection by Colletotrichum destructivum and C. orbiculare and involvement of one in resistance 56(416): 1525-1533.

4) Avesani L., Marconi G., Morandini F., Albertini E., Bruschetta M., Bortesi L., Pezzotti M., Porceddu A. (2007) Stability of Potato Virus X expression vectors is related to insert size: implications for replication models and risk assessment, Transgenic Res 16(5): 587-97.

5) Mathews H., Clendennen S.K., Caldwell C.G., Liu X.L., Connors K., Matheis N., Schuster D.K., Menasco D.J., Wagoner W., Lightner, J. and Wagner D.R. (2003) Activation tagging in tomato identifies a transcriptional regulator of anthocyanin biosynthesis, modification, and transport, Plant Cell 15 (8), 1689-1703.


Nucleotide and amino acid sequences

**SEQ ID NO: 1**
AtFT (NM_001334207.1) / one nucleotide exchange (deletion of Bsal-cleavage site)

Atgtctataaatataagggaccctcttatagtaagcagagttgttggagacgttcttgatccgtttaatagatcaatcactctaaaggttacttatgg
ccaaagagaggtgactaatggcttggatctaaggccttctcaggttcaaaacaagccaagagttgagattggtggagaagacctcaggaac
ttctatactttggttatggtggatccagatgttccaagtcctagcaaccctcacctccgagaatatctccattggttggtgactgatatccctgctac
aactggaacaacctttggcaatgagattgtgtgttacgaaaatccaagtcccactgcaggaattcatcgtgtcgtgtttatattgtttcgacagctt
ggcaggcaaacagtgtatgcaccagggtggcgccagaacttcaacactcgcgagtttgctgagatctacaatctcggccttcccgtggccgc
agttttctacaattgtcagagggagagtggctgcggaggaagaagactttag

**SEQ ID NO: 2**
>CaDREB-LP1 (NM_001324857.1)

ATGAACATCTTTAGAAGCTATTATTCGGACCCACTTACTGAATCTTCATCATCTTTTTCTGATAGT
AGCATTTACTCCCCTAATAGAGCTATTTTTTCTGATGAGGAAGTTATATTAGCATCAAATAACCCG
AAAAAGCCAGCTGGGAGGAAGAAGTTTCGAGAAACTCGACATCCAGTATACAGGGGAGTTAGG
AAGAGGAATTCAGGCAAATGGGTTTGTGAAGTCAGAGAACCCAATAAGAAATCAAGAATTTGGC
TTGGTACTTTTCCTACAGCTGAAATGGCTGCTAGAGCTCATGACGTGGCGGCTATAGCATTAAG
AGGTCGTTCTGCTTGTTTGAACTTTGCTGATTCTGCTTGGAGGTTGCCTGTTCCGGCTTCCTCT
GACACTAAAGATATTCAAAAGGCGGCCGCTGAGGCCGCGGAAGCCCTCCGACCATTGAAGTTG
GAAGGAATTTCAAAAGAATCATCTAGCAGTACTCCAGAGAGTATGTTCTTTATGGATGAGGAAG
CGCTCTTCTGCATGCCGGGATTACTTACGAATATGGCTGAAGGGCTAATGTTACCACCACCTCA
ATGTGCAGAAATTGGAGATCATGTGGAAACTGCTGATGCGGATACCCCTTTATGGAGCTATTCC
ATTTAA

**SEQ ID NO: 3**
>AmROS1(DQ275529.1)

atggaaaagaattgtcgtggagtgagaaaaggtacttggaccaaagaagaagacactctcttgaggcaatgtatagaagagtatggtgaa
gggaaatggcatcaagttccacacagagcagggttgaaccggtgtaggaagagttgcaggctgaggtggttgaattatctgaggccaaata
tcaaaagaggtcggttttcgagagatgaagtggacctaattgtgaggcttcataagctgttgggtaacaaatggtcgctgattgctggtagaatt
cctggaaggacagctaatgacgtgaagaacttttggaatactcatgtggggaagaatttaggcgaggatggagaacgatgccggaaaaat
gttatgaacacaaaaaccattaagctgactaatatcgtaagaccccgagctcggaccttcaccggattgcacgttacttggccgagagaagt
cggaaaaaccgatgaattttcaaatgtccggttaacaactgatgagattccagattgtgagaagcaaacgcaattttacaatgatgttgcgtcg
ccacaagatgaagttgaagactgcattcagtggtggagtaagttgctagaaacaacggaggatggggaattaggaaacctattcgaggag
gcccaacaaattggaaattaa

**SEQ ID NO: 4**
>GmLOG1(XM_003527643.3)

ATGGAAACTCAACACCAACAACCCACCATCAAGTCTAGGTTCAGACGCATCTGTGTCTACTGTG
GTAGCAGCCCTGGCAAAAACCCCAGCTACCAGCTCGCTGCTATTCAACTCGGAAAACAACTGG
TGGAGAGGAACATTGACTTGGTTTATGGAGGAGGAAGCATAGGGTTGATGGGTCTAATCTCACA
AGTTGTGTATGATGGTGGACGCCACGTGTTAGGGGTGATTCCAGAGACACTTAATGCAAGAGA
GATAACTGGAGAGAGTGTTGGAGAAGTGAGAGCTGTATCGGGCATGCACCAACGCAAAGCCGA
AATGGCCCGACAAGCCGATGCATTTATTGCACTGCCAGGTGGATATGGCACCCTTGAAGAACTA
CTGGAAATTATCACCTGGGCTCAACTAGGCATCCATGATAAACCGGTGGGGTTGTTGAACGTGG
ATGGGTACTACAACTCGCTGCTGGCATTCATGGACAAAGCTGTGGACGAAGGTTTCGTAACACC
AGCTGCCCGTCACATTATTGTTTCTGCCCACACTGCCCAAGAACTCATGTGCAAACTTGAGGAA
TATGTCCCCGAGCACTGTGGCGTGGCCCCCAAGCTAAGTTGGGAGATGGAGCAACAGTTAGTT
AACACTGCAAAGTCAGATATTTCCCGTTGA

**SEQ ID NO: 5**
>SILOG1 (NM_001324502.1)

ATGGAAAACAATCACCAGACACAAATTCAGACCACTAAAACATCAAGATTCAAACGCATATGTGT
TTTTTGTGGAAGCAGTCCAGGCAAAAAGCCAAGTTATCAACTTGCTGCTATTCAACTTGGCAATC
AACTGGTTGAAAGGAACATCGACTTGGTTTATGGAGGTGGCAGTGTGGGCTTGATGGGCCTAG
TTTCTCAATCAGTTTTTAATGGTGGCCGCCACGTGTTAGGGGTGATTCCTAAAACTCTTATGCCA
AGAGAGATTACTGGAGAAAGTGTTGGAGAAGTAAGAGCAGTGTCTGGGATGCATCAAAGAAAA
GCAGAAATGGCAAGACAAGCTGATGCATTCATAGCCTTACCAGGTGGCTATGGGACATTGGAA
GAGCTCCTAGAAGTCATCACTTGGGCTCAACTAGGCATTCATGATAAACCAGTAGGTTTACTTAA
TGTAGATGGCTACTATAATTCATTATTATCATTTATAGACAAAGCTGTTGATGAAGGCTTTGTCAC
ACCCTCTGCCCGTCACATCATTATTTCTGCCCCAACTGCCCAAGAACTCATGTCTAAGCTTGAG
GATTATGTACCAAAGCATAATGGGGTGGCACCAAAATTGAGTTGGGAAATGGAACAACAACTTG
GCTACACAACAACAAAATTGGAAATTGCTCGTTAA

**SEQ ID NO: 6**
>sGFP (U43284.1), nucleotide positions 826-1545 / nucleotide exchanges C96T and T695A

atggtgagcaagggcgaggagctgttcaccggggtggtgcccatcctggtcgagctggacggcgacgtaaacggccacaagttcagcgtg
tccggcgagggcgagggcgatgccacctacggcaagctgaccctgaagttcatctgcaccaccggcaagctgcccgtgccctggcccac
cctcgtgaccaccttcagctacggcgtgcagtgcttcagccgctaccccgaccacatgaagcagcacgacttcttcaagtccgccatgcccg
aaggctacgtccaggagcgcaccatcttcttcaaggacgacggcaactacaagacccgcgccgaggtgaagttcgagggcgacaccctg
gtgaaccgcatcgagctgaagggcatcgacttcaaggaggacggcaacatcctggggcacaagctggagtacaactacaacagccaca
acgtctatatcatggccgacaagcagaagaacggcatcaaggtgaacttcaagatccgccacaacatcgaggacggcagcgtgcagctc
gccgaccactaccagcagaacacccccatcggcgacggccccgtgctgctgcccgacaaccactacctgagcacccagtccgccctga
gcaaagaccccaacgagaagcgcgatcacatggtcctgctggagttcgtgaccgccgccgggatcactcacggcatggacgagctgtac
aagtaa

**SEQ ID NO: 7**
>SIGR (XM_010325884.2)

atgctgccaagaagatatcctcagatggatgctaatcctagtaatgggggtgaaagggataatgctttgcgaggaattctgcaggacttatgg
ccactggatgaaattgatccaagcactcaaaagttcccttgttgccttgtttggactcctctccctgtgatttcttggcttgcaccttttgttggacatgt
tggcatatgcagggaggatggtaccattgtggatttttctggagatagcatgattcattttggtcagctcttctatggaactgtagccaaatactatc
aggtagacagacagcagtgctgttttgctcgcaactttggtggacacacatgccgtaagggttatgaacatgttgtatttgggacagcagtaag
ttgggatgatgctgttcagttgtttaggcgcacctttgagaacagaaacttcaaagttttcagttgcaacggccactcattcgctgctgattgcctg
aacctgctatcatttagaggatcaatgcgctggaacatgattaatgttggagctcttataatgtttgagggaaagtgggtcagtcgctggtcaatg
ttacgatcatttctgcctttcattgggatactttgcttcggctatttaatgattggatggatgtttccaattggtctgctctcctttgttattgggacttttggat
ggtatgtcatgatctgttactgttgcaagattgaggatgacaattag

**SEQ ID NO: 8**
>SIDREB1 (NM_001247760.1)

ATGGCTATTATGGATGAAGCTGCTAATATGGTTTGTGTGCCGTTGGATTATAGTAGAAAGAGGA
AATCAAGGAGTAGAAGGGACAGAACAAAAAATGTGGAAGAGACACTAGCTAAATGGAAGGAGT
ATAATGAGAAACTAGACAATGAAGGGAAAGGGAAGCCAGTGCGTAAAGTTCCTGCTAAAGGTTC
AAAGAAGGGGTGTATGAGAGGTAAAGGGGGACCAGAAAATTGGCGGTGTAAATACAGAGGTGT
TAGACAGAGGATATGGGGTAAATGGGTTGCTGAGATTAGGGAACCTAAAAGAGGTAGTAGGTTA
TGGTTGGGTACATTTGGTACAGCAATTGAAGCTGCTTTAGCATATGATGATGCTGCAAGAGCTA
TGTATGGTCCTTGTGCAAGGCTTAATTTGCCAAATTACGCGTGTGATTCTGTTTCCTGGGCAACT
ACATCTGCATCTGCATCTGCATCTGATTGCACCGTTGCTTCTGGTTTCGGCGAGGTATGTCCGG
TTGATGGTGCTCTTCATGAAGCTGACACACCATTGAGCTCAGTGAAAGACGAAGGGACCGCGA
TGGATATTGTTGAACCTACGAGTATTGATGAAGATACGCTTAAGTCTGGATGGGATTGTCTAGAT
AAATTAAATATGGATGAGATGTTTGATGTAGATGAGCTATTGGCTATGTTAGATTCTACTCCAGTT
TTCACCAAGGACTACAATTCAGATGGAAAGCACAACAATATGGTATCAGATTCGCAATGTCAGG
AGCCGAATGCAGTGGTAGATCCTATGACTGTTGACTATGGCTTTGATTTTCTGAAACCAGGCAG

GCAAGAAGATCTTAATTTCAGTTCGGATGACCTTGCATTCATAGACTTGGATTCTGAACTTGTCG
TTTGA

**SEQ ID NO: 9**
>SIOVATE(NM_001247292.2)

ATGGGAAAAAGTTTGAAGCTTCGGTTCTCCAGAGTTATTGCTTCTTTCAATTCGTGCCGTTCGAA
AAACCCTTCTTCTCTTCCCCAAAATCCTAATTTCTTCCCACATAAGCTCACTAGTACAAAACACAT
TTCCCCCGATTTCCCTCTTATTGATCAAAATCAAAATCAAAATCACCGTAATTACGTGCCAGAAT
CCACGATGATCTCCGTTGGGTGTTGTAGATCAGAATTCAAGTGGGAGAAAGAAGAGAAGTTTCA
CGTGGTTTCTAGTTCCTTCGTGTCTGAAGAAGAAGAATGTGAAGAGGAGATCAATTTGGCCTTA
CGACCTCCTCTTACACCTCCGCGATTCAGTAGAATTGTTGTTGAGAAGAAGAAGAAGAAACAAC
AGCGAGTTAAAAAAACGAAAACAAAAAGTAGAATCATCCGAATGAGTACTTCCTCAGCTGATGA
GTACAGCGGGATATTAAGCGGTACTAATACTGATTGGGATAATAATGAAGAGGAAACTGAATCT
TTAGTTTCATCTTCCAGAAGCTGTTACGATTTCTCAAGCGATGACTCATCTACTGATTTCAACCCT
CACTTAGAAACCATATGTGAGACCACTACAATGAGGCGTCGTCACAAGAGAAATGCCAACACCA
AGAGGAGATCAATCAAGCAATCCAGACCAAGTTTTTCCTCTTCAAAAGGTAGAAGATCGTCGGT
TTCTACGTCATCAGATAGCGAGCTACCGGCAAGGTTATCGGTGTTTAAGAAGCTGATACCGTGT
AGTGTGGATGGGAAAGTGAAGGAGAGTTTCGCGATAGTGAAGAAATCTCAGGACCCGTACGAA
GATTTCAAGAGATCGATGATGGAAATGATTTTAGAGAAGGAAATGTTTGAGAAGAATGAGCTGG
AACAGCTTTTACAATGTTTTCTGTCGTTGAACGGAAAGCATTATCATGGAGTGATAGTTGAGGCG
TTCTCAGACATTTGGGAGACTTTGTTTTTAGGTAATAATGATAGAGTAAGGAGGATGTCAATTCA
TGATCCCACACCCACCTACTGTAGGTAG

**SEQ ID NO: 10**
>SISUN (NM_001246864.2)

ATGGGAAAGCGAAGAAACTGGTTTACCTTTGTCAAGAGACTTTTCATTCCTGAAACAGAATCAAC
AGCAGATCAAAAGAAACCAAAGAGATGGAGATGTTGTTTTCTGAGAAAGTTCAAGTTGAGGAAA
TGTCCTGCTATAACATCAGCACCTCAGCAAACGTTACCTGAGGCGAAAGGAACACCTCAGCAAA
CGTTAACTGAGGCGAAAGAACAGCAAAGAAAACATGCTTTTGCAGTTGCTATAGCAACGGCAGC
AGCTGCTGAGGCTGCTGTAGCTGCTGCTAATGCTGCTGCTGATGTTATTCGTCTAACAGATGCT
CCAAGTGAATTCAAAAGGAAACGCAAACAAGCTGCTATTAGAATCCAAAGTGCTTATCGCGCTC
ACCTGGCCCAGAAAGCATTAAGGGCTCTAAAGGGTGTTGTGAAGCTTCAAGCAGTGATTAGAG
GTGAAATTGTGAGAGGAAGACTCATTGCCAAACTGAAGTTCATGTTGCCACTTCATCAAAAGTCA
AAAACAAGAGTTAATCAAATTAGAGTCCCTACTTTTGAAGATCATCATGACAAGAAACTCATCAAT
AGTCCAAGGGAAATTATGAAAGCTAAAGAACTAAAGCTTAAATGCAAGAGCCTTAGCACTTGGA
ATTTCAACTTAGCTTCAGAACAAGACAGTGAAGCCTTGTGGTCAAGAAGAGAAGAAGCCATTGA
CAAAAGAGAGCATTTGATGAAATACTCGTTTTCACATCGGGAGAGAAGAAACGATCAAACTCTA
CAAGACTTACTAAACAGAAAGCAAAACAGAAGAAGCTACAGGATTGACCAGTTAGTAGAACTTG
ACGCACCAAGAAAAGCAGGGTTGTTAGAGAAATTGAGATCATTTACAGACTCAAATGTTCCTCTA
ACTGATATGGATGGAATGACACAGCTTCAAGTGAGAAAAATGCATAGATCAGATTGTATAGAGG
ACCTACATTCTCCTTCTTCACTTCCAAGAAGATCATTTTCTAATGCAAAACGAAAATCAAACGTTG
ATGATAACTCATTACCAAGTTCTCCTATATTTCCTACTTACATGGCAGCCACAGAATCTGCAAAG
GCAAAAACAAGGTCAAACAGCACAGCGAAGCAACACCTAAGGTTACACGAGACATTGTCAGGT
CAACATTCTCCTTATAACCTCAAGATTTCTTCTTGGAGATTGTCTAATGGTGAAATGTATGACAG
CGCCAGAACAAGCAGAACTTCTAGCAGTTATATGTTAATATAG

**SEQ ID NO: 11**
>GUS (S69414.1) / nucleotide exchanges G835C and G903A

atgttacgtcctgtagaaaccccaacccgtgaaatcaaaaaactcgacggcctgtgggcattcagtctggatcgcgaaaactgtggaattgat
cagcgttggtgggaaagcgcgttacaagaaagccgggcaattgctgtgccaggcagttttaacgatcagttcgccgatgcagatattcgtaat
tatgcgggcaacgtctggtatcagcgcgaagtctttataccgaaaggttgggcaggccagcgtatcgtgctgcgtttcgatgcggtcactcatta

cggcaaagtgtgggtcaataatcaggaagtgatggagcatcagggcggctatacgccatttgaagccgatgtcacgccgtatgttattgccg
ggaaaagtgtacgtatcaccgtttgtgtgaacaacgaactgaactggcagactatcccgccgggaatggtgattaccgacgaaaacggca
agaaaaagcagtcttacttccatgatttctttaactatgccggaatccatcgcagcgtaatgctctacaccacgccgaacacctgggtggacg
atatcaccgtggtgacgcatgtcgcgcaagactgtaaccacgcgtctgttgactggcaggtggtggccaatggtgatgtcagcgttgaactgc
gtgatgcggatcaacaggtggttgcaactggacaaggcactagcgggactttgcaagtggtgaatccgcacctctggcaaccgggtgaag
gttatctctatgaactgtgcgtcacagccaaaagccagacagagtgtgatatctacccgcttcgcgtcggcatccggtcagtggcagtgaagg
gccaacagttcctgattaaccacaaaccgttctactttactggctttggtcgtcatgaagatgcggacttacgtggcaaaggattcgataacgtg
ctgatggtgcacgaccacgcattaatggactggattggggccaactcctaccgtacctcgcattacccttacgctgaagagatgctcgactgg
gcagatgaacatggcatcgtggtgattgatgaaactgctgctgtcggctttaacctctctttaggcattggtttcgaagcgggcaacaagccga
aagaactgtacagcgaagaggcagtcaacggggaaactcagcaagcgcacttacaggcgattaaagagctgatagcgcgtgacaaaa
accacccaagcgtggtgatgtggagtattgccaacgaaccggatacccgtccgcaaggtgcacgggaatatttcgcgccactggcggaag
caacgcgtaaactcgacccgacgcgtccgatcacctgcgtcaatgtaatgttctgcgacgctcacaccgataccatcagcgatctctttgatgt
gctgtgcctgaaccgttattacggatggtatgtccaaagcggcgatttggaaacggcagagaaggtactggaaaaagaacttctggcctggc
aggagaaactgcatcagccgattatcatcaccgaatacggcgtggatacgttagccgggctgcactcaatgtacaccgacatgtggagtga
agagtatcagtgtgcatggctggatatgtatcaccgcgtctttgatcgcgtcagcgccgtcgtcggtgaacaggtatggaatttcgccgattttgc
gacctcgcaaggcatattgcgcgttggcggtaacaagaaagggatcttcactcgcgaccgcaaaccgaagtcggcggcttttctgctgcaa
aaacgctggactggcatgaacttcggtgaaaaaccgcagcagggaggcaaacaatga

**SEQ ID NO: 12**
>SIWoolly (XM_004232686.3)

atgtttaataaccaccagcacttgctcgatatatcgtcctcagctcaacgaacacctgataacgagttggatttcattcgtgatgaagagtttgat
agcaactctggtgctgataacatggaagctcccaattcaggtgatgacgatcaagctgatccaaaccaacctccaaacaagaagaagcgtt
atcatcgccacactcagaatcagattcaggaaatggagtccttttacaaggaatgcaatcatccagatgacaagcaaaggaaggaattggg
aagaagacttggtttggagccattacaagtgaaattttggttccagaacaagcgtactcagatgaaggctcaacatgagcgatgtgagaaca
cacagttgaggaatgaaaatgagaagcttcgcgctgagaacataaggtacaaagaagctttgagtaatgcagcatgcccaaattgtggag
ggccagcagctataggagagatgtcatttgatgagcatcagttgaggattgaaaatgctcgtcttagagatgagattgacaggataactggaa
tagctggaaagtatgttggtaaatcagcccttggatattctcatcaacttcctcttcctcagcccgaagctcctcgggttctggatcttgcttttgggc
ctcaatcgggcctgcttggagaaatgtacgctgctggtgaccttctaagaactgctgttacgggccttacagatgctgagaagcccgtggtcatt
gagcttgctgttactgcaatggaggaacttataaggatggctcaaactgaagagccattatggttgccaagctcaggctctgagactttatgtga
gcaagaatatgctcgtattttccctcgaggccttggacctaagccagctacactcaattctgaagcctcacgagaatctgctgttgtgattatgaa
tcatatcaatttagttgagattttgatggatgtgaaccaatggactactgtttttgctggtctggtgtcaaaagcaatgactcttgaagtcttatcaact
ggtgtcgcaggaaatcacaatggagcattgcaagtgatgacagcagaatttcaagttccatctccacttgttccaactcgggagaactatttctt
aagatactgtaaacaacatggtgaagggacttgggtagtggttgatgtttccctggacaacttgcgcactgtttcagttccgcgttgcagaagaa
ggccatctggttgtttaatccaagaaatgccaaatggttactcaagggttatatgggttgaacacgttgaggtggatgaaaatgctgtccatgac
atctacaaacctcttgtcaattctgggattgcatttggagcaaaacgctgggtagcaactttagatagacaatgtgaacgccttgcaagtgtgttg
gcgcttaacatcccaacaggagatgttggaatcattactagtccagctggtcgaaagagtatgctaaaacttgctgagagaatggtgatgagc
ttttgtgctggagttggtgcatcgacaactcacatatggacaactttgtctggaagtggtgcggatgatgttagagtcatgactaggaagagtatc
gatgatccagggagacctcctggtattgtgctgagtgctgcaacatctttttggcttccagtttctcctaagagagtgtttgatttctccgcgatgag
aactctagaaatgagtgggatattctttcaaatggtgggattgttcaggaaatggcacacattgcaaatggtcgtgatccaggaaactgtgtttct
ctactccgtgtcaatactggaacaaactctaaccagagtaacatgctgatactccaagagagcacaactgatgtaacaggatcttacgtcattt
acgctccagttgatattgctgcaatgaacgtggtgttaggtgggggtgaccctgactatgttgctctgttgccatctggttttgctattcttccagacg
gaccgatgaattatcatggtggaggtaattcagaaattgattctcctggtggatcgctactaactgtagcatttcagatattggttgattcagtccca
actgcaaagctttcccttggctctgttgcgactgttaatagtctcatcaaatgcaccgttgaaaagatcaaaggtgctgtaacttccgcaaatgca
tga

**SEQ ID NO: 13**
>SIANT1(NM_001247488.1) native sequence from *Solanum lycopersicum*

atgaacagtacatctatgtcttcattgggagtgagaaaaggttcatggactgatgaagaagattttcttctaagaaaatgtattgataagtatggt
gaaggaaaatggcatcttgttcccataagagctggtctgaatagatgtcggaaaagttgtagattgaggtggctgaattatctaaggccacata
tcaagagaggtgactttgaacaagatgaagtggatctcattttgaggcttcataagctcttaggcaacagatggtcacttattgctggtagacttc
ccggaaggacagctaacgatgtgaaaaactattggaacactaatcttctaaggaagttaaatactactaaaattgttcctcgcgaaaagatta

acaataagtgtggagaaattagtactaagattgaaattataaaacctcaacgacgcaagtatttctcaagcacaatgaagaatgttacaaac
aataatgtaattttggacgaggaggaacattgcaaggaaataataagtgagaaacaaactccagatgcatcgatggacaacgtagatccat
ggtggataaatttactggaaaattgcaatgacgatattgaagaagatgaagaggttgtaattaattatgaaaaaacactaacaagtttgttacat
gaagaaatatcaccaccattaaatattggtgaaggtaactccatgcaacaaggacaaataagtcatgaaaattggggtgaattttctcttaattt
accacccatgcaacaaggagtacaaaatgatgatttttctgctgaaattgacttatggaatctacttgattaa

**SEQ ID NO: 14**
>SlANT1 with *Nicotiana tabacum* codon usage

atgaattctacaagtatgtcaagcttaggcgttcgtaagggatcttggacagatgaagaagatttccttctacgaaagtgtattgacaaatatggt
gagggaaaatggcatttggttccgattagagctggtttgaatcgatgcaggaaatcctgtagacttaggtggttgaactatcttagacctcacat
aaagagaggtgatttcgagcaagatgaagtggatctcatactcagactacacaaacttttagggaatcgttggagtcttattgcaggcagatta
ccaggtagaacagccaatgatgtcaagaactattggaatactaatcttttaaggaagttgaacactacaaagatagtaccaagggagaaaa
tcaacaacaaatgtggggaaatttctacgaaaattgagattatcaagccccaaagacgtaagtacttttcatccactatgaagaatgtcacca
acaacaatgttatcctcgacgaagaagaacattgcaaagagatcatttctgagaagcagactcctgatgcttcaatggacaacgttgatcctt
ggtggataaatcttctagagaattgcaacgatgatatagaagaggatgaagaagtggtgattaactacgagaaaaccttaactagcctgttgc
atgaagaaatctctccacccccttaatattggagaaggaaattcaatgcaacaaggccagatttctcatgagaattggggtgaattttccttgaat
ctgccacctatgcagcaaggagtacagaatgacgactttagtgcagagattgatctctggaatctgttggactaa

**SEQ ID NO: 15**
>SlANT1 with *Arabidopsis thaliana* codon usage

atgaattcaacatcaatgtctagtctaggagtaaggaaaggttcatggacagatgaagaggactttcttctccggaaatgcattgataagtatg
gggaaggaaaatggcatttagtccccattagagctggcttgaatcgttgtaggaaatcgtgtcgactcagatggctaaactatcttagaccgca
tatcaagcggggtgatttcgaacaggacgaagtggacttgattttgaggcttcacaagttattgggtaatcgttggtcccttatagctgggagatt
accaggtagaacagccaatgatgtgaagaattactggaatacgaacttgctgagaaaactcaacactaccaagatcgttccgagagaaaa
gatcaacaacaaatgtggcgagattagcacgaagatagagatcataaagcctcaacgtcgaaaatacttctctagcactatgaagaatgtc
accaataacaacgtgatactagatgaagaagaacactgtaaggagattatcagtgagaaacagactcctgatgcatctatggacaatgttg
atccttggtggattaaccttctggagaattgcaatgacgatattgaggaggatgaagaggttgtaatcaactatgagaaaacacttacttcactc
cttcatgaagagatatctccaccacttaacattggagagggtaactccatgcaacaaggacagatctctcatgaaaattggggagaattttcg
ctgaatttgcctccaatgcaacaaggagttcagaacgacgattttagtgcggaaattgatctctggaacttattggattaa

**SEQ ID NO: 16**
>SlANT1 with Potato Virus X codon usage

atgaatagcactagcatgtcaagcttaggtgtgagaaagggctcatggactgacgaagaggatttcctgttgaggaagtgcatcgacaagta
tggagaaggcaaatggcaccttgtaccgattagggcagggcttaacaggtgcaggaaaagctgtaggttgaggtggttgaactatctcaga
ccccatataaagagaggcgactttgagcaagatgaagtggacctaattcttcgcttacacaaactccttgggaataggtggagtctgatagct
ggaaggctacctggtagaacagctaacgacgtgaagaactactggaataccaacctattacgcaaactgaacactaccaaaatcgttccc
agagagaagatcaacaacaagtgtggcgagataagcacgaagatcgaaatcatcaaaccgcaaagaaggaagtacttcagttcaacca
tgaagaatgtcacaaacaacaatgtcatactggatgaagaagagcactgcaaggagattatttccgagaaacagacaccagacgcatcc
atggacaatgtcgatccatggtggattaacctactcgaaaattgcaacgatgacattgaagaggatgaggaagtagtgatcaactacgaga
aaacactgacttctctcttgcatgaggagatcagtccacctttgaacattggagaagggaattctatgcaacaaggacagataagccacgaa
aattggggagagttttccctcaatctcccacctatgcaacagggtgttcagaacgatgacttctcagccgaaatcgacttatggaacctactcg
actaa

**SEQ ID NO: 17**
>SIANT1 with *Homo sapiens* codon usage

atgaattctacgtccatgtctagcctcggggttaggaaaggctcatggacagacgaagaggactttctgctgcgcaaatgcatagacaagtat
ggcgaaggaaagtggcatctggtgcccattagggctggtctgaaccggtgtcgcaagtcctgtaggttgcggtggcttaactacctcagaccc
cacatcaaacgaggcgatttcgaacaggatgaggtcgacctgattctccgtctgcacaagctgttgggtaacagatggagcctcattgcagg
gagactccctggaagaactgccaatgacgtcaagaactactggaacaccaaccttcttcgcaagctgaataccactaagatcgttcctcgag
agaagatcaacaacaaatgtggagaaatatccaccaaaatcgagatcatcaagccacaacggaggaaatacttctccagcacaatgaa
gaatgtgaccaacaacaacgtgattttggacgaagaggagcattgcaaagagatcatcagtgagaagcagacacctgatgcctctatggat

aatgtggacccctggtggataaatctgctggagaattgcaatgatgacattgaagaagatgaggaagtggtcatcaactatgagaaaacact
gacttcactgctgcatgaagagattagtccaccgctgaacattggggaggggaatagcatgcagcagggacagatcagtcacgaaaattg
gggcgaattcagccttaatctcccacccatgcaacagggcgtacagaacgacgacttttcagcggagattgatctgtggaatttgctggattaa

**SEQ ID NO:** 18
>SIANT1 with *Oryza sativa* codon usage

atgaattcaacgagcatgagctcgttgggtgttcgcaaaggctcttggaccgatgaagaggacttcctcttgcgaaagtgcatcgataagtatg
gggaaggaaagtggcatcttgtacccatacgtgcgggacttaaccggtgtcgcaagtcgtgcagactcaggtggctcaactatctacggcct
cacatcaaacgtggcgatttcgaacaagacgaggttgaccttatcctgagactgcacaaactgctcggcaatcgctggagtctcatagctggt
cgattgcctgggaggactgccaatgacgtcaagaattactggaatacaaaccttctgaggaagctgaataccacgaagatagttcctcggg
agaagatcaacaacaagtgtggggagatttccacgaaaatcgagatcatcaagccgcaaaggcgcaaatacttctcaagcacaatgaag
aacgtcaccaacaacaacgtgattctcgatgaggaggaacactgcaaggagatcatctctgagaaacagactccagatgcctcaatggac
aatgtggatccgtggtggattaacctcctggagaactgcaatgatgacattgaagaggacgaagaggtcgtgatcaactacgaaaagaccc
tcacatctctcctccatgaggaaataagtccaccgctcaatattggcgaaggcaattccatgcagcaaggccagatttcgcatgagaactgg
ggtgagttttccctgaatctaccacccatgcagcaaggagtgcagaatgatgacttttccgcagagattgacttgtggaacttgcttgattaa

**SEQ ID NO: 19**
>SIANT1 with *Hordeum vulgare* codon usage

atgaatagcacctccatgtcctctctgggcgttcgtaaggggtcatggacagatgaggaggacttcttgctccgcaaatgcatcgacaagtatg
gcgaaggcaaatggcatcttgtcccgataagggccggactcaaccgctgcagaaagtcttgccgccttaggtggctaaactacctacggcc
ccacattaagcggggtgactttgagcaggatgaggtagacttgatcttgcggctacacaagcttctgggcaataggtggtcactgattgccggt
agactccctggtcgcactgcgaatgacgtgaagaactactggaacaccaatctgctccgcaaactcaacaccaccaagatcgtcccacgt
gagaagatcaacaacaagtgtggcgagatcagcaccaagatcgagatcatcaagccacaacggaggaagtacttctcctctacgatgaa
gaatgtgacgaacaacaacgtgattctcgacgaagaggagcactgtaaggagatcatctccgagaaacagactcccgatgcttcgatgga
caatgtcgatccgtggtggattaacctcctggagaattgcaacgatgacatagaagaggacgaagaagtcgtgatcaactacgaaaagac
gctgacaagcctcttgcacgaggagatatcgccacccctcaacattggagaggggaacagcatgcagcaagggcagatcagtcatgaaa
actggggagagttcagcctcaatcttcctccgatgcagcaaggcgttcagaacgatgacttcagtgcagagattgacctgtggaaccttctcg
attaa

**SEQ ID NO: 20**
>SIANT1 with Bifidobacterium codon usage

atgaactccacctccatgtcctcgctcggcgttcgcaaaggcagctggaccgatgaggaggacttcctcctgcgcaagtgcatcgacaagta
cggagaaggcaaatggcaccttgtccccattcgcgctggtctgaaccgctgtcgcaagagctgccgtttgcggtggctgaactatctgcgtcc
gcacatcaagcgcggcgacttcgagcaggacgaagtcgacctgattctgcgcctgcataagctgctggggaaccgctggtccctgattgcc
ggccggttgcccggtaggaccgcgaacgacgtgaagaactactggaacaccaacctccttcgcaagctgaataccacgaagatcgtgcc
gagggagaagatcaacaacaaatgcggggaaatctcgacgaagatcgagatcatcaagccccaacgtcggaagtacttcagcagcacc
atgaagaacgtgacgaacaacaacgtgatcctggacgaagaggaacactgcaaggagatcatctcggagaagcagactccggatgcct
ccatggacaacgtggatccgtggtggatcaatctgctggagaactgcaacgacgacatcgaggaggatgaggaagtcgtgatcaactacg
aaaagaccttgacgtccctcctccatgaggagatttcccctccgctgaacatcggcgagggcaactccatgcaacagggccagatctccca
cgagaattggggcgaattctcgctgaatctcccgccgatgcagcagggagtccagaacgacgactttagcgccgaaatcgacctctggaa
ccttctcgattaa

**SEQ ID NO: 21**
>SILOG1 with Oryza sativa codon usage

atggagaacaaccatcaaacgcagattcagactaccaagacttctcgcttcaagcgcatttgcgtgttctgtgggtcaagtccaggcaagaa
gccctcctatcagcttgctgccatccagctggggaatcagctggttgaacggaatatcgatctcgtctatggtggaggctctgttggcctaatgg
gactcgtgagccaatccgtgttcaatggtggtcgacatgtcctcggcgtgataccgaaaccctgatgcccagagagatcacgggagagtc
agtcggagaagtccgggctgtttctggcatgcatcagaggaaagccgagatggcacgtcaagccgatgcgtttatagcgcttcctggcggtt
acggaaccctcgaagagctactggaggtgattacatgggctcagttgggcatacacgacaaaccagttggcctcttgaacgtggatgggta
ctacaactcgttgctttcgttcatcgacaaggcagtagacgaggggtttgtgacaccatccgcaagacacatcatcattagtgcgcctacagcc

caagaactcatgagcaagcttgaggactatgtcccgaagcacaatggggtagccccgaaactgagctgggagatggaacaacagctcg
gctacacgactaccaagctcgagattgcgaggtga

**SEQ ID NO: 22**
>SIOVATE with Oryza sativa codon usage

atgggcaaaagtctcaagctgcgcttttctcgtgtgattgccagcttcaattcgtgcagatctaagaatcccagctcacttccgcaaaatccgaa
cttctttccccacaagcttacatcgacaaaacacatctctccagactttccgctgattgaccagaaccagaaccagaatcacaggaactacgt
tcctgagtcgaccatgatcagtgtgggctgttgcagatccgaattcaagtgggagaaagaggagaagtttcacgtggtatcaagctcgttcgttt
ccgaggaagaggagtgtgaagaagagatcaaccttgctctacgtccaccgctaacaccaccgcgcttctcaaggatagttgtcgagaaga
agaagaagaaacagcaacgggtgaagaaaacgaaaaccaaatcccgcatcattcgcatgtccacttcatctgcggatgagtacagtggg
atcttgagcggtaccaacacagattgggacaacaatgaggaggaaaccgaaagtctggtgtccagctcaaggagctgttacgacttctcga
gtgatgactcgtccacggatttcaatccgcatttggagactatttgcgaaactacgacaatgagaaggcggcataaaaggaatgccaacac
gaagcgacgctctatcaaacaaagccgaccttcattctcctcaagcaagggacgcagaagctccgtgtcgacctcctcagactctgagctcc
cagctaggctcagtgtctttaagaagctcattccttgctctgtggatggaaaggtcaaggagtccttcgcaatcgtcaagaaatcgcaagatcc
ctatgaggacttcaagcggtctatgatggagatgatcctggagaaggaaatgtttgagaagaatgagctcgaacagcttctccagtgcttcctc
tccctcaacggcaagcattaccatggtgtcatagttgaagcgtttagcgacatatgggaaacgctgttcttggggaataacgatcgggtacgtc
gaatgagcattcacgatcctactcccacctattgccggtga

**SEQ ID NO: 23**
>pNMD674

cttctgtcagcgggcccactgcatccaccccagtacattaaaaacgtccgcaatgtgttattaagttgtctaagcgtcaatttgtttacaccacaat
atatcctgccaccagccagccaacagctccccgaccggcagctcggcacaaaatcaccactcgatacaggcagcccatcagtcagatca
ggatctcctttgcgacgctcaccgggctggttgccctcgccgctgggctggcggccgtctatggccctgcaaacgcgccagaaacgccgtcg
aagccgtgtgcgagacaccgcggccgccggcgttgtggatacctcgcggaaaacttggccctcactgacagatgaggggcggacgttga
cacttgaggggccgactcacccggcgcggcgttgacagatgaggggcaggctcgatttcggccggcgacgtggagctggccagcctcgc
aaatcggcgaaaacgcctgattttacgcgagtttcccacagatgatgtggacaagcctggggataagtgccctgcggtattgacacttgagg
ggcgcgactactgacagatgaggggcgcgatccttgacacttgaggggcagagtgctgacagatgaggggcgcacctattgacatttgag
gggctgtccacaggcagaaaatccagcatttgcaagggtttccgcccgtttttcggccaccgctaacctgtcttttaacctgcttttaaaccaatat
ttataaaccttgtttttaaccagggctgcgccctgtgcgcgtgaccgcgcacgccgaaggggggtgcccccccttctcgaaccctcccggccc
gctaacgcgggcctcccatccccccaggggctgcgcccctcggccgcgaacggcctcaccccaaaaatggcagcgctggccaattcgtg
cgcggaacccctatttgtttatttttctaaatacattcaaatatgtatccgctcatgagacaataaccctgataaatgcttcaataatattgaaaaag
gaagagtatggctaaaatgagaatatcaccggaattgaaaaaactgatcgaaaaataccgctgcgtaaaagatacggaaggaatgtctcc
tgctaaggtatataagctggtgggagaaaatgaaaacctatatttaaaaatgacggacagccggtataaagggaccacctatgatgtggaa
cgggaaaaggacatgatgctatggctggaaggaaagctgcctgttccaaaggtcctgcactttgaacggcatgatggctggagcaatctgct
catgagtgaggccgatggcgtcctttgctcggaagagtatgaagatgaacaaagccctgaaaagattatcgagctgtatgcggagtgcatca
ggctctttcactccatcgacatatcggattgtccctatacgaatagcttagacagccgcttagccgaattggattacttactgaataacgatctgg
ccgatgtggattgcgaaaactgggaagaagacactccatttaaagatccgcgcgagctgtatgatttttttaaagacggaaaagcccgaaga
ggaacttgtcttttcccacggcgacctgggagacagcaacatctttgtgaaagatggcaaagtaagtggctttattgatcttgggagaagcggc
agggcggacaagtggtatgacattgccttctgcgtccggtcgatcagggaggatatcggggaagaacagtatgtcgagctatttttgacttact
ggggatcaagcctgattgggagaaaataaaatattatattttactggatgaattgttttagctgtcagaccaagtttactcatatatactttagattg
atttaaaacttcatttttaatttaaaaggatctaggtgaagatcctttttgataatctcatgaccaaaatcccttaacgtgagttttcgttccactgagc
gtcagaccccgtagaaaagatcaaaggatcttcttgagatcctttttttctgcgcgtaatctgctgcttgcaaacaaaaaaaccaccgctacca
gcggtggtttgtttgccggatcaagagctaccaactctttttccgaaggtaactggcttcagcagagcgcagataccaaatactgtccttctagtg
tagccgtagttaggccaccacttcaagaactctgtagcaccgcctacatacctcgctctgctaatcctgttaccagtggctgctgccagtggcg
ataagtcgtgtcttaccgggttggactcaagacgatagttaccggataaggcgcagcggtcgggctgaacggggggttcgtgcacacagcc
cagcttggagcgaacgacctacaccgaactgagatacctacagcgtgagctatgagaaagcgccacgcttcccgaagggagaaaggcg
gacaggtatccggtaagcggcagggtcggaacaggagagcgcacgagggagcttccagggggaaacgcctggtatctttatagtcctgtc
gggtttcgccacctctgacttgagcgtcgatttttgtgatgctcgtcaggggggcggagcctatggaaaaacgccagcaacgcggcctttttac
ggttcctggcagatcctagatgtggcgcaacgatgccggcgacaagcaggagcgcaccgacttcttccgcatcaagtgttttggctctcaggc
cgaggcccacggcaagtatttggcaaggggtcgctggtattcgtgcagggcaagattcggaataccaagtacgagaaggacggccaga
cggtctacgggaccgacttcattgccgataaggtggattatctggacaccaaggcaccaggcgggtcaaatcaggaataagggcacattgc

cccggcgtgagtcggggcaatcccgcaaggagggtgaatgaatcggacgtttgaccggaaggcatacaggcaagaactgatcgacgcg
gggttttccgccgaggatgccgaaaccatcgcaagccgcaccgtcatgcgtgcgccccgcgaaaccttccagtccgtcggctcgatggtcc
agcaagctacggccaagatcgagcgcgacagcgtgcaactggctccccctgccctgcccgcgccatcggccgccgtggagcgttcgcgt
cgtctcgaacaggaggcggcaggtttggcgaagtcgatgaccatcgacacgcgaggaactatgacgaccaagaagcgaaaaaccgcc
ggcgaggacctggcaaaacaggtcagcgaggccaagcaggccgcgttgctgaaacacacgaagcagcagatcaaggaaatgcagctt
tccttgttcgatattgcgccgtggccggacacgatgcgagcgatgccaaacgacacggcccgctctgccctgttcaccacgcgcaacaaga
aaatcccgcgcgaggcgctgcaaaacaaggtcattttccacgtcaacaaggacgtgaagatcacctacaccggcgtcgagctgcgggcc
gacgatgacgaactggtgtggcagcaggtgttggagtacgcgaagcgcacccctatcggcgagccgatcaccttcacgttctacgagctttg
ccaggacctgggctggtcgatcaatggccggtattacacgaaggccgaggaatgcctgtcgcgcctacaggcgacggcgatgggcttcac
gtccgaccgcgttgggcacctggaatcggtgtcgctgctgcaccgcttccgcgtcctggaccgtggcaagaaaacgtcccgttgccaggtcct
gatcgacgaggaaatcgtcgtgctgtttgctggcgaccactacacgaaattcatatgggagaagtaccgcaagctgtcgccgacggcccga
cggatgttcgactatttcagctcgcaccgggagccgtacccgctcaagctggaaaccttccgcctcatgtgcggatcggattccacccgcgtg
aagaagtggcgcgagcaggtcggcgaagcctgcgaagagttgcgaggcagcggcctggtggaacacgcctgggtcaatgatgacctgg
tgcattgcaaacgctagggccttgtggggtcagttccggctgggggttcagcagccagcgcctgatctggggaaccctgtggttggcacatac
aaatggacgaacggataaaccttttcacgcccttttaaatatccgattattctaataaacgctcttttctcttaggtttacccgccaatatatcctgtc
aaacactgatagtttaaactgaaggcgggaaacgacaatctgatctaagctaggcatgcctgcaggtcaacatggtggagcacgacacgc
ttgtctactccaaaaatatcaaagatacagtctcagaagaccaaagggcaattgagacttttcaacaaagggtaatatccggaaacctcctc
ggattccattgcccagctatctgtcactttattgtgaagatagtggaaaaggaaggtggctcctacaaatgccatcattgcgataaaggaaagg
ccatcgttgaagatgcctctgccgacagtggtcccaaagatggacccccacccacgaggagcatcgtggaaaaagaagacgttccaacc
acgtcttcaaagcaagtggattgatgtgatatctccactgacgtaagggatgacgcacaatcccactatccttcgcaagacccttcctctatata
aggaagttcatttcatttggagaggagaaaactaaaccatacaccaccaacacaaccaaacccaccacgcccaattgttacacacccgctt
gaaaaagaaagtttaacaaatggccaaggtgcgcgaggtttaccaatcttttacagactccaccacaaaaactctcatccaagatgaggctt
atagaaacattcgccccatcatggaaaaacacaaactagctaacccttacgctcaaacggttgaagcggctaatgatctagaggggttcgg
catagccaccaatccctatagcattgaattgcatacacatgcagccgctaagaccatagagaataaacttctagaggtgcttggttccatccta
ccacaagaacctgttacatttatgtttcttaaacccagaaagctaaactacatgagaagaaacccgcggatcaaggacattttccaaaatgtt
gccattgaaccaagagacgtagccaggtaccccaaggaaacaataattgacaaactcacagagatcacaacggaaacagcatacatta
gtgacactctgcacttcttggatccgagctacatagtggagacattccaaaactgcccaaaattgcaaacattgtatgcgaccttagttctcccc
gttgaggcagcctttaaaatggaaagcactcacccgaacatatacagcctcaaatacttcggagatggtttccagtatataccaggcaaccat
ggtggcggggcataccatcatgaattcgctcatctacaatggctcaaagtgggaaagatcaagtggagggaccccaaggatagctttctcg
gacatctcaattacacgactgagcaggttgagatgcacacagtgacagtacagttgcaggaatcgttcgcggcaaaccacttgtactgcatc
aggagaggagacttgctcacaccggaggtgcgcactttcggccaacctgacaggtacgtgattccaccacagatcttcctcccaaaagttca
caactgcaagaagccgattctcaagaaaactatgatgcagctcttcttgtatgttaggacagtcaaggtcgcaaaaaattgtgacatttttgcca
aagtcagacaattaattaaatcatctgacttggacaaatactctgctgtggaactggtttacttagtaagctacatggagttccttgccgatttaca
agctaccacctgcttctcagacacactttctggtggcttgctaacaaagacccttgcaccggtgagggcttggatacaagagaaaaagatgc
agctgtttggtcttgaggactacgcgaagttagtcaaagcagttgatttccacccggtggatttttctttcaaagtggaaacttgggacttcagattc
cacccettgcaagcgtggaaagccttccgaccaagggaagtgtcggatgtagaggaaatggaaagtttgttctcagatggggacctgcttga
ttgcttcacaagaatgccagcttatgcggtaaacgcagaggaagatttagctgcaatcaggaaaacgcccgagatggatgtcggtcaagaa
gttaaagagcctgcaggagacagaaatcaatactcaaaccctgcagaaactttcctcaacaagctccacaggaaacacagtagggaggt
gaaacaccaggccgcaaagaaagctaaacgcctagctgaaatccaggagtcaatgagagctgaaggtgatgccgaaccaaatgaaat
aagcgggacgatgggggcaatacccagcaacgccgaacttcctggcacgaatgatgccagacaagaactcacactcccaaccactaaa
cctgtccctgcaaggtgggaagatgcttcattcacagattctagtgtggaagaggagcaggttaaactccttggaaaagaaaccgttgaaac
agcgacgcaacaagtcatcgaaggacttccttggaaacactggattcctcaattaaatgctgttggattcaaggcgctggaaattcagaggg
ataggagtggaacaatgatcatgcccatcacagaaatggtgtccgggctggaaaaagaggacttccctgaaggaactccaaaagagttgg
cacgagaattgttcgctatgaacagaagccctgccaccatccctttggacctgcttagagccagagactacggcagtgatgtaaagaacaa
gagaattggtgccatcacaaagacacaggcaacgagttggggcgaatacttgacaggaaagatagaaagcttaactgagaggaaagttg
cgacttgtgtcattcatggagctggaggttctggaaaaagtcatgccatccagaaggcattgagagaaattggcaagggctcggacatcact
gtagtcctgccgaccaatgaactgcggctagattggagtaagaaagtgcctaacactgagccctatatgttcaagacctctgaaaaggcgtt
aattgggggaacaggcagcatagtcatctttgacgattactcaaaacttcctcccggttacatagaagccttagtctgtttctactctaaaatcaa
gctaatcattctaacaggagatagcagacaaagcgtctaccatgaaactgctgaggacgcctccatcaggcatttgggaccagcaacaga
gtacttctcaaaatactgccgatactatctcaatgccacacaccgcaacaagaaagatcttgcgaacatgcttggtgtctacagtgagagaac
gggagtcaccgaaatcagcatgagcgccgagttcttagaaggaatcccaactttggtaccctcggatgagaagagaaagctgtacatggg

caccgggaggaatgacacgttcacatacgctggatgccaggggctaactaagccgaaggtacaaatagtgttggaccacaacacccaag
tgtgtagcgcgaatgtgatgtacacggcactttctagagccaccgataggattcacttcgtgaacacaagtgcaaattcctctgccttctgggaa
aagttggacagcaccccttacctcaagactttcctatcagtggtgagagaacaagcactcagggagtacgagccggcagaggcagagcc
aattcaagagcctgagccccagacacacatgtgtgtcgagaatgaggagtccgtgctagaagagtacaaagaggaactcttggaaaagttt
gacagagagatccactctgaatcccatggtcattcaaactgtgtccaaactgaagacacaaccattcagttgttttcgcatcaacaagcaaaa
gatgagactctcctctgggcgactatagatgcgcggctcaagaccagcaatcaagaaacaaacttccgagaattcctgagcaagaaggac
attggggacgttctgtttttaaactaccaaaaagctatgggtttacccaaagagcgtattcctttttcccaagaggtctgggaagcttgtgcccac
gaagtacaaagcaagtacctcagcaagtcaaagtgcaacttgatcaatgggactgtgagacagagcccagacttcgatgaaaataagatt
atggtattcctcaagtcgcagtgggtcacaaaggtggaaaaactaggtctacccaagattaagccaggtcaaaccatagcagccttttacca
gcagactgtgatgctttttggaactatggctaggtacatgcgatggttcagacaggctttccagccaaaagaagtcttcataaactgtgagacg
acgccagatgacatgtctgcatgggccttgaacaactggaatttcagcagacctagcttggctaatgactacacagctttcgaccagtctcag
gatggagccatgttgcaatttgaggtgctcaaagccaaacaccactgcataccagaggaaatcattcaggcatacatagatattaagactaa
tgcacagattttcctaggcacgttatcaattatgcgcctgactggtgaaggtcccacttttgatgcaaacactgagtgcaacatagcttacaccc
atacaaagtttgacatcccagccggaactgctcaagtttatgcaggagacgactccgcactggactgtgttccagaagtgaagcatagtttcc
acaggcttgaggacaaattactcctaaagtcaaagcctgtaatcacgcagcaaaagaagggcagttggcctgagttttgtggttggctgatca
caccaaaaggggtgatgaaagacccaattaagctccatgttagcttaaaattggctgaagctaagggtgaactcaagaaatgtcaagattcc
tatgaaattgatctgagttatgcctatgaccacaaggactctctgcatgacttgttcgatgagaaacagtgtcaggcacacacactcacttgcag
aacactaatcaagtcagggagaggcactgtctcactttcccgcctcagaaactttctttaaccgttaagttaccttagagatttgaataagatgtc
agcaccagctagtacaacacagcccatagggtcaactacctcaactaccacaaaaactgcaggcgcaactcctgccacagcttcaggcct
gttcactatcccggatggggatttctttagtacagcccgtgccatagtagccagcaatgctgtcgcaacaaatgaggacctcagcaagattga
ggctatttggaaggacatgaaggtgcccacagacactatggcacaggctgcttgggacttagtcagacactgtgctgatgtaggatcatccg
ctcaaacagaaatgatagatacaggtccctattccaacggcatcagcagagctagactggcagcagcaattaaagaggtgtgcacactta
ggcaattttgcatgaagtatgccccagtggtatggaactggatgttaactaacaacagtccacctgctaactggcaagcacaaggtttcaagc
ctgagcacaaattcgctgcattcgacttcttcaatggagtcaccaacccagctgccatcatgcccaaagaggggctcatccggccaccgtctg
aagctgaaatgaatgctgcccaaactgctgcctttgtgaagattacaaaggccagggcacaatccaacgactttgccagcctagatgcagct
gtcactcgaggaaggatcaccggaacgaccacagcagaggcagtcgttactctgcctcctccataacagaaactttctttaaccgttaagtta
ccttagagatttgaataagatggatattctcatcagtagtttgaaaagtttaggttattctaggacttccaaatctttagattcaggacctttggtagta
catgcagtagccggagccggtaagtccacagccctaaggaagttgatcctcagacacccaacattcaccgtgcatacactcggtgtccctg
acaaggtgagtatcagaactagaggcatacagaagccaggacctattcctgagggcaacttcgcaatcctcgatgagtatactttggacaa
caccacaaggaactcataccaggcactttttgctgacccttatcaggcaccggagtttagcctagagccccacttctacttggaaacatcatttc
gagttccgaggaaagtggcagatttgatagctggctgtggcttcgatttcgagacgaactcaccggaagaagggcacttagagatcactggc
atattcaaagggcccctactcggaaaggtgatagccattgatgaggagtctgagacaacactgtccaggcatggtgttgagtttgttaagccct
gccaagtgacgggacttgagttcaaagtagtcactattgtgtctgccgcaccaatagaggaaattggccagtccacagctttctacaacgctat
caccaggtcaaagggattgacatatgtccgcgcagggccataggctgaccgctccggtcaattctgaaaaagtgtacatagtattaggtctat
catttgctttagtttcaattacctttctgctttctagaaatagcttaccccacgtcggtgacaacattcacagcttgccacacggaggagcttacag
agacggcaccaaagcaatcttgtacaactccccaaatctagggtcacgagtgagtctacacaacggaaagaacgcagcatttgctgccgtt
ttgctactgactttgctgatctatggaagtaaatacatatctcaacgcaatcatacttgtgcttgtggtaacaatcatagcagtcattagcacttcctt
agtgaggactgaaccttgtgtcatcaagattactggggaatcaatcacagtgttggcttgcaaactagatgcagaaaccataagggccattgc
cgatctcaagccactctccgttgaacggttaagtttccattgatactcgaaagaggtcagcaccagctagcaacaaacaagaacatgagag
acctcgcgatttaaatcgatggtctcagatcggtcgtatcactggaacaacaaccgctgaggctgttgtcactctaccaccaccataactacgt
ctacataaccgacgcctaccccagtttcatagtattttctggtttgattgtatgaataatataaataaaaaaaaaaaaaaaaaaaaaaaactag
tgagct

**SEQ ID NO: 24**
>pNMD4300

aaactgaaggcgggaaacgacaatctgatctaagctaggcatgcctgcaggtcaacatggtggagcacgacacgcttgtctactccaaaa
atatcaaagatacagtctcagaagaccaaagggcaattgagacttttcaacaaagggtaatatccggaaacctcctcggattccattgccca
gctatctgtcactttattgtgaagatagtggaaaaggaaggtggctcctacaaatgccatcattgcgataaaggaaaggccatcgttgaagat
gcctctgccgacagtggtcccaaagatggacccccacccacgaggagcatcgtggaaaaagaagacgttccaaccacgtcttcaaagca
agtggattgatgtgatatctccactgacgtaagggatgacgcacaatcccactatccttcgcaagacccttcctctatataaggaagttcatttca
tttggagaggagaaaactaaaccatacaccaccaacacaaccaaacccaccacgcccaattgttacacacccgcttgaaaaagaaagttt

aacaaatggccaaggtgcgcgaggtttaccaatcttttacagactccaccacaaaaactctcatccaagatgaggcttatagaaacattcgc
cccatcatggaaaaacacaaactagctaacccttacgctcaaacggttgaagcggctaatgatctagaggggttcggcatagccaccaatc
cctatagcattgaattgcatacacatgcagccgctaagaccatagagaataaacttctagaggtgcttggttccatcctaccacaagaacctgt
tacatttatgtttcttaaacccagaaagctaaactacatgagaagaaacccgcggatcaaggacattttccaaaatgttgccattgaaccaag
agacgtagccaggtaccccaaggaaacaataattgacaaactcacagagatcacaacggaaacagcatacattagtgacactctgcactt
cttggatccgagctacatagtggagacattccaaaactgcccaaaattgcaaacattgtatgcgaccttagttctccccgttgaggcagccttta
aaatggaaagcactcacccgaacatatacagcctcaaatacttcggagatggtttccagtatataccaggcaaccatggtggcggggcata
ccatcatgaattcgctcatctacaatggctcaaagtgggaaagatcaagtggagggaccccaaggatagctttctcggacatctcaattacac
gactgagcaggttgagatgcacacagtgacagtacagttgcaggaatcgttcgcggcaaaccacttgtactgcatcaggagaggagacttg
ctcacaccggaggtgcgcactttcggccaacctgacaggtacgtgattccaccacagatcttcctcccaaaagttcacaactgcaagaagcc
gattctcaagaaaactatgatgcagctcttcttgtatgttaggacagtcaaggtcgcaaaaaattgtgacatttttgccaaagtcagacaattaat
taaatcatctgacttggacaaatactctgctgtggaactggtttacttagtaagctacatggagttccttgccgatttacaagctaccacctgcttct
cagacacactttctggtggcttgctaacaaagacccttgcaccggtgagggcttggatacaagagaaaaagatgcagctgtttggtcttgagg
actacgcgaagttagtcaaagcagttgatttccacccggtggatttttctttcaaagtggaaacttgggacttcagattccaccccttgcaagcgt
ggaaagccttccgaccaagggaagtgtcggatgtagaggaaatggaaagtttgttctcagatggggacctgcttgattgcttcacaagaatg
ccagcttatgcggtaaacgcagaggaagatttagctgcaatcaggaaaacgcccgagatggatgtcggtcaagaagttaaagagcctgca
ggagacagaaatcaatactcaaaccctgcagaaactttcctcaacaagctccacaggaaacacagtagggaggtgaaacaccaggccg
caaagaaagctaaacgcctagctgaaatccaggagtcaatgagagctgaaggtgatgccgaaccaaatgaaataagcgggacgatgg
gggcaatacccagcaacgccgaacttcctggcacgaatgatgccagacaagaactcacactcccaaccactaaacctgtccctgcaaggt
gggaagatgcttcattcacagattctagtgtggaagaggagcaggttaaactccttggaaaagaaaccgttgaaacagcgacgcaacaag
tcatcgaaggacttccttggaaacactggattcctcaattaaatgctgttggattcaaggcgctggaaattcagagggataggagtggaacaa
tgatcatgcccatcacagaaatggtgtccgggctggaaaaagaggacttccctgaaggaactccaaaagagttggcacgagaattgttcgc
tatgaacagaagccctgccaccatccctttggacctgcttagagccagagactacggcagtgatgtaaagaacaagagaattggtgccatc
acaaagacacaggcaacgagttggggcgaatacttgacaggaaagatagaaagcttaactgagaggaaagttgcgacttgtgtcattcat
ggagctggaggttctggaaaaagtcatgccatccagaaggcattgagagaaattggcaagggctcggacatcactgtagtcctgccgacc
aatgaactgcggctagattggagtaagaaagtgcctaacactgagccctatatgttcaagacctctgaaaaggcgttaattgggggaacag
gcagcatagtcatctttgacgattactcaaaacttcctcccggttacatagaagccttagtctgtttctactctaaaatcaagctaatcattctaaca
ggagatagcagacaaagcgtctaccatgaaactgctgaggacgcctccatcaggcatttgggaccagcaacagagtacttctcaaaatact
gccgatactatctcaatgccacacaccgcaacaagaaagatcttgcgaacatgcttggtgtctacagtgagagaacggagtcaccgaaat
cagcatgagcgccgagttcttagaaggaatcccaactttggtaccctcggatgagaagagaaagctgtacatgggcaccgggaggaatga
cacgttcacatacgctggatgccaggggctaactaagccgaaggtacaaatagtgttggaccacaacacccaagtgtgtagcgcgaatgtg
atgtacacggcactttctagagccaccgataggattcacttcgtgaacacaagtgcaaattcctctgccttctgggaaaagttggacagcacc
ccttacctcaagactttcctatcagtggtgagagaacaagcactcagggagtacgagccggcagaggcagagccaattcaagagcctgag
ccccagacacacatgtgtgtcgagaatgaggagtccgtgctagaagagtacaaagaggaactcttggaaaagtttgacagagagatccac
tctgaatcccatggtcattcaaactgtgtccaaactgaagacacaaccattcagttgttttcgcatcaacaagcaaaagatgagactctcctctg
ggcgactatagatgcgcggctcaagaccagcaatcaagaaacaaacttccgagaattcctgagcaagaaggacattggggacgttctgttt
ttaaactaccaaaaagctatgggtttacccaaagagcgtattcctttttcccaagaggtctgggaagcttgtgcccacgaagtacaaagcaag
tacctcagcaagtcaaagtgcaacttgatcaatgggactgtgagacagagcccagacttcgatgaaaataagattatggtattcctcaagtcg
cagtgggtcacaaaggtggaaaaactaggtctacccaagattaagccaggtcaaaccatagcagccttttaccagcagactgtgatgcttttt
ggaactatggctaggtacatgcgatggttcagacaggctttccagccaaaagaagtcttcataaactgtgagacgacgccagatgacatgtc
tgcatgggccttgaacaactggaatttcagcagacctagcttggctaatgactacacagctttcgaccagtctcaggatggagccatgttgcaa
tttgaggtgctcaaagccaaacaccactgcataccagaggaaatcattcaggcatacatagatattaagactaatgcacagattttcctaggc
acgttatcaattatgcgcctgactggtgaaggtcccacttttgatgcaaacactgagtgcaacatagcttacacccatacaaagtttgacatccc
agccggaactgctcaagtttatgcaggagacgactccgcactggactgtgttccagaagtgaagcatagtttccacaggcttgaggacaaat
tactcctaaagtcaaagcctgtaatcacgcagcaaaagaagggcagttggcctgagttttgtggttggctgatcacaccaaaaggggtgatg
aaagacccaattaagctccatgttagcttaaaattggctgaagctaagggtgaactcaagaaatgtcaagattcctatgaaattgatctgagtt
atgcctatgaccacaaggactctctgcatgacttgttcgatgagaaacagtgtcaggcacacacactcacttgcagaacactaatcaagtca
gggagaggcactgtctcactttcccgcctcagaaactttctttaaccgttaagttaccttagagatttgaataagatgtcagcaccagctagtaca
acacagcccatagggtcaactacctcaactaccacaaaaactgcaggcgcaactcctgccacagcttcaggcctgttcactatcccggatg
gggatttctttagtacagcccgtgccatagtagccagcaatgctgtcgcaacaaatgaggacctcagcaagattgaggctatttggaaggac
atgaaggtgcccacagacactatggcacaggctgcttgggacttagtcagacactgtgctgatgtaggatcatccgctcaaacagaaatgat

agatacaggtccctattccaacggcatcagcagagctagactggcagcagcaattaaagaggtgtgcacacttaggcaattttgcatgaagt
atgccccagtggtatggaactggatgttaactaacaacagtccacctgctaactggcaagcacaaggtttcaagcctgagcacaaattcgct
gcattcgacttcttcaatggagtcaccaacccagctgccatcatgcccaaagaggggctcatccggccaccgtctgaagctgaaatgaatgc
tgcccaaactgctgcctttgtgaagattacaaaggccagggcacaatccaacgactttgccagcctagatgcagctgtcactcgaggaagg
atcaccggaacgaccacagcagaggcagtcgttactctgcctcctccataacagaaactttctttaaccgttaagttaccttagagatttgaata
agatggatattctcatcagtagtttgaaaagtttaggttattctaggacttccaaatctttagattcaggacctttggtagtacatgcagtagccgga
gccggtaagtccacagccctaaggaagttgatcctcagacacccaacattcaccgtgcatacactcggtgtccctgacaaggtgagtatcag
aactagaggcatacagaagccaggacctattcctgagggcaacttcgcaatcctcgatgagtatactttggacaacaccacaaggaactca
taccaggcacttttgctgacccttatcaggcaccggagtttagcctagagccccacttctacttggaaacatcatttcgagttccgaggaaagt
ggcagatttgatagctggctgtggcttcgatttcgagacgaactcaccggaagaagggcacttagagatcactggcatattcaaaggcccc
tactcggaaaggtgatagccattgatgaggagtctgagacaacactgtccaggcatggtgttgagtttgttaagccctgccaagtgacgggac
ttgagttcaaagtagtcactattgtgtctgccgcaccaatagaggaaattggccagtccacagctttctacaacgctatcaccaggtcaaaggg
attgacatatgtccgcgcagggccataggctgaccgctccggtcaattctgaaaaagtgtacatagtattaggtctatcatttgctttagtttcaatt
acctttctgctttctagaaatagcttaccccacgtcggtgacaacattcacagcttgccacacggaggagcttacagagacggcaccaaagc
aatcttgtacaactccccaaatctagggtcacgagtgagtctacacaacggaaagaacgcagcatttgctgccgttttgctactgactttgctga
tctatggaagtaaatacatatctcaacgcaatcatacttgtgcttgtggtaacaatcatagcagtcattagcacttccttagtgaggactgaacctt
gtgtcatcaagattactggggaatcaatcacagtgttggcttgcaaactagatgcagaaaccataagggccattgccgatctcaagccactct
ccgttgaacggttaagtttccattgatactcgaaagaggtcagcaccagctagcaacaaacaagaacatgagagacctcgcgatttaaatc
gatggtctcagatcggtcgtatcactggaacaacaaccgctgaggctgttgtcactctaccaccaccataactacgtctacataaccgacgcc
taccccagtttcatagtattttctggtttgattgtatgaataatataaataaaaaaaaaaaaaaaaaaaaaaaactagtgagctcttctgtcagcg
ggcccactgcatccaccccagtacattaaaaacgtccgcaatgtgttattaagttgtctaagcgtcaatttgtttacaccacaatatatcctgcca
ccagccagccaacagctccccgaccggcagctcggcacaaaatcaccactcgatacaggcagcccatcagtcagatcaggatctcctttg
cgacgctcaccgggctggttgccctcgccgctgggctggcggccgtctatggccctgcaaacgcgccagaaacgccgtcgaagccgtgtg
cgagacaccgcggccgccggcgttgtggatacctcgcggaaaacttggccctcactgacagatgaggggcggacgttgacacttgaggg
gccgactcacccggcgcggcgttgacagatgaggggcaggctcgatttcggccggcgacgtggagctggccagcctcgcaaatcggcga
aaacgcctgatttacgcgagtttcccacagatgatgtggacaagcctggggataagtgccctgcggtattgacacttgaggggcgcgactac
tgacagatgaggggcgcgatccttgacacttgaggggcagagtgctgacagatgaggggcgcacctattgacatttgaggggctgtccaca
ggcagaaaatccagcatttgcaagggtttccgcccgtttttcggccaccgctaacctgtcttttaacctgcttttaaaccaatatttataaaccttgtt
tttaaccagggctgcgccctgtgcgcgtgaccgcgcacgccgaaggggggtgcccccccttctcgaaccctcccggcccgctaacgcggg
cctcccatccccccaggggctgcgcccctcggccgcgaacggcctcaccccaaaaatggcagcctgtcgatcagatctggctcgcggcgg
acgcacgacgccggggcgagaccataggcgatctcctaaatcaatagtagctgtaacctcgaagcgtttcacttgtaacaacgattgagaat
ttttgtcataaaattgaaatacttggttcgcatttttgtcatccgcggtcagccgcaattctgacgaactgcccatttagctggagatgattgtacatc
cttcacgtgaaaatttctcaagtgctgtgaacaagggttcagattttagattgaaaggtgagccgttgaaacacgttcttcttgtcgatgacgacgt
cgctatgcggcatcttattattgaataccttacgatccacgccttcaaagtgaccgcggtagccgacagcacccagttcacaagagtactctctt
ccgcgacggtcgatgtcgtggttgttgatctagatttaggtcgtgaagatgggctcgagatcgttcgtaatctggcggcaaagtctgatattccaa
tcataattatcagtggcgaccgccttgaggagacggataaagttgttgcactcgagctaggagcaagtgattttatcgctaagccgttcagtatc
agagagtttctagcacgcattcgggttgccttgcgcgtgcgccccaacgttgtccgctccaaagaccgacggtctttttgttttactgactggaca
cttaatctcaggcaacgtcgcttgatgtccgaagctggcggtgaggtgaaacttacggcaggtgagttcaatcttctcctcgcgtttttagagaa
accccgcgacgttctatcgcgcgagcaacttctcattgccagtcgagtacgcgacgaggaggtttatgacaggagtatagatgttctcattttga
ggctgcgccgcaaacttgaggcggatccgtcaagccctcaactgataaaaacagcaagaggtgccggttatttctttgacgcggacgtgca
ggtttcgcacgggggacgatggcagcctaagatcgacaggctggccaattcgtgcgcggaacccctatttgtttatttttctaaatacattcaa
atatgtatccgctcatgagacaataaccctgataaatgcttcaataatattgaaaaaggaagagtatggctaaaatgagaatatcaccggaat
tgaaaaaactgatcgaaaaataccgctgcgtaaaagatacggaaggaatgtctcctgctaaggtatataagctggtgggagaaaatgaaa
acctatatttaaaaatgacggacagccggtataaagggaccacctatgatgtggaacgggaaaaggacatgatgctatggctggaaggaa
agctgcctgttccaaaggtcctgcactttgaacggcatgatggctggagcaatctgctcatgagtgaggccgatggcgtcctttgctcggaaga
gtatgaagatgaacaaagccctgaaaagattatcgagctgtatgcggagtgcatcaggctctttcactccatcgacatatcggattgtccctat
acgaatagcttagacagccgcttagccgaattggattacttactgaataacgatctggccgatgtggattgcgaaaactgggaagaagacac
tccatttaaagatccgcgcgagctgtatgatttttttaaagacggaaaagcccgaagaggaacttgtcttttcccacggcgacctgggagacag
caacatctttgtgaaagatggcaaagtaagtggctttattgatcttgggagaagcggcagggcggacaagtggtatgacattgccttctgcgtc
cggtcgatcagggaggatatcggggaagaacagtatgtcgagctattttttgacttactggggatcaagcctgattgggagaaaataaaatatt
atattttactggatgaattgttttagctgtcagaccaagtttactcatatatactttagattgatttaaaacttcatttttaatttaaaaggatctaggtga

agatcctttttgataatctcatgaccaaaatcccttaacgtgagttttcgttccactgagcgtcagaccccgtagaaaagatcaaaggatcttcttg agatcctttttttctgcgcgtaatctgctgcttgcaaacaaaaaaaccaccgctaccagcggtggtttgtttgccggatcaagagctaccaactct ttttccgaaggtaactggcttcagcagagcgcagataccaaatactgtccttctagtgtagccgtagttaggccaccacttcaagaactctgtag caccgcctacatacctcgctctgctaatcctgttaccagtggctgctgccagtggcgataagtcgtgtcttaccgggttggactcaagacgatag ttaccggataaggcgcagcggtcgggctgaacggggggttcgtgcacacagcccagcttggagcgaacgacctacaccgaactgagata cctacagcgtgagctatgagaaagcgccacgcttcccgaagggagaaaggcggacaggtatccggtaagcggcagggtcggaacagg agagcgcacgagggagcttccagggggaaacgcctggtatctttatagtcctgtcgggtttcgccacctctgacttgagcgtcgatttttgtgatg ctcgtcaggggggcggagcctatggaaaaacgccagcaacgcggcctttttacggttcctggcagatcctagatgtggcgcaacgatgccg gcgacaagcaggagcgcaccgacttcttccgcatcaagtgttttggctctcaggccgaggcccacggcaagtatttgggcaaggggtcgct ggtattcgtgcagggcaagattcggaataccaagtacgagaaggacggccagacggtctacgggaccgacttcattgccgataaggtgga ttatctggacaccaaggcaccaggcgggtcaaatcaggaataagggcacattgccccggcgtgagtcggggcaatcccgcaaggagggt gaatgaatcggacgtttgaccggaaggcatacaggcaagaactgatcgacgcggggttttccgccgaggatgccgaaaccatcgcaagc cgcaccgtcatgcgtgcgccccgcgaaaccttccagtccgtcggctcgatggtccagcaagctacggccaagatcgagcgcgacagcgtg caactggctccccctgccctgcccgcgccatcggccgccgtggagcgttcgcgtcgtctcgaacaggaggcggcaggtttggcgaagtcga tgaccatcgacacgcgaggaactatgacgaccaagaagcgaaaaaccgccggcgaggacctggcaaaacaggtcagcgaggccaa gcaggccgcgttgctgaaacacacgaagcagcagatcaaggaaatgcagctttccttgttcgatattgcgccgtggccggacacgatgcga gcgatgccaaacgacacggcccgctctgccctgttcaccacgcgcaacaagaaaatcccgcgcgaggcgctgcaaaacaaggtcattttc cacgtcaacaaggacgtgaagatcacctacaccggcgtcgagctgcgggccgacgatgacgaactggtgtggcagcaggtgttggagta cgcgaagcgcacccctatcggcgagccgatcaccttcacgttctacgagctttgccaggacctgggctggtcgatcaatggccggtattaca cgaaggccgaggaatgcctgtcgcgcctacaggcgacggcgatgggcttcacgtccgaccgcgttgggcacctggaatcggtgtcgctgct gcaccgcttccgcgtcctggaccgtggcaagaaaacgtcccgttgccaggtcctgatcgacgaggaaatcgtcgtgctgtttgctggcgacc actacacgaaattcatatgggagaagtaccgcaagctgtcgccgacggcccgacggatgttcgactatttcagctcgcaccgggagccgta cccgctcaagctggaaaccttccgcctcatgtgcggatcggattccacccgcgtgaagaagtggcgcgagcaggtcggcgaagcctgcga agagttgcgaggcagcggcctggtggaacacgcctgggtcaatgatgacctggtgcattgcaaacgctagggccttgtggggtcagttccg gctgggggttcagcagccagcgcctgatctggggaaccctgtggttggcacatacaaatggacgaacggataaaccttttcacgcccttttaa atatccgattattctaataaacgctcttttctcttaggtttacccgccaatatatcctgtcaaacactgatagttt

SEQ ID NO: 25: tttgaagacatctcaacgcaatcatacttgtgc
SEQ ID NO: 26: tttgaagacttctcggttatgtagacgtagttatggtg
SEQ ID NO: 27: GGTCTCNNNNN
SEQ ID NO: 28: AGGTRAG/GCAGGT

SEQ ID NO: 29: PVX 25K nucleotide sequence

atggatattc tcatcagtag tttgaaaagt ttaggttatt ctaggacttc caaatcttta gattcaggac ctttggtagt acatgcagta gccggagccg gtaagtccac agccctaagg aagttgatcc tcagacaccc aacattcacc gtgcatacac tcggtgtccc tgacaaggtg agtatcagaa ctagaggcat acagaagcca ggacctattc ctgagggcaa cttcgcaatc ctcgatgagt atactttgga caacaccaca aggaactcat accaggcact ttttgctgac ccttatcagg caccggagtt tagcctagag ccccacttct acttggaaac atcatttcga gttccgagga aagtggcaga tttgatagct ggctgtggct tcgatttcga gacgaactca ccggaagaag ggcacttaga gatcactggc atattcaaag ggcccctact cggaaaggtg atagccattg atgaggagtc tgagacaaca ctgtccaggc atggtgttga gtttgttaag ccctgccaag tgacgggact tgagttcaaa gtagtcacta ttgtgtctgc cgcaccaata gaggaaattg gccagtccac agctttctac aacgctatca ccaggtcaaa gggattgaca tatgtccgcg cagggccata g

SEQ ID NO: 30: PVX 25K protein sequence

MDILISSLKSLGYSRTSKSL DSGPLVVHAVAGAGKSTALR KLILRHPTFTVHTLGVPDKV SIRTRGIQKPGPIPEGNFAI LDEYTLDNTTRNSYQALFAD PYQAPEFSLEPHFYLETSFR VPRKVADLIAGCGFDFETNS PEEGHLEITGIFKGPLLGKV IAIDEESETTLSRHGVEFVK PCQVTGLEFKVVTIVSAAPI EEIGQSTAFYNAITRSKGLT YVRAGP

SEQ ID NO: 31: PVX 12K nucleotide sequence

atgtccgcgc agggccatag gctgaccgct ccggtcaatt ctgaaaaagt gtacatagta ttaggtctat catttgcttt agtttcaatt acctttctgc tttctagaaa tagcttaccc cacgtcggtg acaacattca cagcttgcca cacggaggag cttacagaga cggcaccaaa gcaatcttgt acaactcccc aaatctaggg tcacgagtga gtctacacaa cggaaagaac gcagcatttg ctgccgtttt gctactgact ttgctgatct atggaagtaa atacatatct caacgcaatc atacttgtgc ttgtggtaac aatcatagca gtcat

SEQ ID NO: 32: PVX 12K protein sequence

MSAQGHRLTAPVNSEKVYIVLGLSFALVSITFLLSRNSLPHVGDNIHSLPHGGAYRDGTK
AILYNSPNLGSRVSLHNGKNAAFAAVLLLTLLIYGSKYISQRNHTCACGNNHSSH

SEQ ID NO: 33: PVX 8K nucleotide sequence

atggaagtaa atacatatct caacgcaatc atacttgtgc ttgtggtaac aatcatagca gtcattagca cttccttagt gaggactgaa ccttgtgtca tcaagattac tggggaatca atcacagtgt tggcttgcaa actagatgca gaaaccataa gggccattgc cgatctcaag ccactctccg ttgaacggtt aagtttccat

SEQ ID NO: 34: PVX 8K protein sequence

MEVNTYLNAIILVLVVTIIAVISTSLVRTEPCVIKITGESITVLACKLDAETIRAIADLK
PLSVERLSFH

SEQ ID NO: 35: PVX coat protein coding sequence

atgtcagcac cagctagcac aacacagccc atagggtcaa ctacctcaac taccacaaaa actgcaggcg caactcctgc cacagcttca ggcctgttca ctatcccgga tggggatttc tttagtacag cccgtgccat agtagccagc aatgctgtcg caacaaatga ggacctcagc aagattgagg ctatttggaa ggacatgaag gtgcccacag acactatggc acaggctgct tgggacttag tcagacactg tgctgatgta ggatcatccg ctcaaacaga aatgatagat acaggtccct attccaacgg catcagcaga gctagactgg cagcagcaat taaagaggtg tgcacactta ggcaattttg catgaagtat gccccagtgg tatggaactg gatgttaact aacaacagtc cacctgctaa ctggcaagca caaggtttca agcctgagca caaattcgct gcattcgact tcttcaatgg agtcaccaac ccagctgcca tcatgcccaa agaggggctc atccggccac cgtctgaagc tgaaatgaat gctgcccaaa ctgctgcctt tgtgaagatt acaaaggcca gggcacaatc caacgacttt gccagcctag atgcagctgt cactcgaggt cgtatcactg gaacaacaac cgctgaggct gttgtcactc taccaccacc ataa

SEQ ID NO: 36: PVX coat protein

MSAPASTTQPIGSTTSTTTKTAGATPATASGLFTIPDGDFFSTARAIVASNAVATNEDLS
KIEAIWKDMKVPTDTMAQAAWDLVRHCADVGSSAQTEMIDTGPYSNGISRARLAAAIKEV
CTLRQFCMKYAPVVWNWMLTNNSPPANWQAQGFKPEHKFAAFDFFNGVTNPAAIMPKEGL
IRPPSEAEMNAAQTAAFVKITKARAQSNDFASLDAAVTRGRITGTTTAEAVVTLPPP

SEQ ID NO: 37: PVX RdRp coding sequence

atggccaagg tgcgcgaggt ttaccaatct tttacagact ccaccacaaa aactctcatc caagatgagg cttatagaaa
cattcgcccc atcatggaaa aacacaaact agctaaccct tacgctcaaa cggttgaagc ggctaatgat ctagaggggt
tcggcatagc caccaatccc tatagcattg aattgcatac acatgcagcc gctaagacca tagagaataa acttctagag
gtgcttggtt ccatcctacc acaagaacct gttacattta tgtttcttaa acccagaaag ctaaactaca tgagaagaaa cccgcggatc
aaggacattt tccaaaatgt tgccattgaa ccaagagacg tagccaggta ccccaaggaa acaataattg acaaactcac
agagatcaca acggaaacag catacattag tgacactctg cacttcttgg atccgagcta catagtggag acattccaaa
actgcccaaa attgcaaaca ttgtatgcga ccttagttct ccccgttgag gcagccttta aaatggaaag cactcacccg
aacatataca gcctcaaata cttcggagat ggtttccagt atataccagg caaccatggt ggcggggcat accatcatga
attcgctcat ctacaatggc tcaaagtggg aaagatcaag tggagggacc ccaaggatag ctttctcgga catctcaatt
acacgactga gcaggttgag atgcacacag tgacagtaca gttgcaggaa tcgttcgcgg caaaccactt gtactgcatc
aggagaggag acttgctcac accggaggtg cgcactttcg gccaacctga caggtacgtg attccaccac agatcttcct
cccaaaagtt cacaactgca agaagccgat tctcaagaaa actatgatgc agctcttctt gtatgttagg
acagtcaagg tcgcaaaaaa ttgtgacatt tttgccaaag tcagacaatt aattaaatca tctgacttgg acaaatactc tgctgtggaa
ctggtttact tagtaagcta catggagttc cttgccgatt tacaagctac cacctgcttc tcagacacac tttctggtgg cttgctaaca

aagacccttg caccggtgag ggcttggata caagagaaaa agatgcagct gtttggtctt gaggactacg cgaagttagt caaagcagtt gatttccacc cggtggattt ttctttcaaa gtggaaactt gggacttcag attccacccc ttgcaagcgt ggaaagcctt ccgaccaagg gaagtgtcgg atgtagagga aatggaaagt ttgttctcag atggggacct gcttgattgc ttcacaagaa tgccagctta tgcggtaaac gcagaggaag atttagctgc aatcaggaaa acgcccgaga tggatgtcgg tcaagaagtt aaagagcctg caggagacag aaatcaatactcaaaccctg cagaaacttt cctcaacaag ctccacagga aacacagtag ggaggtgaaa caccaggccg caaagaaagc taaacgccta gctgaaatcc aggagtcaat gagagctgaa ggtgatgccg aaccaaatga aataagcggg acgatggggg caatacccag caacgccgaa cttcctggca cgaatgatgc cagacaagaa ctcacactcc caaccactaa acctgtccct gcaaggtggg aagatgcttc attcacagat tctagtgtgg aagaggagca ggttaaactc cttggaaaag aaaccgttga aacagcgacg caacaagtca tcgaaggact tccttggaaa cactggattc ctcaattaaa tgctgttgga ttcaaggcgc tggaaattca gagggatagg agtggaacaa tgatcatgcc catcacagaa atggtgtccg ggctggaaaa agaggacttc cctgaaggaa ctccaaaaga gttggcacga gaattgttcg ctatgaacag aagccctgcc accatccctt tggacctgct tagagccaga gactacggca gtgatgtaaa gaacaagaga attggtgcca tcacaaagac acaggcaacg agttggggcg aatacttgac aggaaagata gaaagcttaa ctgagaggaa agttgcgact tgtgtcattc atggagctgg aggttctgga aaaagtcatg ccatccagaa ggcattgaga gaaattggca agggctcgga catcactgta gtcctgccga ccaatgaact gcggctagat tggagtaaga aagtgcctaa cactgagccc tatatgttca agacctctga aaaggcgtta attgggggaa caggcagcat agtcatcttt gacgattact caaaacttcc tcccggttac atagaagcct tagtctgttt ctactctaaa atcaagctaa tcattctaac aggagatagc agacaaagcg tctaccatga aactgctgag gacgcctcca tcaggcattt gggaccagca acagagtact tctcaaaata ctgccgatac tatctcaatg ccacacaccg caacaagaaa gatcttgcga acatgcttgg tgtctacagt gagagaacgg gagtcaccga aatcagcatg agcgccgagt tcttagaagg aatcccaact ttggtaccct cggatgagaa gagaaagctg tacatgggca ccgggaggaa tgacacgttc acatacgctg gatgccaggg gctaactaag ccgaaggtac aaatagtgtt ggaccacaac acccaagtgt gtagcgcgaa tgtgatgtac acggcacttt ctagagccac cgataggatt cacttcgtga acacaagtgc aaattcctct gccttctggg aaaagttgga cagcacccct tacctcaaga ctttcctatc agtggtgaga gaacaagcac tcagggagta cgagccggca gaggcagagc caattcaaga gcctgagccc cagacacaca tgtgtgtcga gaatgaggag tccgtgctag aagagtacaa agaggaactc ttggaaaagt ttgacagaga gatccactct gaatcccatg gtcattcaaa ctgtgtccaa actgaagaca caaccattca gttgttttcg catcaacaag caaaagatga gactctcctc tgggcgacta tagatgcgcg gctcaagacc agcaatcaag aaacaaactt ccgagaattc ctgagcaaga aggacattgg ggacgttctg tttttaaact accaaaaagc tatgggttta cccaaagagc gtattccttt ttcccaagag gtctgggaag cttgtgccca cgaagtacaa agcaagtacc tcagcaagtc aaagtgcaac ttgatcaatg ggactgtgag acagagccca gacttcgatg aaaataagat tatggtattc ctcaagtcgc agtgggtcac aaaggtggaa aaactaggtc tacccaagat taagccaggt caaaccatag cagcctttta ccagcagact gtgatgcttt ttggaactat ggctaggtac atgcgatggt tcagacaggc tttccagcca aaagaagtct tcataaactg tgagacgacg ccagatgaca tgtctgcatg ggccttgaac aactggaatt tcagcagacc tagcttggct aatgactaca cagctttcga ccagtctcag gatggagcca tgttgcaatt tgaggtgctc aaagccaaac accactgcat accagaggaa atcattcagg catacataga tattaagact aatgcacaga ttttcctagg cacgttatca attatgcgcc tgactggtga aggtcccact tttgatgcaa acactgagtg caacatagct tacacccata caaagtttga catcccagcc ggaactgctc aagtttatgc aggagacgac tccgcactgg actgtgttcc agaagtgaag catagtttcc acaggcttga ggacaaatta ctcctaaagt caaagcctgt aatcacgcag caaaagaagg gcagttggcc tgagttttgt ggttggctga tcacaccaaa aggggtgatg aaagacccaa ttaagctcca tgttagctta aaattggctg aagctaaggg tgaactcaag aaatgtcaag attcctatga aattgatctg agttatgcct atgaccacaa ggactctctg catgacttgt tcgatgagaa acagtgtcag gcacacacac tcacttgcag aacactaatc aagtcaggga gaggcactgt ctcactttcc cgcctcagaa actttcttta a

## Claims

1. A method of producing a potexviral vector for expressing a protein of interest in a plant, comprising

   producing a second heterologous nucleic acid sequence comprising a second ORF encoding said protein of interest and having, in the second ORF, an increased GC-content compared to a first ORF encoding said protein in a first heterologous nucleic acid sequence, and
   providing said potexviral vector comprising the following segments: (i) a nucleic acid sequence encoding a potexviral RNA-dependent RNA polymerase, (ii) a nucleic acid sequence comprising or encoding a potexviral triple-gene block, and (iii) said second heterologous nucleic acid sequence or a portion thereof comprising said second ORF.

2. A method of improving the capability for long-distance movement in a plant of a potexviral replicon encoding a protein of interest to be expressed in said plant, comprising

   producing a second heterologous nucleic acid sequence comprising a second ORF encoding said protein of interest and having, in the second ORF, an increased GC-content compared to a first ORF encoding said protein in a first heterologous nucleic acid sequence, and
   providing said potexviral replicon, or a potexviral vector comprising or encoding said potexviral replicon, said potexviral replicon comprising the following segments: (i) a nucleic acid sequence encoding a potexviral RNA-dependent RNA polymerase, (ii) a nucleic acid sequence comprising a potexviral triple-gene block, and (iii) said second heterologous nucleic acid sequence or a portion thereof comprising said second ORF.

3. The method according to claim 1 or 2, wherein said step of providing a potexviral vector or potexviral replicon comprises inserting said second heterologous nucleic acid sequence, or a portion thereof comprising said second ORF, into a nucleic acid comprising (i) a nucleic acid sequence encoding a potexviral RNA-dependent RNA polymerase and (ii) a nucleic acid sequence encoding a potexviral triple-gene block to produce the potexviral vector or the potexviral replicon comprising the second heterologous nucleic acid sequence or a portion thereof comprising said second ORF.

4. A process of expressing a protein of interest in a plant or in plant tissue, comprising producing a potexviral vector according to the method of claim 1 and providing the produced potexviral vector to at least a part of said plant.

5. The method or process according to any one of claims 1 to 4, wherein said plant is selected from *Nicotiana* species such as *Nicotiana benthamiana* and *Nicotiana tabacum,* tomato, potato, pepper, eggplant, soybean, *Petunia hybrida, Brassica napus, Brassica campestris, Brassica juncea,* cress, arugula, mustard, strawberry, spinach, *Chenopodium capitatum,* alfalfa, lettuce, sunflower, potato, cucumber, corn, wheat, and rice.

6. The method or process according to any one of claims 1 to 5, wherein said (ii) nucleic acid sequence comprising or encoding a potexviral triple-gene block said further comprises a nucleic acid sequence encoding a potexviral coat protein or a nucleic acid sequence encoding a tobamoviral movement protein.

7. A method of improving the capability for long-distance movement in a plant of a potexviral replicon encoding a protein of interest to be expressed in said plant, comprising

   increasing the GC-content of a first ORF encoding said protein in a first heterologous nucleic acid sequence, thereby obtaining a second heterologous nucleic acid sequence comprising a second ORF, said second ORF encoding said protein and having an increased GC-content, and
   inserting said second heterologous nucleic acid sequence, or a portion thereof containing said second ORF, into a nucleic acid comprising (i) a nucleic acid sequence encoding a potexviral RNA-dependent RNA polymerase and (ii) a nucleic acid sequence comprising or encoding a potexviral triple-gene block to produce a potexviral vector comprising or encoding said potexviral replicon, said potexviral vector comprising the second heterologous nucleic acid sequence or a portion thereof comprising said second ORF.

**8.** A potexviral vector obtained or obtainable by the method of claim 1.

**9.** A nucleic acid comprising the following segments: (i) a nucleic acid sequence encoding a potexviral RNA-dependent RNA polymerase, (ii) nucleic acid sequence comprising or encoding a potexviral triple-gene block, and (iii) a heterologous nucleic acid sequence comprising an ORF encoding a protein of interest, wherein

said ORF consists of at least 200 and at most 400 nucleotides and has a GC-content of at least 50%; or
said ORF consists of at least 401 and at most 800 nucleotides has a GC-content of at least 55%; and/or
said ORF consists of at least 801 nucleotides and has a GC-content of at least 58%.

**10.** A nucleic acid comprising the following segments: (i) a nucleic acid sequence encoding a potexviral RNA-dependent RNA polymerase, (ii) nucleic acid sequence comprising or encoding a potexviral triple-gene block, and (iii) a heterologous nucleic acid sequence comprising an ORF encoding a protein of interest, wherein

said ORF consists of at least 100 and at most 500 nucleotides and has a GC-content of at least 50%; or
said ORF consists of at least 501 and at most 1000 nucleotides has a GC-content of at least 55%; and/or
said ORF consists of at least 1001 nucleotides and has a GC-content of at least 58%.

**11.** The nucleic acid according to claim 9 or 10, said nucleic acid further comprising, preferably in the nucleic acid sequence of (ii), a nucleic acid sequence encoding a potexviral coat protein or a nucleic acid sequence encoding a tobamoviral movement protein.

**12.** A combination or kit comprising a first and a second nucleic acid, said first nucleic acid comprising segments (i) and (ii) as defined in claim 9, 10 or 11, said second nucleic acid comprising segment (iii) as defined in claim 9 or 10.

**13.** The combination or kit according to claim 12, wherein said first nucleic acid has, downstream of segment (ii) a first site-specific recombination site recognizable by a site-specific recombinase, and said second nucleic acid has, upstream of segment (iii), a second site-specific recombination site recognizable by said site-specific recombinase for allowing site-specific recombination between said first and said second site-specific recombination site and formation of a nucleic acid according to claim 9, 10, or 11, or a potexviral vector according to claim 8.

**14.** A process of expressing a heterologous nucleic acid sequence of interest in a plant or in plant tissue, comprising providing the plant or plant tissue with said nucleic acid of claim 9 or 10, with a potexviral vector according to claim 8, or with a combination or kit of nucleic acids according to claim 13 or 14.

**15.** Use of a heterologous nucleic acid as defined in claim 9 or 10, a potexviral vector according to claim 8, or a combination or kit of nucleic acids according to claim 13 or 14 for expressing a protein encoded by said heterologous nucleic acid and for achieving improved long-distance movement of a potexviral vector in a plant.

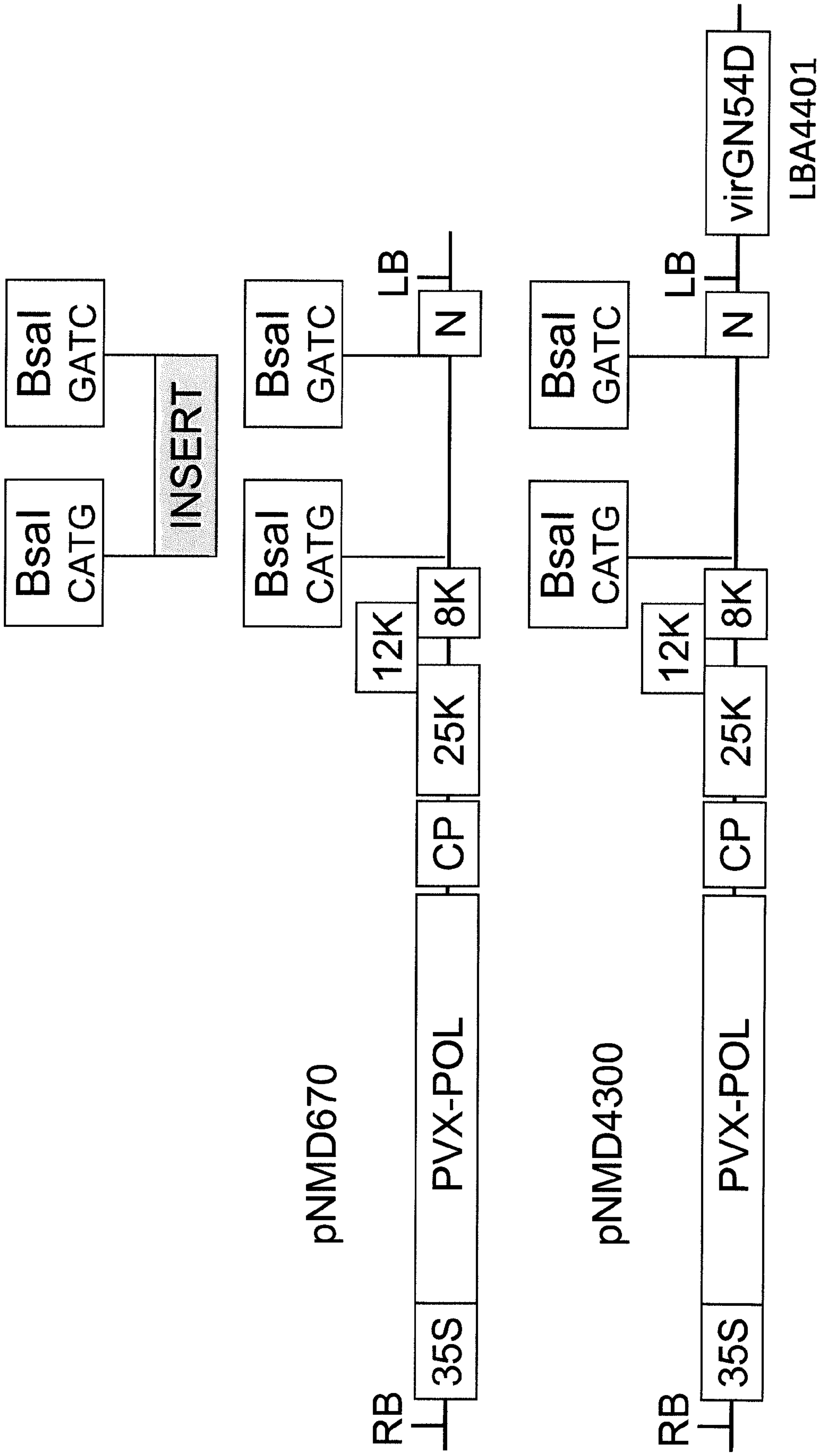
BsaI
CATG
BsaI
GATC
INSERT
pNMD670
RB
35S
PVX-POL
CP
25K
12K
8K
BsaI
CATG
BsaI
GATC
N
LB
pNMD4300
RB
35S
PVX-POL
CP
25K
12K
8K
BsaI
CATG
BsaI
GATC
N
LB
virGN54D
LBA4401

Fig. 1

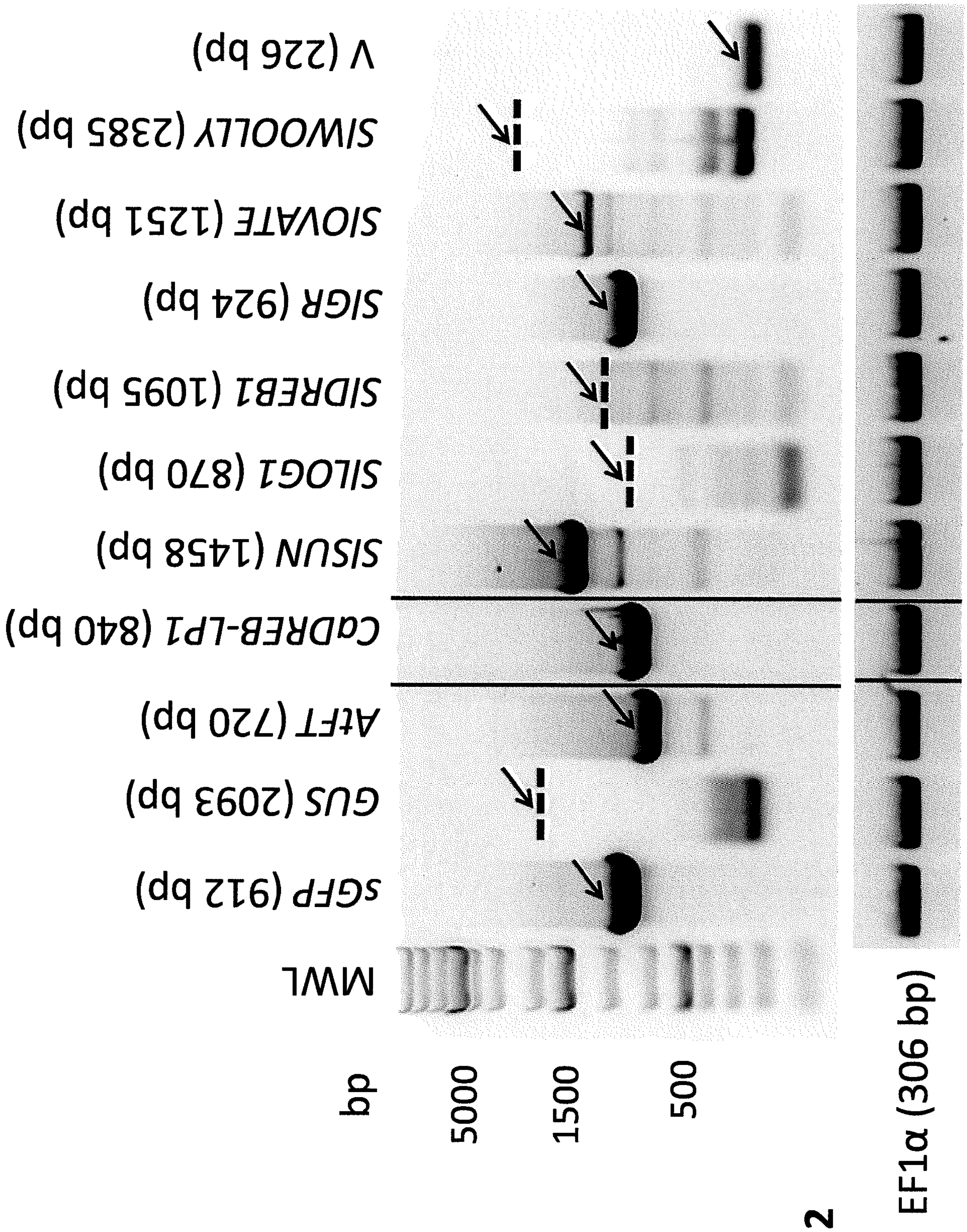
V (226 bp)
SlWOOLLY (2385 bp)
SlOVATE (1251 bp)
SlGR (924 bp)
SlDREB1 (1095 bp)
SlLOG1 (870 bp)
SlSUN (1458 bp)
CaDREB-LP1 (840 bp)
AtFT (720 bp)
GUS (2093 bp)
sGFP (912 bp)
MWL
bp
5000
1500
500
EF1α (306 bp)

Fig. 2

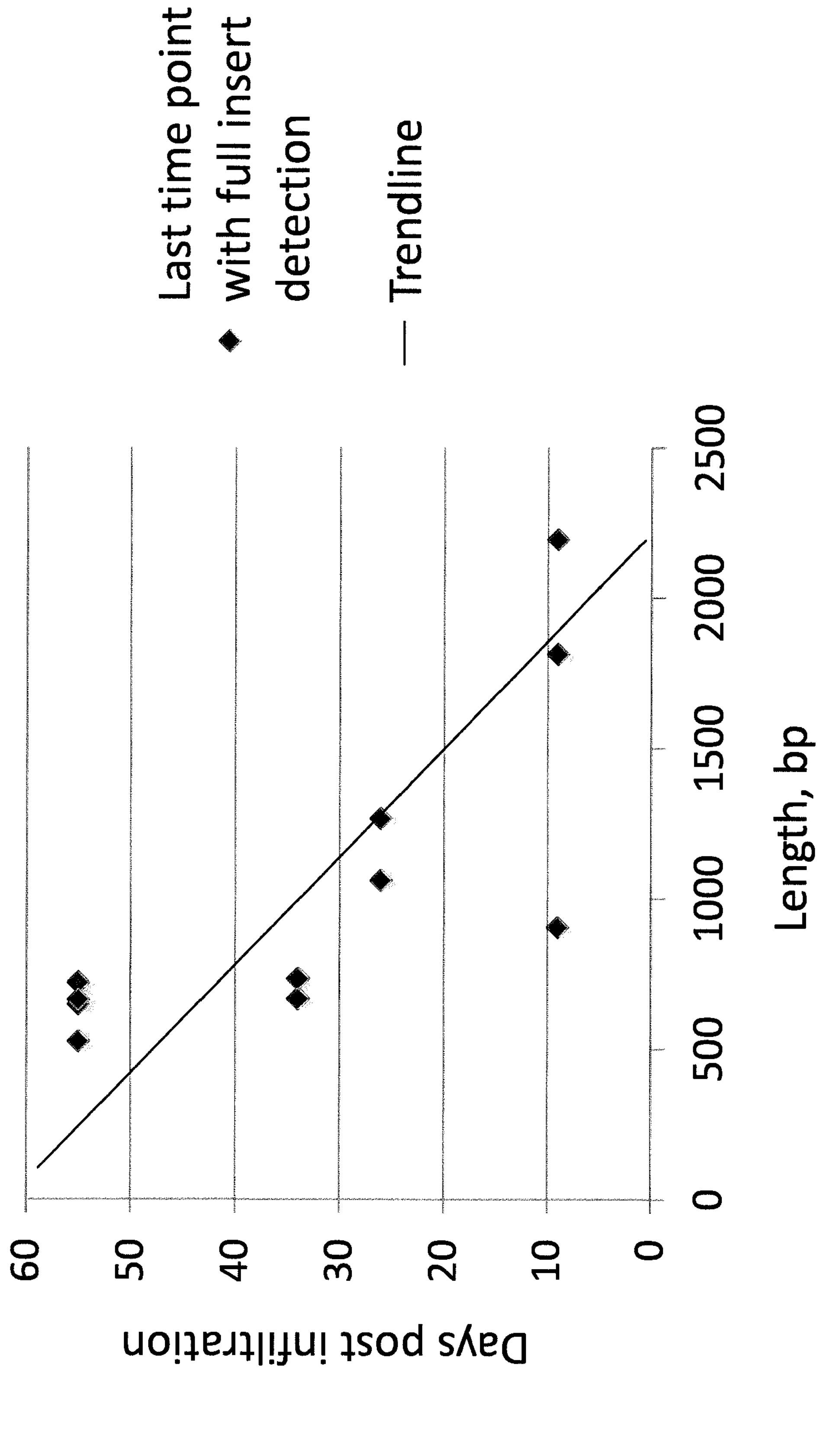
Days post infiltration
60
50
40
30
20
10
0
0
500
1000
1500
2000
2500
Length, bp
Last time point with full insert detection
Trendline

Fig. 3

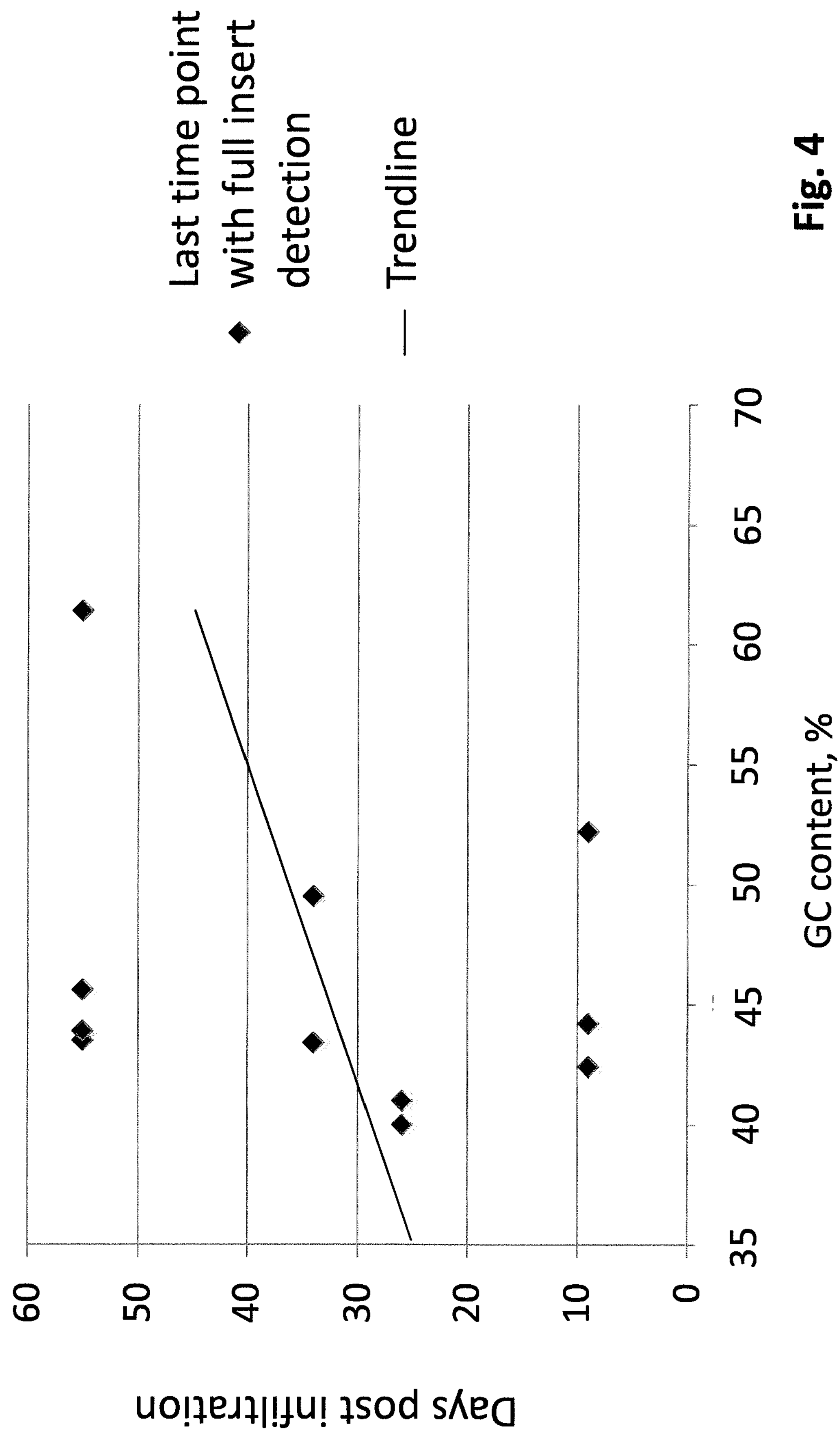
Last time point with full insert detection
Trendline
Days post infiltration
0
10
20
30
40
50
60
35
40
45
50
55
60
65
70
GC content, %

**Fig. 4**

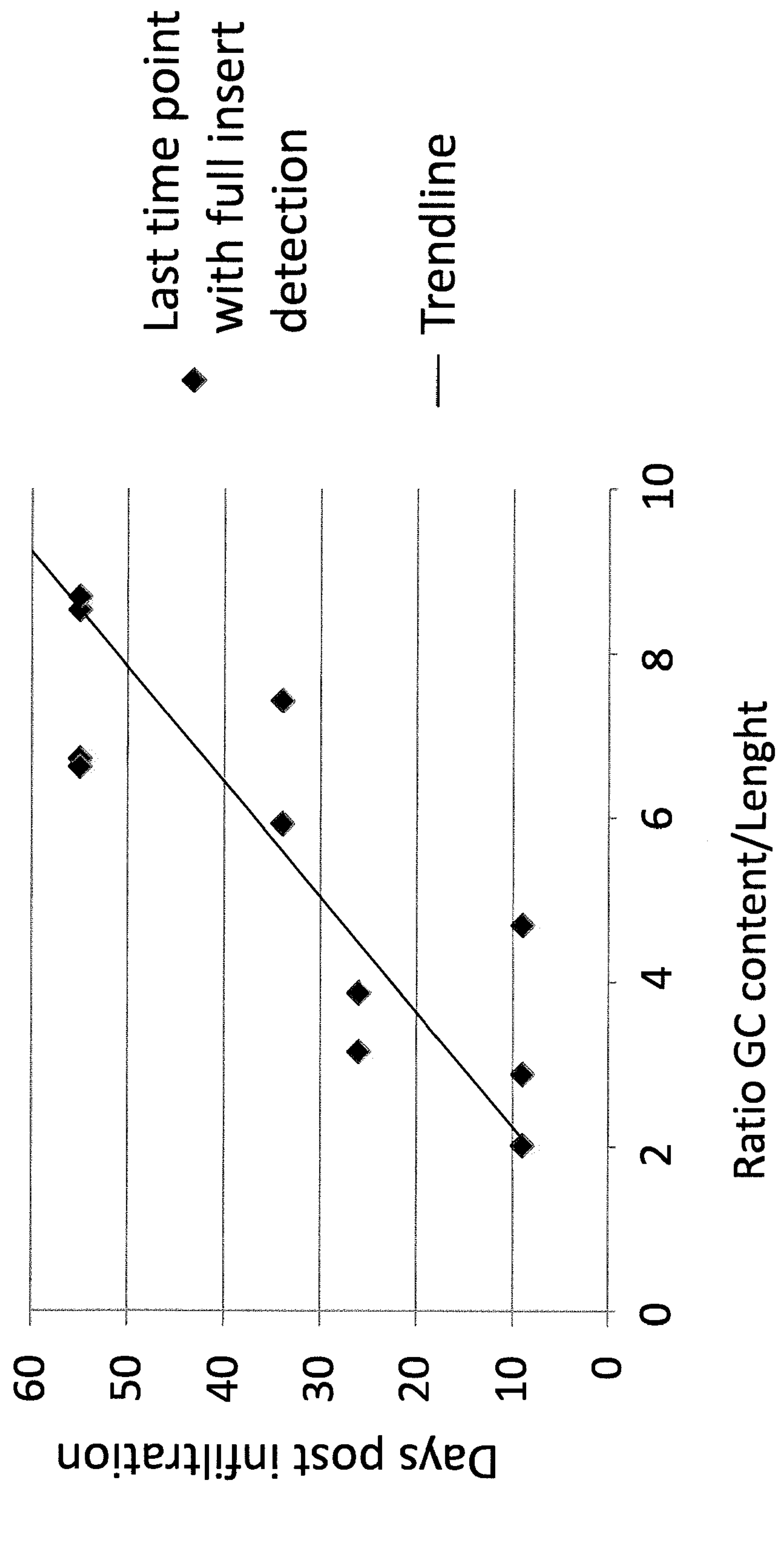
Days post infiltration
0
10
20
30
40
50
60
0
2
4
6
8
10
Ratio GC content/Lenght
Last time point with full insert detection
Trendline

Fig. 5

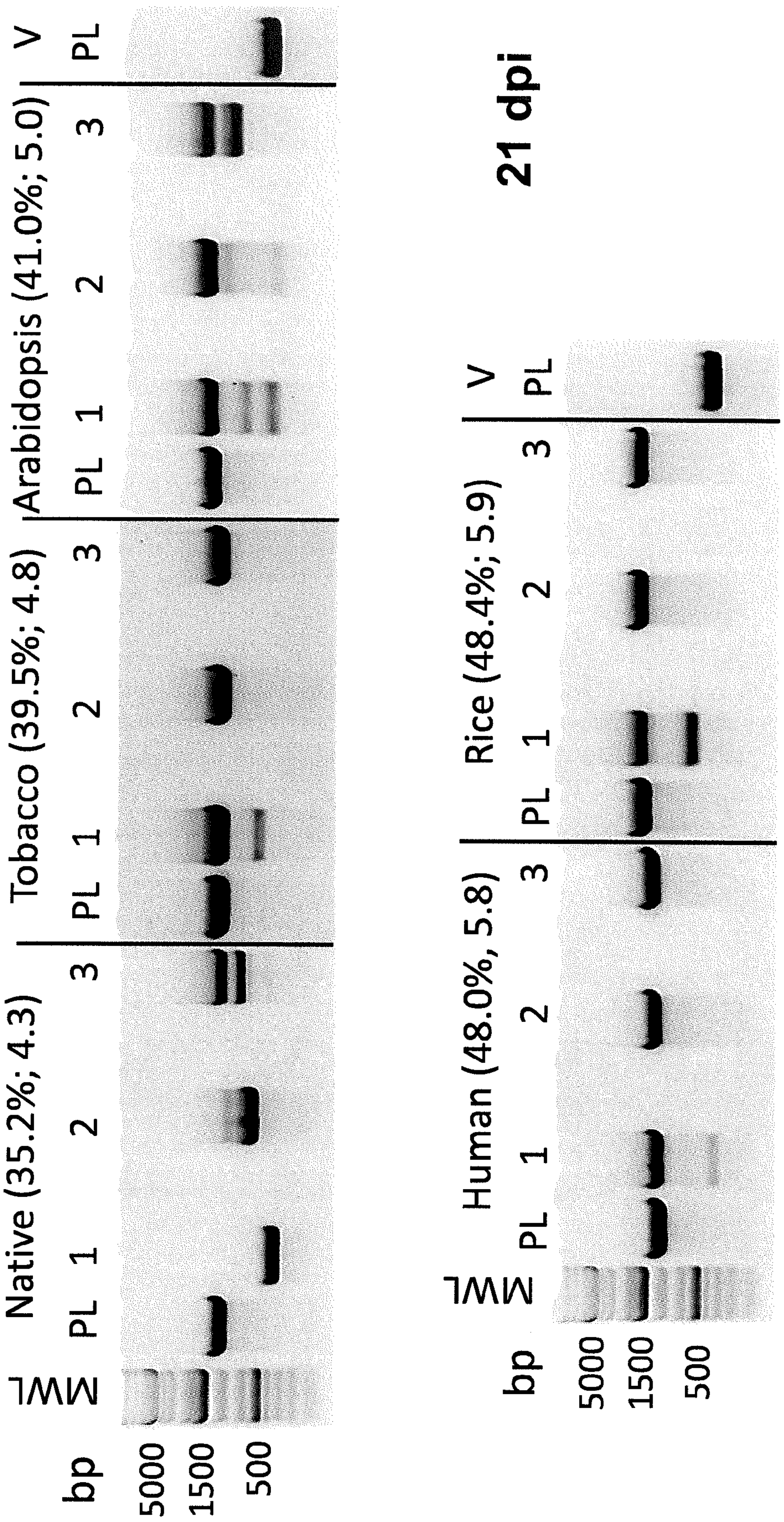
Native (35.2%; 4.3)
Tobacco (39.5%; 4.8)
Arabidopsis (41.0%; 5.0)
V
MWL
PL
1
2
3
bp
5000
1500
500
Human (48.0%, 5.8)
Rice (48.4%; 5.9)
21 dpi

Fig. 6

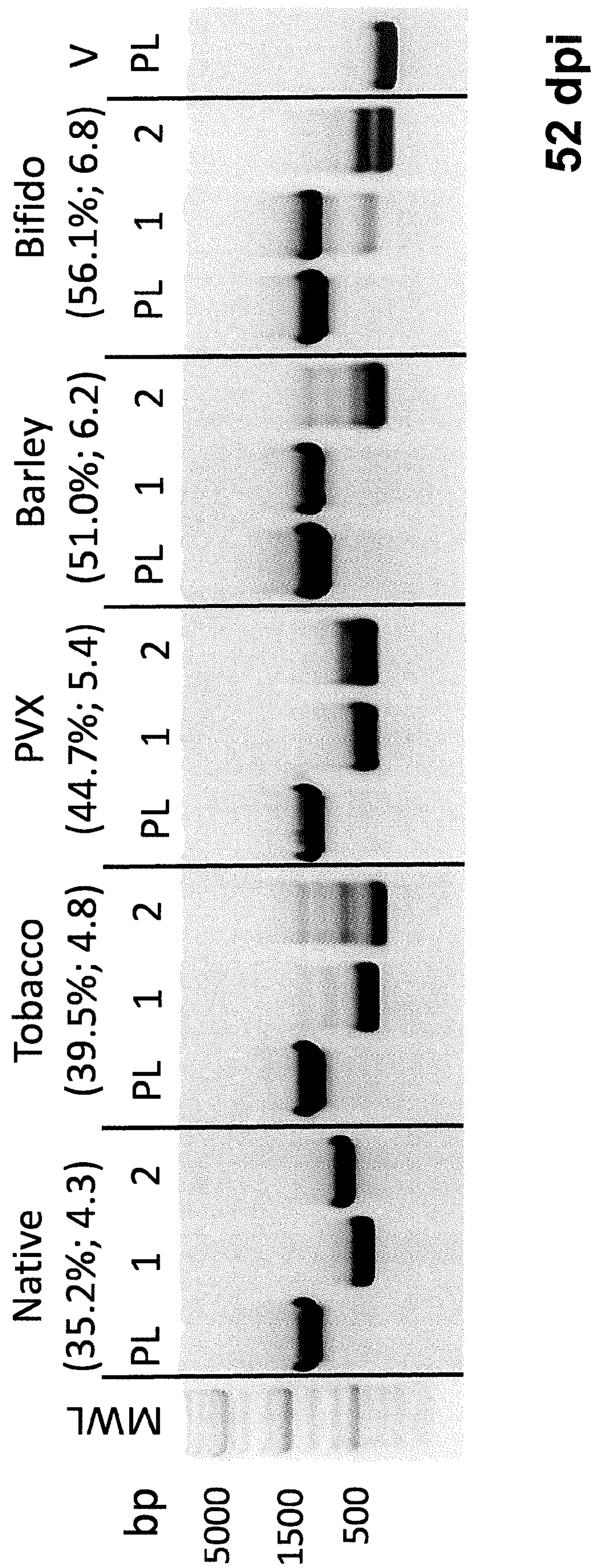
Native
(35.2%; 4.3)
PL 1 2
Tobacco
(39.5%; 4.8)
PL 1 2
PVX
(44.7%; 5.4)
PL 1 2
Barley
(51.0%; 6.2)
PL 1 2
Bifido
(56.1%; 6.8)
PL 1 2
V
PL
MWL
bp
5000
1500
500
52 dpi

Fig. 7

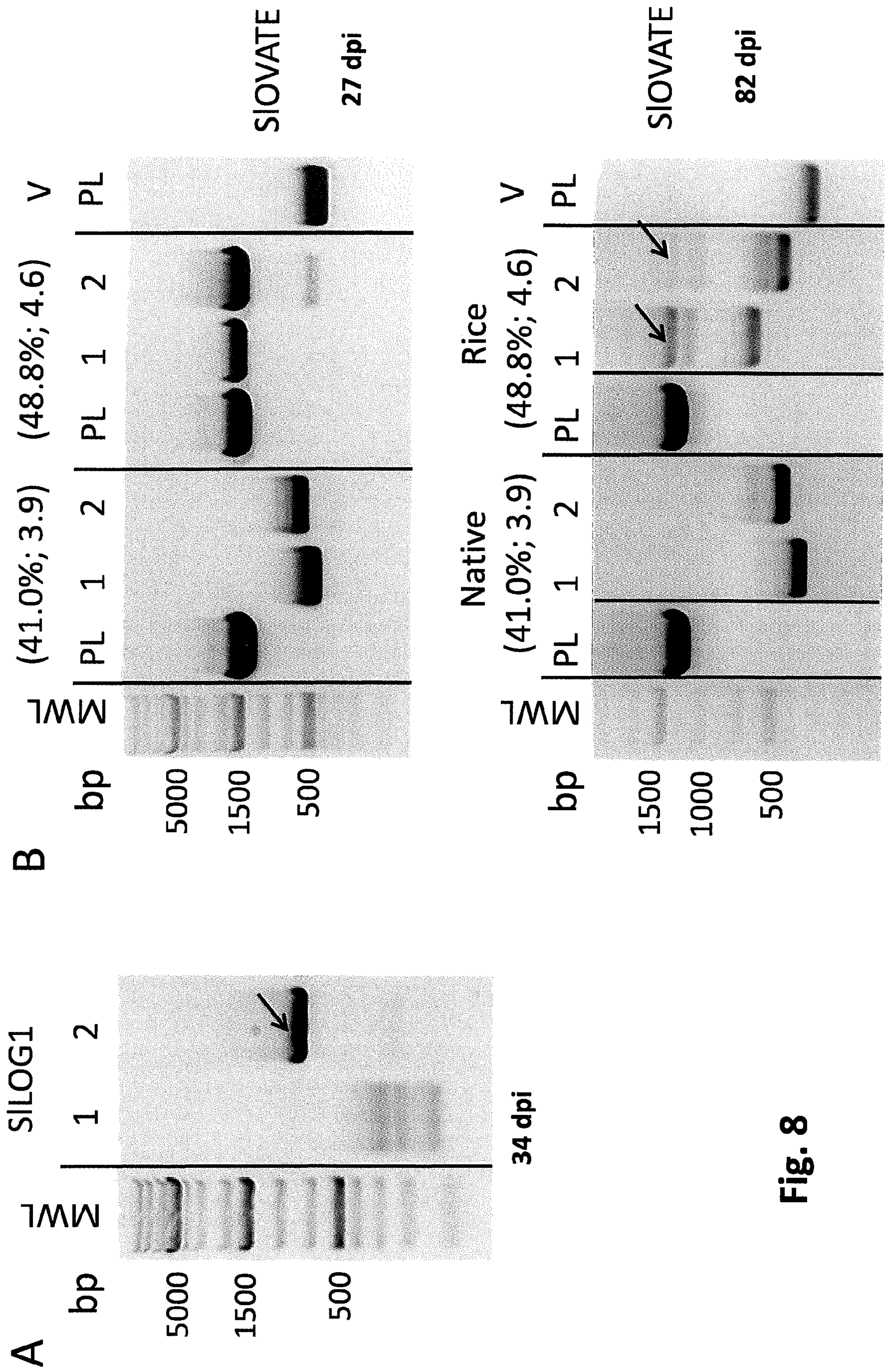
A
bp
5000
1500
500
MWL
SlLOG1
1
2
34 dpi
B
bp
5000
1500
500
MWL
Native
(41.0%; 3.9)
PL
1
2
Rice
(48.8%; 4.6)
PL
1
2
V
PL
SlOVATE
27 dpi
bp
1500
1000
500
MWL
Native
(41.0%; 3.9)
PL
1
2
Rice
(48.8%; 4.6)
PL
1
2
V
PL
SlOVATE
82 dpi

Fig. 8

Table 1

| SEQ ID NO | Plasmid | Insert | Insert Description | GenBank Accession Nr | Length, bp | GC content, % | Ratio GC Content / Length | Last Time Point with Full Insert, days |
|---|---|---|---|---|---|---|---|---|
| 1 | pNMD11632 | *AtFT* | Phosphatidylethanolamine-binding protein family protein CDS from *Arabidopsis thaliana* | NM_001334207.1 | 528 | 45.6 | 8.6 | 55 |
| 2 | pNMD25152 | *CaDREB-LP1* | Dehydration-responsive element-binding protein 1A-like CDS from *Capsicum annuum* | NM_001324857.1 | 648 | 43.5 | 6.7 | 55 |
| 3 | pNMD29012 | *AmROS1* | ROSEA1 MYB transcriptional factor CDS from *Antirrhinum majus* | DQ275529.1 | 663 | 43.9 | 6.6 | 55 |
| 4 | pNMD28832 | *GmLOG1* | Cytokinin riboside 5'-monophosphate phosphoribohydrolase CDS from *Glycine max* | XM_003527643.3 | 666 | 49.5 | 7.4 | 34 |
| 5 | pNMD27533 | *SlLOG1* | Cytokinin riboside 5'-monophosphate phosphoribohydrolase CDS from *Solanum lycopersicum* | NM_001324502.1 | 678 | 41.9 | 6.2 | 9 |
| 6 | pNMD5800 | *sGFP* | Green Fluorescent Protein CDS, synthetic sequence | U43284.1 | 720 | 61.4 | 8.5 | 55 |
| 7 | pNMD27832 | *SlGR* | Green Ripe Protein CDS from *Solanum lycopersicum* | XM_010325884.2 | 732 | 43.4 | 5.9 | 34 |
| 8 | pNMD27712 | *SlDREB1* | Dehydration responsive element binding protein CDS from *Solanum lycopersicum* | NM_001247760.1 | 903 | 42.4 | 4.7 | 9 |
| 9 | pNMD27931 | *SlOVATE* | OVATE repressor of transcription CDS from *Solanum lycopersicum* | NM_001247292.2 | 1059 | 41.0 | 3.9 | 26 |
| 10 | pNMD27524 | *SlSUN* | Calmodulin binding protein SUN CDS from *Solanum lycopersicum* | NM_001246864.2 | 1266 | 40.0 | 3.2 | 26 |
| 11 | pNMD5970 | *GUS* | β-glucuronidase gene (*uidA*) CDS from *Escherichia coli* K-12 | S69414.1 | 1812 | 52.2 | 2.9 | 9 |
| 12 | pNMD28732 | *SlWoolly* | homeobox-leucine zipper protein PROTODERMAL FACTOR 2 | XM_010318312.2 | 2193 | 44.2 | 2.0 | 9 |

**Fig. 9**

Table 3

| SEQ ID NO | Plasmid | Insert | Insert Description | GenBank Accession Nr | Length, bp | GC content, % | Ratio GC content / Length |
|---|---|---|---|---|---|---|---|
| 5 | pNMD27533 | SlLOG1 | Cytokinin riboside 5'-monophosphate phosphoribohydrolase CDS from *Solanum lycopersicum* | NM_001324502.1 | 678 | 41.9 | 6.2 |
| 21 | pNMD31084 | SlLOG1-*rice* | Cytokinin riboside 5'-monophosphate phosphoribohydrolase CDS from *Solanum lycopersicum* | | 678 | *53.2* | 7.8 |
| 9 | pNMD27931 | SlOVATE | OVATE repressor of transcription CDS from *Solanum lycopersicum* | NM_001247292.2 | 1059 | 41.0 | 3.9 |
| 22 | pNMD31611 | SlOVATE-*rice* | OVATE repressor of transcription CDS from *Solanum lycopersicum* | | 1059 | 48.8 | 4.6 |

Fig. 10

Europäisches Patentamt
European Patent Office
Office européen des brevets

# EUROPEAN SEARCH REPORT

Application Number
EP 17 19 1524

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>Y | EP 2 061 890 A2 (ICON GENETICS GMBH [DE]) 27 May 2009 (2009-05-27)<br>* claim 1; sequence 7 * | 8-15<br>1-7 | INV.<br>C12N15/82 |
| X | ADAMS M J ET AL: "Codon usage bias amongst plant viruses", ARCHIVES OF VIROLOGY, SPRINGER WIEN, AT, vol. 149, no. 1, 1 January 2004 (2004-01-01), pages 113-135, XP002404994, ISSN: 0304-8608<br>* page 116, lines 17-37; table 3 * | 8-13 | |
| Y | EP 2 568 048 A1 (PIONEER HI BRED INT [US]) 13 March 2013 (2013-03-13)<br>* paragraph [0183] * | 1-7 | |
| A | ROUHOLLAH BARAHIMIPOUR ET AL: "Dissecting the contributions of GC content and codon usage to gene expression in the model alga Chlamydomonas reinhardtii", THE PLANT JOURNAL, vol. 84, no. 4, 16 October 2015 (2015-10-16), pages 704-717, XP055421909, GB ISSN: 0960-7412, DOI: 10.1111/tpj.13033<br>* the whole document * | 1-15 | TECHNICAL FIELDS SEARCHED (IPC)<br>C12N |

The present search report has been drawn up for all claims

1

| Place of search | Date of completion of the search | Examiner |
|---|---|---|
| Munich | 9 November 2017 | Obel, Nicolai |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# ANNEX TO THE EUROPEAN SEARCH REPORT ON EUROPEAN PATENT APPLICATION NO. EP 17 19 1524

This annex lists the patent family members relating to the patent documents cited in the above-mentioned European search report. The members are as contained in the European Patent Office EDP file on
The European Patent Office is in no way liable for these particulars which are merely given for the purpose of information.

09-11-2017

| Patent document cited in search report | Publication date | Patent family member(s) | Publication date |
|---|---|---|---|
| EP 2061890 A2 | 27-05-2009 | AU 2007294097 A1 | 13-03-2008 |
| | | BR PI0716193 A2 | 12-11-2013 |
| | | CA 2657132 A1 | 13-03-2008 |
| | | CN 101512002 A | 19-08-2009 |
| | | DK 2061890 T3 | 23-07-2012 |
| | | EP 1897953 A1 | 12-03-2008 |
| | | EP 2061890 A2 | 27-05-2009 |
| | | ES 2389783 T3 | 31-10-2012 |
| | | JP 2010502203 A | 28-01-2010 |
| | | US 2010071084 A1 | 18-03-2010 |
| | | WO 2008028661 A2 | 13-03-2008 |
| EP 2568048 A1 | 13-03-2013 | BR PI0816750 A2 | 29-09-2015 |
| | | CA 2691440 A1 | 08-01-2009 |
| | | CN 101849010 A | 29-09-2010 |
| | | EP 2167666 A2 | 31-03-2010 |
| | | EP 2568048 A1 | 13-03-2013 |
| | | US 2009133152 A1 | 21-05-2009 |
| | | US 2015074843 A1 | 12-03-2015 |
| | | WO 2009006297 A2 | 08-01-2009 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2005049839 A
- WO 2008028661 A
- WO 2007137788 A1
- WO 2012019660 A
- WO 2013056829 A
- WO 02088369 A
- WO 03020938 A
- WO 2006079546 A


### Non-patent literature cited in the description


- **DONSON et al.** *Proc Natl Acad Sci U S A,* vol. 88, 7204-7208
- **CHAPMAN, KAVANAGH ; BAULCOMBE.** *Plant J.,* 1992, vol. 2, 549-557
- **MARILLONNET et al.** *Nat Biotechnol.,* 2005, vol. 23, 718-723
- **SANTI et al.** *Proc Natl Acad Sci U S A.,* 2006, vol. 103, 861-866
- **BAULCOMBE, CHAPMAN ; SANTA CRUZ.** *Plant J.,* 1995, vol. 7, 1045-1053
- **ZHOU et al.** *Appl. Microbiol. Biotechnol.,* 2006, vol. 72 (4), 756-762
- **ZELADA et al.** *Tuberculosis,* 2006, vol. 86, 263-267
- **MARILLONNET et al.** *Nat. Biotechnol.,* 2005, vol. 23, 718-723
- **STEPHEN F. ALTSCHUL ; THOMAS L. MADDEN ; ALEJANDRO A. SCHÄFFER ; JINGHUI ZHANG ; ZHENG ZHANG ; WEBB MILLER ; DAVID J. LIPMAN.** Gapped BLAST and PSI-BLAST: a new generation of protein database search programs. *Nucleic Acids Res.,* 1997, vol. 25, 3389-3402
- *J. Gen. Virol.,* 2003, vol. 84, 1351-1366
- **MARILLONNET et al.** *Proc. Natl. Acad. Sci. USA,* 2004, vol. 101, 6852-6857
- **HAAS J. ; PARK E.C. ; SEED B.** Codon usage limitation in the expression of HIV-1 envelope glycoprotein. *Curr Biol,* 1996, vol. 6 (3), 315-24
- **CHIU W. ; NIWA Y. ; ZENG W. ; HIRANO T. ; KOBAYASHI H. ; SHEEN J.** Engineered GFP as a vital reporter in plants. *Curr Biol,* 1996, vol. 6 (3), 325-30
- **DEAN J.D. ; GOODWIN P.H. ; HSIANG T.** *Induction of glutathione S-transferase genes of Nicotiana benthamiana following infection by Colletotrichum destructivum and C. orbiculare and involvement of one in resistance,* 2005, vol. 56 (416), 1525-1533
- **AVESANI L. ; MARCONI G. ; MORANDINI F. ; ALBERTINI E. ; BRUSCHETTA M. ; BORTESI L. ; PEZZOTTI M. ; PORCEDDU A.** Stability of Potato Virus X expression vectors is related to insert size: implications for replication models and risk assessment. *Transgenic Res,* 2007, vol. 16 (5), 587-97
- **MATHEWS H. ; CLENDENNEN S.K. ; CALDWELL C.G. ; LIU X.L. ; CONNORS K. ; MATHEIS N. ; SCHUSTER D.K. ; MENASCO D.J. ; WAGONER W. ; LIGHTNER, J.** Activation tagging in tomato identifies a transcriptional regulator of anthocyanin biosynthesis, modification, and transport. *Plant Cell,* 2003, vol. 15 (8), 1689-1703

SEQUENCE LISTING

<110> Nomad Bioscience GmbH

<120> Method of Improving Potexviral Vector Stability

<130> EAD-17316

<160> 37

<170> PatentIn version 3.5

<210> 1
<211> 528
<212> DNA
<213> Artificial Sequence

<220>
<223> AtFT, one nucleotide exchange

<400> 1
atgtctataa atataaggga ccctcttata gtaagcagag ttgttggaga cgttcttgat 60

ccgtttaata gatcaatcac tctaaaggtt acttatggcc aaagagaggt gactaatggc 120

ttggatctaa ggccttctca ggttcaaaac aagccaagag ttgagattgg tggagaagac 180

ctcaggaact tctatacttt ggttatggtg gatccagatg ttccaagtcc tagcaaccct 240

cacctccgag aatatctcca ttggttggtg actgatatcc ctgctacaac tggaacaacc 300

tttggcaatg agattgtgtg ttacgaaaat ccaagtccca ctgcaggaat tcatcgtgtc 360

gtgtttatat tgtttcgaca gcttggcagg caaacagtgt atgcaccagg gtggcgccag 420

aacttcaaca ctcgcgagtt tgctgagatc tacaatctcg gccttcccgt ggccgcagtt 480

ttctacaatt gtcagaggga gagtggctgc ggaggaagaa gactttag 528

<210> 2
<211> 648
<212> DNA
<213> Capsicum annuum

<400> 2
atgaacatct ttagaagcta ttattcggac ccacttactg aatcttcatc atctttttct 60

gatagtagca tttactcccc taatagagct attttttctg atgaggaagt tatattagca 120

tcaaataacc cgaaaaagcc agctgggagg aagaagtttc gagaaactcg acatccagta 180

tacaggggag ttaggaagag gaattcaggc aaatgggttt gtgaagtcag agaacccaat 240

aagaaatcaa gaatttggct tggtactttt cctacagctg aaatggctgc tagagctcat 300

gacgtggcgg ctatagcatt aagaggtcgt tctgcttgtt tgaactttgc tgattctgct 360

tggaggttgc ctgttccggc ttcctctgac actaaagata ttcaaaaggc ggccgctgag 420

gccgcggaag ccctccgacc attgaagttg gaaggaattt caaaagaatc atctagcagt 480

actccagaga gtatgttctt tatggatgag gaagcgctct tctgcatgcc gggattactt 540

acgaatatgg ctgaagggct aatgttacca ccacctcaat gtgcagaaat tggagatcat 600

gtggaaactg ctgatgcgga taccccttta tggagctatt ccatttaa 648

<210> 3
<211> 663
<212> DNA
<213> Antirrhinum majus

<400> 3
atggaaaaga attgtcgtgg agtgagaaaa ggtacttgga ccaaagaaga agacactctc 60

ttgaggcaat gtatagaaga gtatggtgaa gggaaatggc atcaagttcc acacagagca 120

gggttgaacc ggtgtaggaa gagttgcagg ctgaggtggt tgaattatct gaggccaaat 180

atcaaaagag gtcggttttc gagagatgaa gtggacctaa ttgtgaggct tcataagctg 240

ttgggtaaca aatggtcgct gattgctggt agaattcctg gaaggacagc taatgacgtg 300

aagaactttt ggaatactca tgtggggaag aatttaggcg aggatggaga acgatgccgg 360

aaaaatgtta tgaacacaaa aaccattaag ctgactaata tcgtaagacc ccgagctcgg 420

accttcaccg gattgcacgt tacttggccg agagaagtcg gaaaaaccga tgaattttca 480

aatgtccggt taacaactga tgagattcca gattgtgaga agcaaacgca attttacaat 540

gatgttgcgt cgccacaaga tgaagttgaa gactgcattc agtggtggag taagttgcta 600

gaaacaacgg aggatgggga attaggaaac ctattcgagg aggcccaaca aattggaaat 660

taa 663

<210> 4
<211> 666
<212> DNA
<213> Glycine max

<400> 4
atggaaactc aacaccaaca acccaccatc aagtctaggt tcagacgcat ctgtgtctac 60

tgtggtagca gccctggcaa aaacccccagc taccagctcg ctgctattca actcggaaaa 120

caactggtgg agaggaacat tgacttggtt tatggaggag gaagcatagg gttgatgggt 180

ctaatctcac aagttgtgta tgatggtgga cgccacgtgt taggggtgat tccagagaca 240

cttaatgcaa gagagataac tggagagagt gttggagaag tgagagctgt atcgggcatg 300

caccaacgca aagccgaaat ggcccgacaa gccgatgcat ttattgcact gccaggtgga 360

tatggcaccc ttgaagaact actggaaatt atcacctggg ctcaactagg catccatgat 420

aaaccggtgg ggttgttgaa cgtggatggg tactacaact cgctgctggc attcatggac 480

aaagctgtgg acgaaggttt cgtaacacca gctgcccgtc acattattgt ttctgcccac 540

actgcccaag aactcatgtg caaacttgag gaatatgtcc ccgagcactg tggcgtggcc 600

cccaagctaa gttgggagat ggagcaacag ttagttaaca ctgcaaagtc agatatttcc 660

cgttga 666

<210> 5
<211> 678
<212> DNA
<213> Lycopersicon esculentum

<400> 5
atggaaaaca atcaccagac acaaattcag accactaaaa catcaagatt caaacgcata 60

tgtgtttttt gtggaagcag tccaggcaaa aagccaagtt atcaacttgc tgctattcaa 120

cttggcaatc aactggttga aaggaacatc gacttggttt atggaggtgg cagtgtgggc 180

ttgatgggcc tagtttctca atcagttttt aatggtggcc gccacgtgtt aggggtgatt 240

cctaaaactc ttatgccaag agagattact ggagaaagtg ttggagaagt aagagcagtg 300

tctgggatgc atcaaagaaa agcagaaatg gcaagacaag ctgatgcatt catagcctta 360

ccaggtggct atgggacatt ggaagagctc ctagaagtca tcacttgggc tcaactaggc 420

attcatgata aaccagtagg tttacttaat gtagatggct actataattc attattatca 480

tttatagaca aagctgttga tgaaggcttt gtcacaccct ctgcccgtca catcattatt 540

tctgccccaa ctgcccaaga actcatgtct aagcttgagg attatgtacc aaagcataat 600

ggggtggcac caaaattgag ttgggaaatg gaacaacaac ttggctacac aacaacaaaa 660

ttggaaattg ctcgttaa 678

<210> 6
<211> 720
<212> DNA
<213> Artificial Sequence

<220>
<223> sGFP

<400> 6
atggtgagca agggcgagga gctgttcacc ggggtggtgc ccatcctggt cgagctggac 60

ggcgacgtaa acggccacaa gttcagcgtg tccggcgagg gcgagggcga tgccacctac 120

ggcaagctga ccctgaagtt catctgcacc accggcaagc tgcccgtgcc ctggcccacc 180

ctcgtgacca ccttcagcta cggcgtgcag tgcttcagcc gctaccccga ccacatgaag 240

cagcacgact tcttcaagtc cgccatgccc gaaggctacg tccaggagcg caccatcttc 300

ttcaaggacg acggcaacta caagacccgc gccgaggtga agttcgaggg cgacaccctg 360

gtgaaccgca tcgagctgaa gggcatcgac ttcaaggagg acggcaacat cctggggcac 420

aagctggagt acaactacaa cagccacaac gtctatatca tggccgacaa gcagaagaac 480

ggcatcaagg tgaacttcaa gatccgccac aacatcgagg acggcagcgt gcagctcgcc 540

gaccactacc agcagaacac ccccatcggc gacggccccg tgctgctgcc cgacaaccac 600

tacctgagca cccagtccgc cctgagcaaa gaccccaacg agaagcgcga tcacatggtc 660

ctgctggagt tcgtgaccgc cgccgggatc actcacggca tggacgagct gtacaagtaa 720

<210> 7
<211> 732
<212> DNA
<213> Lycopersicon esculentum

<400> 7
atgctgccaa gaagatatcc tcagatggat gctaatccta gtaatggggg tgaaagggat 60

aatgctttgc gaggaattct gcaggactta tggccactgg atgaaattga tccaagcact 120

caaaagttcc cttgttgcct tgtttggact cctctccctg tgatttcttg gcttgcacct 180

tttgttggac atgttggcat atgcagggag gatggtacca ttgtggattt ttctggagat 240

agcatgattc attttggtca gctcttctat ggaactgtag ccaaatacta tcaggtagac 300

agacagcagt gctgttttgc tcgcaacttt ggtggacaca catgccgtaa gggttatgaa 360

catgttgtat ttgggacagc agtaagttgg gatgatgctg ttcagttgtt taggcgcacc 420

tttgagaaca gaaacttcaa agttttcagt tgcaacggcc actcattcgc tgctgattgc 480

ctgaacctgc tatcatttag aggatcaatg cgctggaaca tgattaatgt tggagctctt 540

ataatgtttg agggaaagtg ggtcagtcgc tggtcaatgt tacgatcatt tctgcctttc 600

attgggatac tttgcttcgg ctatttaatg attggatgga tgtttccaat tggtctgctc 660

tcctttgtta ttgggacttt tggatggtat gtcatgatct gttactgttg caagattgag 720

gatgacaatt ag 732

<210> 8
<211> 903
<212> DNA
<213> Lycopersicon esculentum

<400> 8
atggctatta tggatgaagc tgctaatatg gtttgtgtgc cgttggatta tagtagaaag 60

aggaaatcaa ggagtagaag ggacagaaca aaaaatgtgg aagagacact agctaaatgg 120

aaggagtata atgagaaact agacaatgaa gggaaaggga agccagtgcg taaagttcct 180

gctaaaggtt caaagaaggg gtgtatgaga ggtaaagggg gaccagaaaa ttggcggtgt 240

aaatacagag gtgttagaca gaggatatgg ggtaaatggg ttgctgagat tagggaacct 300

aaaagaggta gtaggttatg gttgggtaca tttggtacag caattgaagc tgctttagca 360

tatgatgatg ctgcaagagc tatgtatggt ccttgtgcaa ggcttaattt gccaaattac 420

gcgtgtgatt ctgtttcctg ggcaactaca tctgcatctg catctgcatc tgattgcacc 480

gttgcttctg gtttcggcga ggtatgtccg gttgatggtg ctcttcatga agctgacaca 540

ccattgagct cagtgaaaga cgaagggacc gcgatggata ttgttgaacc tacgagtatt 600

gatgaagata cgcttaagtc tggatgggat tgtctagata aattaaatat ggatgagatg 660

tttgatgtag atgagctatt ggctatgtta gattctactc cagttttcac caaggactac 720

aattcagatg gaaagcacaa caatatggta tcagattcgc aatgtcagga gccgaatgca 780

gtggtagatc ctatgactgt tgactatggc tttgattttc tgaaaccagg caggcaagaa 840

gatcttaatt tcagttcgga tgaccttgca ttcatagact tggattctga acttgtcgtt 900

tga 903

<210> 9
<211> 1059
<212> DNA
<213> Lycopersicon esculentum

<400> 9
atgggaaaaa gtttgaagct tcggttctcc agagttattg cttctttcaa ttcgtgccgt 60

tcgaaaaacc cttcttctct tccccaaaat cctaatttct tcccacataa gctcactagt 120

acaaaacaca tttcccccga tttccctctt attgatcaaa atcaaaatca aaatcaccgt 180

aattacgtgc cagaatccac gatgatctcc gttgggtgtt gtagatcaga attcaagtgg 240

gagaaagaag agaagtttca cgtggtttct agttccttcg tgtctgaaga agaagaatgt 300

gaagaggaga tcaatttggc cttacgacct cctcttacac ctccgcgatt cagtagaatt 360

gttgttgaga agaagaagaa gaaacaacag cgagttaaaa aaacgaaaac aaaaagtaga 420

atcatccgaa tgagtacttc ctcagctgat gagtacagcg ggatattaag cggtactaat 480

actgattggg ataataatga agaggaaact gaatctttag tttcatcttc cagaagctgt 540

tacgatttct caagcgatga ctcatctact gatttcaacc ctcacttaga aaccatatgt 600

gagaccacta caatgaggcg tcgtcacaag agaaatgcca acaccaagag gagatcaatc 660

aagcaatcca gaccaagttt ttcctcttca aaaggtagaa gatcgtcggt ttctacgtca 720

tcagatagcg agctaccggc aaggttatcg gtgtttaaga agctgatacc gtgtagtgtg 780

gatgggaaag tgaaggagag tttcgcgata gtgaagaaat ctcaggaccc gtacgaagat 840

ttcaagagat cgatgatgga aatgatttta gagaaggaaa tgtttgagaa gaatgagctg 900

gaacagcttt tacaatgttt tctgtcgttg aacggaaagc attatcatgg agtgatagtt 960

gaggcgttct cagacatttg ggagactttg tttttaggta ataatgatag agtaaggagg 1020

atgtcaattc atgatcccac acccacctac tgtaggtag 1059

<210> 10
<211> 1266
<212> DNA
<213> Lycopersicon esculentum

<400>  10
atgggaaagc gaagaaactg gtttaccttt gtcaagagac ttttcattcc tgaaacagaa    60

tcaacagcag atcaaaagaa accaaagaga tggagatgtt gttttctgag aaagttcaag   120

ttgaggaaat gtcctgctat aacatcagca cctcagcaaa cgttacctga ggcgaaagga   180

acacctcagc aaacgttaac tgaggcgaaa gaacagcaaa gaaaacatgc ttttgcagtt   240

gctatagcaa cggcagcagc tgctgaggct gctgtagctg ctgctaatgc tgctgctgat   300

gttattcgtc taacagatgc tccaagtgaa ttcaaaagga aacgcaaaca agctgctatt   360

agaatccaaa gtgcttatcg cgctcacctg gcccagaaag cattaagggc tctaaagggt   420

gttgtgaagc ttcaagcagt gattagaggt gaaattgtga gaggaagact cattgccaaa   480

ctgaagttca tgttgccact tcatcaaaag tcaaaaacaa gagttaatca aattagagtc   540

cctacttttg aagatcatca tgacaagaaa ctcatcaata gtccaaggga aattatgaaa   600

gctaaagaac taaagcttaa atgcaagagc cttagcactt ggaatttcaa cttagcttca   660

gaacaagaca gtgaagcctt gtggtcaaga agagaagaag ccattgacaa aagagagcat   720

ttgatgaaat actcgttttc acatcgggag agaagaaacg atcaaactct acaagactta   780

ctaaacagaa agcaaaacag aagaagctac aggattgacc agttagtaga acttgacgca   840

ccaagaaaag caggggttgtt agagaaattg agatcattta cagactcaaa tgttcctcta   900

actgatatgg atggaatgac acagcttcaa gtgagaaaaa tgcatagatc agattgtata   960

gaggacctac attctccttc ttcacttcca agaagatcat tttctaatgc aaaacgaaaa  1020

tcaaacgttg atgataactc attaccaagt tctcctatat ttcctactta catggcagcc  1080

acagaatctg caaaggcaaa aacaaggtca aacagcacag cgaagcaaca cctaaggtta  1140

cacgagacat tgtcaggtca acattctcct tataacctca agatttcttc ttggagattg  1200

tctaatggtg aaatgtatga cagcgccaga acaagcagaa cttctagcag ttatatgtta  1260

atatag                                                             1266

<210>  11
<211>  1812
<212>  DNA
<213>  Artificial Sequence

<220>
<223>  GUS, nucleotide exchanges G835C and G903A

<400>  11
atgttacgtc ctgtagaaac cccaacccgt gaaatcaaaa aactcgacgg cctgtgggca    60

ttcagtctgg atcgcgaaaa ctgtggaatt gatcagcgtt ggtgggaaag cgcgttacaa   120

gaaagccggg caattgctgt gccaggcagt tttaacgatc agttcgccga tgcagatatt   180

cgtaattatg cgggcaacgt ctggtatcag cgcgaagtct ttataccgaa aggttgggca   240

ggccagcgta tcgtgctgcg tttcgatgcg gtcactcatt acggcaaagt gtgggtcaat 300

aatcaggaag tgatggagca tcagggcggc tatacgccat ttgaagccga tgtcacgccg 360

tatgttattg ccgggaaaag tgtacgtatc accgtttgtg tgaacaacga actgaactgg 420

cagactatcc cgccgggaat ggtgattacc gacgaaaacg gcaagaaaaa gcagtcttac 480

ttccatgatt tctttaacta tgccggaatc catcgcagcg taatgctcta caccacgccg 540

aacacctggg tggacgatat caccgtggtg acgcatgtcg cgcaagactg taaccacgcg 600

tctgttgact ggcaggtggt ggccaatggt gatgtcagcg ttgaactgcg tgatgcggat 660

caacaggtgg ttgcaactgg acaaggcact agcgggactt tgcaagtggt gaatccgcac 720

ctctggcaac cgggtgaagg ttatctctat gaactgtgcg tcacagccaa aagccagaca 780

gagtgtgata tctacccgct tcgcgtcggc atccggtcag tggcagtgaa gggccaacag 840

ttcctgatta accacaaacc gttctacttt actggctttg gtcgtcatga agatgcggac 900

ttacgtggca aaggattcga taacgtgctg atggtgcacg accacgcatt aatggactgg 960

attggggcca actcctaccg tacctcgcat tacccttacg ctgaagagat gctcgactgg 1020

gcagatgaac atggcatcgt ggtgattgat gaaactgctg ctgtcggctt taacctctct 1080

ttaggcattg gtttcgaagc gggcaacaag ccgaaagaac tgtacagcga agaggcagtc 1140

aacggggaaa ctcagcaagc gcacttacag gcgattaaag agctgatagc gcgtgacaaa 1200

aaccacccaa gcgtggtgat gtggagtatt gccaacgaac cggatacccg tccgcaaggt 1260

gcacgggaat atttcgcgcc actggcggaa gcaacgcgta aactcgaccc gacgcgtccg 1320

atcacctgcg tcaatgtaat gttctgcgac gctcacaccg ataccatcag cgatctcttt 1380

gatgtgctgt gcctgaaccg ttattacgga tggtatgtcc aaagcggcga tttggaaacg 1440

gcagagaagg tactggaaaa agaacttctg gcctggcagg agaaactgca tcagccgatt 1500

atcatcaccg aatacggcgt ggatacgtta gccgggctgc actcaatgta caccgacatg 1560

tggagtgaag agtatcagtg tgcatggctg gatatgtatc accgcgtctt tgatcgcgtc 1620

agcgccgtcg tcggtgaaca ggtatggaat ttcgccgatt ttgcgacctc gcaaggcata 1680

ttgcgcgttg gcggtaacaa gaaagggatc ttcactcgcg accgcaaacc gaagtcggcg 1740

gcttttctgc tgcaaaaacg ctggactggc atgaacttcg gtgaaaaacc gcagcaggga 1800

ggcaaacaat ga 1812

<210> 12
<211> 2193
<212> DNA
<213> Lycopersicon esculentum

<400> 12
atgtttaata accaccagca cttgctcgat atatcgtcct cagctcaacg aacacctgat 60

```
aacgagttgg atttcattcg tgatgaagag tttgatagca actctggtgc tgataacatg      120
gaagctccca attcaggtga tgacgatcaa gctgatccaa accaacctcc aaacaagaag      180
aagcgttatc atcgccacac tcagaatcag attcaggaaa tggagtcctt ttacaaggaa      240
tgcaatcatc cagatgacaa gcaaaggaag gaattgggaa gaagacttgg tttggagcca      300
ttacaagtga aattttggtt ccagaacaag cgtactcaga tgaaggctca acatgagcga      360
tgtgagaaca cacagttgag gaatgaaaat gagaagcttc gcgctgagaa cataaggtac      420
aaagaagctt tgagtaatgc agcatgccca aattgtggag ggccagcagc tataggagag      480
atgtcatttg atgagcatca gttgaggatt gaaaatgctc gtcttagaga tgagattgac      540
aggataactg gaatagctgg aaagtatgtt ggtaaatcag cccttggata ttctcatcaa      600
cttcctcttc ctcagcccga agctcctcgg gttctggatc ttgcttttgg gcctcaatcg      660
ggcctgcttg gagaaatgta cgctgctggt gaccttctaa gaactgctgt tacgggcctt      720
acagatgctg agaagcccgt ggtcattgag cttgctgtta ctgcaatgga ggaacttata      780
aggatggctc aaactgaaga gccattatgg ttgccaagct caggctctga gactttatgt      840
gagcaagaat atgctcgtat ttccctcga ggccttggac ctaagccagc tacactcaat       900
tctgaagcct cacgagaatc tgctgttgtg attatgaatc atatcaattt agttgagatt      960
ttgatggatg tgaaccaatg gactactgtt tttgctggtc tggtgtcaaa agcaatgact     1020
cttgaagtct tatcaactgg tgtcgcagga aatcacaatg gagcattgca agtgatgaca     1080
gcagaatttc aagttccatc tccacttgtt ccaactcggg agaactattt cttaagatac     1140
tgtaaacaac atggtgaagg gacttgggta gtggttgatg tttccctgga caacttgcgc     1200
actgtttcag ttccgcgttg cagaagaagg ccatctggtt gtttaatcca agaaatgcca     1260
aatggttact caagggttat atgggttgaa cacgttgagg tggatgaaaa tgctgtccat     1320
gacatctaca aacctcttgt caattctggg attgcatttg gagcaaaacg ctgggtagca     1380
actttagata gacaatgtga acgccttgca agtgtgttgg cgcttaacat cccaacagga     1440
gatgttggaa tcattactag tccagctggt cgaaagagta tgctaaaact tgctgagaga     1500
atggtgatga gcttttgtgc tggagttggt gcatcgacaa ctcacatatg gacaactttg     1560
tctggaagtg gtgcggatga tgttagagtc atgactagga agagtatcga tgatccaggg     1620
agacctcctg gtattgtgct gagtgctgca acatcttttt ggcttccagt ttctcctaag     1680
agagtgtttg attttctccg cgatgagaac tctagaaatg agtgggatat tctttcaaat     1740
ggtgggattg ttcaggaaat ggcacacatt gcaaatggtc gtgatccagg aaactgtgtt     1800
tctctactcc gtgtcaatac tggaacaaac tctaaccaga gtaacatgct gatactccaa     1860
gagagcacaa ctgatgtaac aggatcttac gtcatttacg ctccagttga tattgctgca     1920
```

atgaacgtgg tgttaggtgg gggtgaccct gactatgttg ctctgttgcc atctggtttt 1980

gctattcttc cagacggacc gatgaattat catggtggag gtaattcaga aattgattct 2040

cctggtggat cgctactaac tgtagcattt cagatattgg ttgattcagt cccaactgca 2100

aagctttccc ttggctctgt tgcgactgtt aatagtctca tcaaatgcac cgttgaaaag 2160

atcaaaggtg ctgtaacttc cgcaaatgca tga 2193

<210> 13
<211> 825
<212> DNA
<213> Lycopersicon esculentum

<400> 13
atgaacagta catctatgtc ttcattggga gtgagaaaag gttcatggac tgatgaagaa 60

gattttcttc taagaaaatg tattgataag tatggtgaag gaaaatggca tcttgttccc 120

ataagagctg gtctgaatag atgtcggaaa agttgtagat tgaggtggct gaattatcta 180

aggccacata tcaagagagg tgactttgaa caagatgaag tggatctcat tttgaggctt 240

cataagctct taggcaacag atggtcactt attgctggta gacttcccgg aaggacagct 300

aacgatgtga aaaactattg gaacactaat cttctaagga agttaaatac tactaaaatt 360

gttcctcgcg aaaagattaa caataagtgt ggagaaatta gtactaagat tgaaattata 420

aaacctcaac gacgcaagta tttctcaagc acaatgaaga atgttacaaa caataatgta 480

attttggacg aggaggaaca ttgcaaggaa ataataagtg agaaacaaac tccagatgca 540

tcgatggaca acgtagatcc atggtggata aatttactgg aaaattgcaa tgacgatatt 600

gaagaagatg aagaggttgt aattaattat gaaaaaacac taacaagttt gttacatgaa 660

gaaatatcac caccattaaa tattggtgaa ggtaactcca tgcaacaagg acaaataagt 720

catgaaaatt ggggtgaatt ttctcttaat ttaccaccca tgcaacaagg agtacaaaat 780

gatgattttt ctgctgaaat tgacttatgg aatctacttg attaa 825

<210> 14
<211> 825
<212> DNA
<213> Artificial Sequence

<220>
<223> SlANT1 with Nicotiana tabacum codon usage

<400> 14
atgaattcta caagtatgtc aagcttaggc gttcgtaagg gatcttggac agatgaagaa 60

gatttccttc tacgaaagtg tattgacaaa tatggtgagg gaaaatggca tttggttccg 120

attagagctg gtttgaatcg atgcaggaaa tcctgtagac ttaggtggtt gaactatctt 180

agacctcaca taaagagagg tgatttcgag caagatgaag tggatctcat actcagacta 240

cacaaacttt tagggaatcg ttggagtctt attgcaggca gattaccagg tagaacagcc 300

aatgatgtca agaactattg gaatactaat cttttaagga agttgaacac tacaaagata 360

gtaccaaggg agaaaatcaa caacaaatgt ggggaaattt ctacgaaaat tgagattatc 420

aagccccaaa gacgtaagta cttttcatcc actatgaaga atgtcaccaa caacaatgtt 480

atcctcgacg aagaagaaca ttgcaaagag atcatttctg agaagcagac tcctgatgct 540

tcaatggaca acgttgatcc ttggtggata aatcttctag agaattgcaa cgatgatata 600

gaagaggatg aagaagtggt gattaactac gagaaaacct taactagcct gttgcatgaa 660

gaaatctctc caccccttaa tattggagaa ggaaattcaa tgcaacaagg ccagatttct 720

catgagaatt ggggtgaatt ttccttgaat ctgccaccta tgcagcaagg agtacagaat 780

gacgacttta gtgcagagat tgatctctgg aatctgttgg actaa 825

<210> 15
<211> 825
<212> DNA
<213> Artificial Sequence

<220>
<223> SlANT1 with Arabidopsis thaliana codon usage

<400> 15
atgaattcaa catcaatgtc tagtctagga gtaaggaaag gttcatggac agatgaagag 60

gactttcttc tccggaaatg cattgataag tatggggaag gaaaatggca tttagtcccc 120

attagagctg gcttgaatcg ttgtaggaaa tcgtgtcgac tcagatggct aaactatctt 180

agaccgcata tcaagcgggg tgatttcgaa caggacgaag tggacttgat tttgaggctt 240

cacaagttat tgggtaatcg ttggtccctt atagctggga gattaccagg tagaacagcc 300

aatgatgtga agaattactg gaatacgaac ttgctgagaa aactcaacac taccaagatc 360

gttccgagag aaaagatcaa caacaaatgt ggcgagatta gcacgaagat agagatcata 420

aagcctcaac gtcgaaaata cttctctagc actatgaaga atgtcaccaa taacaacgtg 480

atactagatg aagaagaaca ctgtaaggag attatcagtg agaaacagac tcctgatgca 540

tctatggaca atgttgatcc ttggtggatt aaccttctgg agaattgcaa tgacgatatt 600

gaggaggatg aagaggttgt aatcaactat gagaaaacac ttacttcact ccttcatgaa 660

gagatatctc caccacttaa cattggagag ggtaactcca tgcaacaagg acagatctct 720

catgaaaatt ggggagaatt ttcgctgaat ttgcctccaa tgcaacaagg agttcagaac 780

gacgatttta gtgcggaaat tgatctctgg aacttattgg attaa 825

<210> 16
<211> 825
<212> DNA
<213> Artificial Sequence

<220>
<223> SlANT1 with Potato Virus X codon usage

<400> 16
atgaatagca ctagcatgtc aagcttaggt gtgagaaagg gctcatggac tgacgaagag 60

gatttcctgt tgaggaagtg catcgacaag tatggagaag gcaaatggca ccttgtaccg 120

attagggcag ggcttaacag gtgcaggaaa agctgtaggt tgaggtggtt gaactatctc 180

agaccccata taaagagagg cgactttgag caagatgaag tggacctaat tcttcgctta 240

cacaaactcc ttgggaatag gtggagtctg atagctggaa ggctacctgg tagaacagct 300

aacgacgtga agaactactg gaataccaac ctattacgca aactgaacac taccaaaatc 360

gttcccagag agaagatcaa caacaagtgt ggcgagataa gcacgaagat cgaaatcatc 420

aaaccgcaaa gaaggaagta cttcagttca accatgaaga atgtcacaaa caacaatgtc 480

atactggatg aagaagagca ctgcaaggag attatttccg agaaacagac accagacgca 540

tccatggaca atgtcgatcc atggtggatt aacctactcg aaaattgcaa cgatgacatt 600

gaagaggatg aggaagtagt gatcaactac gagaaaacac tgacttctct cttgcatgag 660

gagatcagtc cacctttgaa cattggagaa gggaattcta tgcaacaagg acagataagc 720

cacgaaaatt ggggagagtt ttccctcaat ctcccaccta tgcaacaggg tgttcagaac 780

gatgacttct cagccgaaat cgacttatgg aacctactcg actaa 825

<210> 17
<211> 825
<212> DNA
<213> Artificial Sequence

<220>
<223> SlANT1 with Homo sapiens codon usage

<400> 17
atgaattcta cgtccatgtc tagcctcggg gttaggaaag gctcatggac agacgaagag 60

gactttctgc tgcgcaaatg catagacaag tatggcgaag gaaagtggca tctggtgccc 120

attagggctg gtctgaaccg gtgtcgcaag tcctgtaggt tgcggtggct taactacctc 180

agaccccaca tcaaacgagg cgatttcgaa caggatgagg tcgacctgat tctccgtctg 240

cacaagctgt tgggtaacag atggagcctc attgcaggga gactccctgg aagaactgcc 300

aatgacgtca agaactactg gaacaccaac cttcttcgca agctgaatac cactaagatc 360

gttcctcgag agaagatcaa caacaaatgt ggagaaatat ccaccaaaat cgagatcatc 420

aagccacaac ggaggaaata cttctccagc acaatgaaga atgtgaccaa caacaacgtg 480

attttggacg aagaggagca ttgcaaagag atcatcagtg agaagcagac acctgatgcc 540

tctatggata atgtggaccc ctggtggata aatctgctgg agaattgcaa tgatgacatt 600

```
gaagaagatg aggaagtggt catcaactat gagaaaacac tgacttcact gctgcatgaa      660

gagattagtc caccgctgaa cattggggag gggaatagca tgcagcaggg acagatcagt      720

cacgaaaatt ggggcgaatt cagccttaat ctcccaccca tgcaacaggg cgtacagaac      780

gacgactttt cagcggagat tgatctgtgg aatttgctgg attaa                      825
```

<210> 18
<211> 825
<212> DNA
<213> Artificial Sequence

<220>
<223> SlANT1 with Oryza sativa codon usage

<400> 18

```
atgaattcaa cgagcatgag ctcgttgggt gttcgcaaag gctcttggac cgatgaagag       60

gacttcctct tgcgaaagtg catcgataag tatggggaag gaaagtggca tcttgtaccc      120

atacgtgcgg gacttaaccg gtgtcgcaag tcgtgcagac tcaggtggct caactatcta      180

cggcctcaca tcaaacgtgg cgatttcgaa caagacgagg ttgaccttat cctgagactg      240

cacaaactgc tcggcaatcg ctggagtctc atagctggtc gattgcctgg gaggactgcc      300

aatgacgtca agaattactg gaatacaaac cttctgagga agctgaatac cacgaagata      360

gttcctcggg agaagatcaa caacaagtgt ggggagattt ccacgaaaat cgagatcatc      420

aagccgcaaa ggcgcaaata cttctcaagc acaatgaaga acgtcaccaa caacaacgtg      480

attctcgatg aggaggaaca ctgcaaggag atcatctctg agaaacagac tccagatgcc      540

tcaatggaca atgtggatcc gtggtggatt aacctcctgg agaactgcaa tgatgacatt      600

gaagaggacg aagaggtcgt gatcaactac gaaaagaccc tcacatctct cctccatgag      660

gaaataagtc caccgctcaa tattggcgaa ggcaattcca tgcagcaagg ccagatttcg      720

catgagaact ggggtgagtt ttccctgaat ctaccaccca tgcagcaagg agtgcagaat      780

gatgactttt ccgcagagat tgacttgtgg aacttgcttg attaa                      825
```

<210> 19
<211> 825
<212> DNA
<213> Artificial Sequence

<220>
<223> SlANT1 with Hordeum vulgare codon usage

<400> 19

```
atgaatagca cctccatgtc ctctctgggc gttcgtaagg ggtcatggac agatgaggag       60

gacttcttgc tccgcaaatg catcgacaag tatggcgaag gcaaatggca tcttgtcccg      120

ataagggccg gactcaaccg ctgcagaaag tcttgccgcc ttaggtggct aaactaccta      180

cggccccaca ttaagcgggg tgactttgag caggatgagg tagacttgat cttgcggcta      240
```

cacaagcttc tgggcaatag gtggtcactg attgccggta gactccctgg tcgcactgcg 300

aatgacgtga agaactactg gaacaccaat ctgctccgca aactcaacac caccaagatc 360

gtcccacgtg agaagatcaa caacaagtgt ggcgagatca gcaccaagat cgagatcatc 420

aagccacaac ggaggaagta cttctcctct acgatgaaga atgtgacgaa caacaacgtg 480

attctcgacg aagaggagca ctgtaaggag atcatctccg agaaacagac tcccgatgct 540

tcgatggaca atgtcgatcc gtggtggatt aacctcctgg agaattgcaa cgatgacata 600

gaagaggacg aagaagtcgt gatcaactac gaaaagacgc tgacaagcct cttgcacgag 660

gagatatcgc cacccctcaa cattggagag gggaacagca tgcagcaagg gcagatcagt 720

catgaaaact ggggagagtt cagcctcaat cttcctccga tgcagcaagg cgttcagaac 780

gatgacttca gtgcagagat tgacctgtgg aaccttctcg attaa 825

<210> 20
<211> 825
<212> DNA
<213> Artificial Sequence

<220>
<223> SlANT1 with Bifidobacterium codon usage

<400> 20
atgaactcca cctccatgtc ctcgctcggc gttcgcaaag gcagctggac cgatgaggag 60

gacttcctcc tgcgcaagtg catcgacaag tacggagaag gcaaatggca ccttgtcccc 120

attcgcgctg gtctgaaccg ctgtcgcaag agctgccgtt tgcggtggct gaactatctg 180

cgtccgcaca tcaagcgcgg cgacttcgag caggacgaag tcgacctgat tctgcgcctg 240

cataagctgc tggggaaccg ctggtccctg attgccggcc ggttgcccgg taggaccgcg 300

aacgacgtga agaactactg gaacaccaac ctccttcgca agctgaatac cacgaagatc 360

gtgccgaggg agaagatcaa caacaaatgc ggggaaatct cgacgaagat cgagatcatc 420

aagccccaac gtcggaagta cttcagcagc accatgaaga acgtgacgaa caacaacgtg 480

atcctggacg aagaggaaca ctgcaaggag atcatctcgg agaagcagac tccggatgcc 540

tccatggaca acgtggatcc gtggtggatc aatctgctgg agaactgcaa cgacgacatc 600

gaggaggatg aggaagtcgt gatcaactac gaaaagacct tgacgtccct cctccatgag 660

gagatttccc ctccgctgaa catcggcgag ggcaactcca tgcaacaggg ccagatctcc 720

cacgagaatt ggggcgaatt ctcgctgaat ctcccgccga tgcagcaggg agtccagaac 780

gacgacttta gcgccgaaat cgacctctgg aaccttctcg attaa 825

<210> 21
<211> 678
<212> DNA

<213>  Artificial Sequence

<220>
<223>  SlLOG1 with Oryza sativa codon usage

<400>  21
atggagaaca accatcaaac gcagattcag actaccaaga cttctcgctt caagcgcatt    60

tgcgtgttct gtgggtcaag tccaggcaag aagccctcct atcagcttgc tgccatccag    120

ctggggaatc agctggttga acggaatatc gatctcgtct atggtggagg ctctgttggc    180

ctaatgggac tcgtgagcca atccgtgttc aatggtggtc gacatgtcct cggcgtgata    240

ccgaaaaccc tgatgcccag agagatcacg ggagagtcag tcggagaagt ccgggctgtt    300

tctggcatgc atcagaggaa agccgagatg gcacgtcaag ccgatgcgtt tatagcgctt    360

cctggcggtt acggaaccct cgaagagcta ctggaggtga ttacatgggc tcagttgggc    420

atacacgaca aaccagttgg cctcttgaac gtggatgggt actacaactc gttgctttcg    480

ttcatcgaca aggcagtaga cgaggggttt gtgacaccat ccgcaagaca catcatcatt    540

agtgcgccta cagcccaaga actcatgagc aagcttgagg actatgtccc gaagcacaat    600

ggggtagccc cgaaactgag ctgggagatg gaacaacagc tcggctacac gactaccaag    660

ctcgagattg cgaggtga    678

<210>  22
<211>  1059
<212>  DNA
<213>  Artificial Sequence

<220>
<223>  SlOVATE with Oryza sativa codon usage

<400>  22
atgggcaaaa gtctcaagct gcgcttttct cgtgtgattg ccagcttcaa ttcgtgcaga    60

tctaagaatc ccagctcact tccgcaaaat ccgaacttct ttccccacaa gcttacatcg    120

acaaaacaca tctctccaga ctttccgctg attgaccaga accagaacca gaatcacagg    180

aactacgttc ctgagtcgac catgatcagt gtgggctgtt gcagatccga attcaagtgg    240

gagaaagagg agaagtttca cgtggtatca agctcgttcg tttccgagga agaggagtgt    300

gaagaagaga tcaaccttgc tctacgtcca ccgctaacac caccgcgctt ctcaaggata    360

gttgtcgaga agaagaagaa gaaacagcaa cgggtgaaga aaacgaaaac caaatcccgc    420

atcattcgca tgtccacttc atctgcggat gagtacagtg ggatcttgag cggtaccaac    480

acagattggg acaacaatga ggaggaaacc gaaagtctgg tgtccagctc aaggagctgt    540

tacgacttct cgagtgatga ctcgtccacg gatttcaatc cgcatttgga gactatttgc    600

gaaactacga caatgagaag gcggcataaa aggaatgcca acacgaagcg acgctctatc    660

aaacaaagcc gaccttcatt ctcctcaagc aagggacgca gaagctccgt gtcgacctcc    720

tcagactctg agctcccagc taggctcagt gtctttaaga agctcattcc ttgctctgtg 780

gatggaaagg tcaaggagtc cttcgcaatc gtcaagaaat cgcaagatcc ctatgaggac 840

ttcaagcggt ctatgatgga gatgatcctg gagaaggaaa tgtttgagaa gaatgagctc 900

gaacagcttc tccagtgctt cctctccctc aacggcaagc attaccatgg tgtcatagtt 960

gaagcgttta gcgacatatg ggaaacgctg ttcttgggga ataacgatcg ggtacgtcga 1020

atgagcattc acgatcctac tcccacctat tgccggtga 1059

<210> 23
<211> 11362
<212> DNA
<213> Artificial Sequence

<220>
<223> pNMD674

<400> 23
cttctgtcag cgggcccact gcatccaccc cagtacatta aaaacgtccg caatgtgtta 60

ttaagttgtc taagcgtcaa tttgtttaca ccacaatata tcctgccacc agccagccaa 120

cagctccccg accggcagct cggcacaaaa tcaccactcg atacaggcag cccatcagtc 180

agatcaggat ctcctttgcg acgctcaccg ggctggttgc cctcgccgct gggctggcgg 240

ccgtctatgg ccctgcaaac gcgccagaaa cgccgtcgaa gccgtgtgcg agacaccgcg 300

gccgccggcg ttgtggatac ctcgcggaaa acttggccct cactgacaga tgaggggcgg 360

acgttgacac ttgaggggcc gactcacccg gcgcggcgtt gacagatgag gggcaggctc 420

gatttcggcc ggcgacgtgg agctggccag cctcgcaaat cggcgaaaac gcctgatttt 480

acgcgagttt cccacagatg atgtggacaa gcctggggat aagtgccctg cggtattgac 540

acttgagggg cgcgactact gacagatgag gggcgcgatc cttgacactt gaggggcaga 600

gtgctgacag atgaggggcg cacctattga catttgaggg gctgtccaca ggcagaaaat 660

ccagcatttg caagggtttc cgcccgtttt tcggccaccg ctaacctgtc ttttaacctg 720

cttttaaacc aatatttata aaccttgttt ttaaccaggg ctgcgccctg tgcgcgtgac 780

cgcgcacgcc gaaggggggt gcccccccttt ctcgaaccct cccggcccgc taacgcgggc 840

ctcccatccc cccaggggct gcgcccctcg gccgcgaacg gcctcacccc aaaaatggca 900

gcgctggcca attcgtgcgc ggaacccccta tttgtttatt tttctaaata cattcaaata 960

tgtatccgct catgagacaa taaccctgat aaatgcttca ataatattga aaaaggaaga 1020

gtatggctaa aatgagaata tcaccggaat tgaaaaaact gatcgaaaaa taccgctgcg 1080

taaaagatac ggaaggaatg tctcctgcta aggtatataa gctggtggga gaaaatgaaa 1140

acctatattt aaaaatgacg gacagccggt ataaagggac cacctatgat gtggaacggg 1200

aaaaggacat gatgctatgg ctggaaggaa agctgcctgt tccaaaggtc ctgcactttg 1260
aacggcatga tggctggagc aatctgctca tgagtgaggc cgatggcgtc ctttgctcgg 1320
aagagtatga agatgaacaa agccctgaaa agattatcga gctgtatgcg gagtgcatca 1380
ggctctttca ctccatcgac atatcggatt gtccctatac gaatagctta gacagccgct 1440
tagccgaatt ggattactta ctgaataacg atctggccga tgtggattgc gaaaactggg 1500
aagaagacac tccatttaaa gatccgcgcg agctgtatga tttttaaag acggaaaagc 1560
ccgaagagga acttgtcttt tcccacggcg acctgggaga cagcaacatc tttgtgaaag 1620
atggcaaagt aagtggcttt attgatcttg ggagaagcgg cagggcggac aagtggtatg 1680
acattgcctt ctgcgtccgg tcgatcaggg aggatatcgg ggaagaacag tatgtcgagc 1740
tattttttga cttactgggg atcaagcctg attgggagaa aataaaatat tatattttac 1800
tggatgaatt gttttagctg tcagaccaag tttactcata tatactttag attgatttaa 1860
aacttcattt ttaatttaaa aggatctagg tgaagatcct ttttgataat ctcatgacca 1920
aaatccctta acgtgagttt tcgttccact gagcgtcaga ccccgtagaa aagatcaaag 1980
gatcttcttg agatcctttt tttctgcgcg taatctgctg cttgcaaaca aaaaaaccac 2040
cgctaccagc ggtggtttgt ttgccggatc aagagctacc aactcttttt ccgaaggtaa 2100
ctggcttcag cagagcgcag ataccaaata ctgtccttct agtgtagccg tagttaggcc 2160
accacttcaa gaactctgta gcaccgccta catacctcgc tctgctaatc ctgttaccag 2220
tggctgctgc cagtggcgat aagtcgtgtc ttaccgggtt ggactcaaga cgatagttac 2280
cggataaggc gcagcggtcg ggctgaacgg ggggttcgtg cacacagccc agcttggagc 2340
gaacgaccta caccgaactg agatacctac agcgtgagct atgagaaagc gccacgcttc 2400
ccgaagggag aaaggcggac aggtatccgg taagcggcag ggtcggaaca ggagagcgca 2460
cgagggagct tccaggggga aacgcctggt atctttatag tcctgtcggg tttcgccacc 2520
tctgacttga gcgtcgattt ttgtgatgct cgtcaggggg gcggagccta tggaaaaacg 2580
ccagcaacgc ggccttttta cggttcctgg cagatcctag atgtggcgca acgatgccgg 2640
cgacaagcag gagcgcaccg acttcttccg catcaagtgt tttggctctc aggccgaggc 2700
ccacggcaag tatttgggca aggggtcgct ggtattcgtg cagggcaaga ttcggaatac 2760
caagtacgag aaggacggcc agacggtcta cgggaccgac ttcattgccg ataaggtgga 2820
ttatctggac accaaggcac caggcgggtc aaatcaggaa taagggcaca ttgccccggc 2880
gtgagtcggg gcaatcccgc aaggagggtg aatgaatcgg acgtttgacc ggaaggcata 2940
caggcaagaa ctgatcgacg cggggttttc cgccgaggat gccgaaacca tcgcaagccg 3000
caccgtcatg cgtgcgcccc gcgaaacctt ccagtccgtc ggctcgatgg tccagcaagc 3060
tacggccaag atcgagcgcg acagcgtgca actggctccc cctgccctgc ccgcgccatc 3120

```
ggccgccgtg gagcgttcgc gtcgtctcga acaggaggcg gcaggtttgg cgaagtcgat      3180
gaccatcgac acgcgaggaa ctatgacgac caagaagcga aaaaccgccg gcgaggacct      3240
ggcaaaacag gtcagcgagg ccaagcaggc cgcgttgctg aaacacacga agcagcagat      3300
caaggaaatg cagctttcct tgttcgatat tgcgccgtgg ccggacacga tgcgagcgat      3360
gccaaacgac acggcccgct ctgccctgtt caccacgcgc aacaagaaaa tcccgcgcga      3420
ggcgctgcaa aacaaggtca ttttccacgt caacaaggac gtgaagatca cctacaccgg      3480
cgtcgagctg cgggccgacg atgacgaact ggtgtggcag caggtgttgg agtacgcgaa      3540
gcgcacccct atcggcgagc cgatcacctt cacgttctac gagctttgcc aggacctggg      3600
ctggtcgatc aatggccggt attacacgaa ggccgaggaa tgcctgtcgc gcctacaggc      3660
gacggcgatg ggcttcacgt ccgaccgcgt tgggcacctg gaatcggtgt cgctgctgca      3720
ccgcttccgc gtcctggacc gtggcaagaa aacgtcccgt tgccaggtcc tgatcgacga      3780
ggaaatcgtc gtgctgtttg ctggcgacca ctacacgaaa ttcatatggg agaagtaccg      3840
caagctgtcg ccgacggccc gacggatgtt cgactatttc agctcgcacc gggagccgta      3900
cccgctcaag ctggaaacct tccgcctcat gtgcggatcg gattccaccc gcgtgaagaa      3960
gtggcgcgag caggtcggcg aagcctgcga agagttgcga ggcagcggcc tggtggaaca      4020
cgcctgggtc aatgatgacc tggtgcattg caaacgctag ggccttgtgg ggtcagttcc      4080
ggctgggggt tcagcagcca gcgcctgatc tggggaaccc tgtggttggc acatacaaat      4140
ggacgaacgg ataaaccttt tcacgccctt ttaaatatcc gattattcta ataaacgctc      4200
ttttctctta ggtttacccg ccaatatatc ctgtcaaaca ctgatagttt aaactgaagg      4260
cgggaaacga caatctgatc taagctaggc atgcctgcag gtcaacatgg tggagcacga      4320
cacgcttgtc tactccaaaa atatcaaaga tacagtctca gaagaccaaa gggcaattga      4380
gacttttcaa caaagggtaa tatccggaaa cctcctcgga ttccattgcc cagctatctg      4440
tcactttatt gtgaagatag tggaaaagga aggtggctcc tacaaatgcc atcattgcga      4500
taaaggaaag gccatcgttg aagatgcctc tgccgacagt ggtcccaaag atggaccccc      4560
acccacgagg agcatcgtgg aaaaagaaga cgttccaacc acgtcttcaa agcaagtgga      4620
ttgatgtgat atctccactg acgtaaggga tgacgcacaa tcccactatc cttcgcaaga      4680
cccttcctct atataaggaa gttcatttca tttggagagg agaaaactaa accatacacc      4740
accaacacaa ccaaacccac cacgcccaat tgttacacac ccgcttgaaa aagaaagttt      4800
aacaaatggc caaggtgcgc gaggtttacc aatcttttac agactccacc acaaaaactc      4860
tcatccaaga tgaggcttat agaaacattc gccccatcat ggaaaaacac aaactagcta      4920
acccttacgc tcaaacggtt gaagcggcta atgatctaga ggggttcggc atagccacca      4980
```

atccctatag cattgaattg catacacatg cagccgctaa gaccatagag aataaacttc 5040
tagaggtgct tggttccatc ctaccacaag aacctgttac atttatgttt cttaaaccca 5100
gaaagctaaa ctacatgaga agaaacccgc ggatcaagga cattttccaa aatgttgcca 5160
ttgaaccaag agacgtagcc aggtacccca aggaaacaat aattgacaaa ctcacagaga 5220
tcacaacgga aacagcatac attagtgaca ctctgcactt cttggatccg agctacatag 5280
tggagacatt ccaaaactgc ccaaaattgc aaacattgta tgcgacctta gttctccccg 5340
ttgaggcagc ctttaaaatg gaaagcactc acccgaacat atacagcctc aaatacttcg 5400
gagatggttt ccagtatata ccaggcaacc atggtggcgg ggcataccat catgaattcg 5460
ctcatctaca atggctcaaa gtgggaaaga tcaagtggag ggaccccaag gatagctttc 5520
tcggacatct caattacacg actgagcagg ttgagatgca cacagtgaca gtacagttgc 5580
aggaatcgtt cgcggcaaac cacttgtact gcatcaggag aggagacttg ctcacaccgg 5640
aggtgcgcac tttcggccaa cctgacaggt acgtgattcc accacagatc ttcctcccaa 5700
aagttcacaa ctgcaagaag ccgattctca agaaaactat gatgcagctc ttcttgtatg 5760
ttaggacagt caaggtcgca aaaaattgtg acatttttgc caaagtcaga caattaatta 5820
aatcatctga cttggacaaa tactctgctg tggaactggt ttacttagta agctacatgg 5880
agttccttgc cgatttacaa gctaccacct gcttctcaga cacactttct ggtggcttgc 5940
taacaaagac ccttgcaccg gtgagggctt ggatacaaga gaaaaagatg cagctgtttg 6000
gtcttgagga ctacgcgaag ttagtcaaag cagttgattt ccacccggtg gatttttctt 6060
tcaaagtgga aacttgggac ttcagattcc accccttgca agcgtggaaa gccttccgac 6120
caagggaagt gtcggatgta gaggaaatgg aaagtttgtt ctcagatggg gacctgcttg 6180
attgcttcac aagaatgcca gcttatgcgg taaacgcaga ggaagattta gctgcaatca 6240
ggaaaacgcc cgagatggat gtcggtcaag aagttaaaga gcctgcagga gacagaaatc 6300
aatactcaaa ccctgcagaa actttcctca acaagctcca caggaaacac agtagggagg 6360
tgaaacacca ggccgcaaag aaagctaaac gcctagctga aatccaggag tcaatgagag 6420
ctgaaggtga tgccgaacca aatgaaataa gcgggacgat gggggcaata cccagcaacg 6480
ccgaacttcc tggcacgaat gatgccagac aagaactcac actcccaacc actaaacctg 6540
tccctgcaag gtgggaagat gcttcattca cagattctag tgtggaagag gagcaggtta 6600
aactccttgg aaaagaaacc gttgaaacag cgacgcaaca agtcatcgaa ggacttcctt 6660
ggaaacactg gattcctcaa ttaaatgctg ttggattcaa ggcgctggaa attcagaggg 6720
ataggagtgg aacaatgatc atgcccatca cagaaatggt gtccgggctg gaaaaagagg 6780
acttccctga aggaactcca aaagagttgg cacgagaatt gttcgctatg aacagaagcc 6840
ctgccaccat ccctttggac ctgcttagag ccagagacta cggcagtgat gtaaagaaca 6900

agagaattgg tgccatcaca aagacacagg caacgagttg gggcgaatac ttgacaggaa 6960
agatagaaag cttaactgag aggaaagttg cgacttgtgt cattcatgga gctggaggtt 7020
ctggaaaaag tcatgccatc cagaaggcat tgagagaaat tggcaagggc tcggacatca 7080
ctgtagtcct gccgaccaat gaactgcggc tagattggag taagaaagtg cctaacactg 7140
agccctatat gttcaagacc tctgaaaagg cgttaattgg gggaacaggc agcatagtca 7200
tctttgacga ttactcaaaa cttcctcccg gttacataga agccttagtc tgtttctact 7260
ctaaaatcaa gctaatcatt ctaacaggag atagcagaca aagcgtctac catgaaactg 7320
ctgaggacgc ctccatcagg catttgggac cagcaacaga gtacttctca aaatactgcc 7380
gatactatct caatgccaca caccgcaaca agaaagatct tgcgaacatg cttggtgtct 7440
acagtgagag aacgggagtc accgaaatca gcatgagcgc cgagttctta gaaggaatcc 7500
caactttggt accctcggat gagaagagaa agctgtacat gggcaccggg aggaatgaca 7560
cgttcacata cgctggatgc caggggctaa ctaagccgaa ggtacaaata gtgttggacc 7620
acaacaccca agtgtgtagc gcgaatgtga tgtacacggc actttctaga gccaccgata 7680
ggattcactt cgtgaacaca agtgcaaatt cctctgcctt ctgggaaaag ttggacagca 7740
ccccttacct caagactttc ctatcagtgg tgagagaaca agcactcagg gagtacgagc 7800
cggcagaggc agagccaatt caagagcctg agccccagac acacatgtgt gtcgagaatg 7860
aggagtccgt gctagaagag tacaaagagg aactcttgga aaagtttgac agagagatcc 7920
actctgaatc ccatggtcat tcaaactgtg tccaaactga agacacaacc attcagttgt 7980
tttcgcatca acaagcaaaa gatgagactc tcctctgggc gactatagat gcgcggctca 8040
agaccagcaa tcaagaaaca aacttccgag aattcctgag caagaaggac attggggacg 8100
ttctgttttt aaactaccaa aaagctatgg gtttacccaa agagcgtatt cctttttccc 8160
aagaggtctg ggaagcttgt gcccacgaag tacaaagcaa gtacctcagc aagtcaaagt 8220
gcaacttgat caatgggact gtgagacaga gcccagactt cgatgaaaat aagattatgg 8280
tattcctcaa gtcgcagtgg gtcacaaagg tggaaaaact aggtctaccc aagattaagc 8340
caggtcaaac catagcagcc ttttaccagc agactgtgat gctttttgga actatggcta 8400
ggtacatgcg atggttcaga caggctttcc agccaaaaga agtcttcata aactgtgaga 8460
cgacgccaga tgacatgtct gcatgggcct tgaacaactg gaatttcagc agacctagct 8520
tggctaatga ctacacagct ttcgaccagt ctcaggatgg agccatgttg caatttgagg 8580
tgctcaaagc caaacaccac tgcataccag aggaaatcat tcaggcatac atagatatta 8640
agactaatgc acagattttc ctaggcacgt tatcaattat gcgcctgact ggtgaaggtc 8700
ccacttttga tgcaaacact gagtgcaaca tagcttacac ccatacaaag tttgacatcc 8760

cagccggaac tgctcaagtt tatgcaggag acgactccgc actggactgt gttccagaag 8820
tgaagcatag tttccacagg cttgaggaca aattactcct aaagtcaaag cctgtaatca 8880
cgcagcaaaa gaagggcagt tggcctgagt tttgtggttg gctgatcaca ccaaaagggg 8940
tgatgaaaga cccaattaag ctccatgtta gcttaaaatt ggctgaagct aagggtgaac 9000
tcaagaaatg tcaagattcc tatgaaattg atctgagtta tgcctatgac cacaaggact 9060
ctctgcatga cttgttcgat gagaaacagt gtcaggcaca cacactcact tgcagaacac 9120
taatcaagtc agggagaggc actgtctcac tttcccgcct cagaaacttt ctttaaccgt 9180
taagttacct tagagatttg aataagatgt cagcaccagc tagtacaaca cagcccatag 9240
ggtcaactac ctcaactacc acaaaaactg caggcgcaac tcctgccaca gcttcaggcc 9300
tgttcactat cccggatggg gatttcttta gtacagcccg tgccatagta gccagcaatg 9360
ctgtcgcaac aaatgaggac ctcagcaaga ttgaggctat ttggaaggac atgaaggtgc 9420
ccacagacac tatggcacag gctgcttggg acttagtcag acactgtgct gatgtaggat 9480
catccgctca aacagaaatg atagatacag gtccctattc caacggcatc agcagagcta 9540
gactggcagc agcaattaaa gaggtgtgca cacttaggca attttgcatg aagtatgccc 9600
cagtggtatg gaactggatg ttaactaaca acagtccacc tgctaactgg caagcacaag 9660
gtttcaagcc tgagcacaaa ttcgctgcat tcgacttctt caatggagtc accaacccag 9720
ctgccatcat gcccaaagag gggctcatcc ggccaccgtc tgaagctgaa atgaatgctg 9780
cccaaactgc tgcctttgtg aagattacaa aggccagggc acaatccaac gactttgcca 9840
gcctagatgc agctgtcact cgaggaagga tcaccggaac gaccacagca gaggcagtcg 9900
ttactctgcc tcctccataa cagaaacttt ctttaaccgt taagttacct tagagatttg 9960
aataagatgg atattctcat cagtagtttg aaaagtttag gttattctag gacttccaaa 10020
tctttagatt caggaccttt ggtagtacat gcagtagccg gagccggtaa gtccacagcc 10080
ctaaggaagt tgatcctcag acacccaaca ttcaccgtgc atacactcgg tgtccctgac 10140
aaggtgagta tcagaactag aggcatacag aagccaggac ctattcctga gggcaacttc 10200
gcaatcctcg atgagtatac tttggacaac accacaagga actcatacca ggcacttttt 10260
gctgaccctt atcaggcacc ggagtttagc ctagagcccc acttctactt ggaaacatca 10320
tttcgagttc cgaggaaagt ggcagatttg atagctggct gtggcttcga tttcgagacg 10380
aactcaccgg aagaagggca cttagagatc actggcatat tcaaagggcc cctactcgga 10440
aaggtgatag ccattgatga ggagtctgag acaacactgt ccaggcatgg tgttgagttt 10500
gttaagccct gccaagtgac gggacttgag ttcaaagtag tcactattgt gtctgccgca 10560
ccaatagagg aaattggcca gtccacagct ttctacaacg ctatcaccag gtcaaaggga 10620
ttgacatatg tccgcgcagg gccataggct gaccgctccg gtcaattctg aaaaagtgta 10680

catagtatta ggtctatcat ttgctttagt ttcaattacc tttctgcttt ctagaaatag 10740

cttaccccac gtcggtgaca acattcacag cttgccacac ggaggagctt acagagacgg 10800

caccaaagca atcttgtaca actccccaaa tctagggtca cgagtgagtc tacacaacgg 10860

aaagaacgca gcatttgctg ccgttttgct actgactttg ctgatctatg gaagtaaata 10920

catatctcaa cgcaatcata cttgtgcttg tggtaacaat catagcagtc attagcactt 10980

ccttagtgag gactgaacct tgtgtcatca agattactgg ggaatcaatc acagtgttgg 11040

cttgcaaact agatgcagaa accataaggg ccattgccga tctcaagcca ctctccgttg 11100

aacggttaag tttccattga tactcgaaag aggtcagcac cagctagcaa caaacaagaa 11160

catgagagac ctcgcgattt aaatcgatgg tctcagatcg gtcgtatcac tggaacaaca 11220

accgctgagg ctgttgtcac tctaccacca ccataactac gtctacataa ccgacgccta 11280

ccccagtttc atagtatttt ctggtttgat tgtatgaata atataaataa aaaaaaaaaa 11340

aaaaaaaaaa aactagtgag ct 11362

<210> 24
<211> 12380
<212> DNA
<213> Artificial Sequence

<220>
<223> pNMD4300

<400> 24
aaactgaagg cgggaaacga caatctgatc taagctaggc atgcctgcag gtcaacatgg 60

tggagcacga cacgcttgtc tactccaaaa atatcaaaga tacagtctca gaagaccaaa 120

gggcaattga gacttttcaa caaagggtaa tatccggaaa cctcctcgga ttccattgcc 180

cagctatctg tcactttatt gtgaagatag tggaaaagga aggtggctcc tacaaatgcc 240

atcattgcga taaaggaaag gccatcgttg aagatgcctc tgccgacagt ggtcccaaag 300

atggaccccc acccacgagg agcatcgtgg aaaaagaaga cgttccaacc acgtcttcaa 360

agcaagtgga ttgatgtgat atctccactg acgtaaggga tgacgcacaa tcccactatc 420

cttcgcaaga cccttcctct atataaggaa gttcatttca tttggagagg agaaaactaa 480

accatacacc accaacacaa ccaaacccac cacgcccaat tgttacacac ccgcttgaaa 540

aagaaagttt aacaaatggc caaggtgcgc gaggtttacc aatcttttac agactccacc 600

acaaaaactc tcatccaaga tgaggcttat agaaacattc gccccatcat ggaaaaacac 660

aaactagcta acccttacgc tcaaacggtt gaagcggcta atgatctaga ggggttcggc 720

atagccacca atccctatag cattgaattg catacacatg cagccgctaa gaccatagag 780

aataaacttc tagaggtgct tggttccatc ctaccacaag aacctgttac atttatgttt 840

```
cttaaaccca gaaagctaaa ctacatgaga agaaacccgc ggatcaagga cattttccaa      900
aatgttgcca ttgaaccaag agacgtagcc aggtacccca aggaaacaat aattgacaaa      960
ctcacagaga tcacaacgga aacagcatac attagtgaca ctctgcactt cttggatccg     1020
agctacatag tggagacatt ccaaaactgc ccaaaattgc aaacattgta tgcgacctta     1080
gttctccccg ttgaggcagc ctttaaaatg gaaagcactc acccgaacat atacagcctc     1140
aaatacttcg gagatggttt ccagtatata ccaggcaacc atggtggcgg ggcataccat     1200
catgaattcg ctcatctaca atggctcaaa gtgggaaaga tcaagtggag ggaccccaag     1260
gatagctttc tcggacatct caattacacg actgagcagg ttgagatgca cacagtgaca     1320
gtacagttgc aggaatcgtt cgcggcaaac cacttgtact gcatcaggag aggagacttg     1380
ctcacaccgg aggtgcgcac tttcggccaa cctgacaggt acgtgattcc accacagatc     1440
ttcctcccaa aagttcacaa ctgcaagaag ccgattctca agaaaactat gatgcagctc     1500
ttcttgtatg ttaggacagt caaggtcgca aaaaattgtg acatttttgc caaagtcaga     1560
caattaatta aatcatctga cttggacaaa tactctgctg tggaactggt ttacttagta     1620
agctacatgg agttccttgc cgatttacaa gctaccacct gcttctcaga cacactttct     1680
ggtggcttgc taacaaagac ccttgcaccg gtgagggctt ggatacaaga gaaaaagatg     1740
cagctgtttg gtcttgagga ctacgcgaag ttagtcaaag cagttgattt ccaccccggtg    1800
gatttttctt tcaaagtgga aacttgggac ttcagattcc accccttgca agcgtggaaa     1860
gccttccgac caagggaagt gtcggatgta gaggaaatgg aaagtttgtt ctcagatggg     1920
gacctgcttg attgcttcac aagaatgcca gcttatgcgg taaacgcaga ggaagattta     1980
gctgcaatca ggaaaacgcc cgagatggat gtcggtcaag aagttaaaga gcctgcagga     2040
gacagaaatc aatactcaaa ccctgcagaa actttcctca acaagctcca caggaaacac     2100
agtagggagg tgaaacacca ggccgcaaag aaagctaaac gcctagctga aatccaggag     2160
tcaatgagag ctgaaggtga tgccgaacca aatgaaataa gcgggacgat gggggcaata     2220
cccagcaacg ccgaacttcc tggcacgaat gatgccagac aagaactcac actcccaacc     2280
actaaacctg tccctgcaag gtgggaagat gcttcattca cagattctag tgtggaagag     2340
gagcaggtta aactccttgg aaaagaaacc gttgaaacag cgacgcaaca agtcatcgaa     2400
ggacttcctt ggaaacactg gattcctcaa ttaaatgctg ttggattcaa ggcgctggaa     2460
attcagaggg ataggagtgg aacaatgatc atgcccatca cagaaatggt gtccgggctg     2520
gaaaaagagg acttccctga aggaactcca aaagagttgg cacgagaatt gttcgctatg     2580
aacagaagcc ctgccaccat cccctttggac ctgcttagag ccagagacta cggcagtgat    2640
gtaaagaaca agagaattgg tgccatcaca aagacacagg caacgagttg gggcgaatac     2700
ttgacaggaa agatagaaag cttaactgag aggaaagttg cgacttgtgt cattcatgga     2760
```

gctggaggtt ctggaaaaag tcatgccatc cagaaggcat tgagagaaat tggcaagggc 2820
tcggacatca ctgtagtcct gccgaccaat gaactgcggc tagattggag taagaaagtg 2880
cctaacactg agccctatat gttcaagacc tctgaaaagg cgttaattgg gggaacaggc 2940
agcatagtca tctttgacga ttactcaaaa cttcctcccg gttacataga agccttagtc 3000
tgtttctact ctaaaatcaa gctaatcatt ctaacaggag atagcagaca aagcgtctac 3060
catgaaactg ctgaggacgc ctccatcagg catttgggac cagcaacaga gtacttctca 3120
aaatactgcc gatactatct caatgccaca caccgcaaca agaaagatct tgcgaacatg 3180
cttggtgtct acagtgagag aacgggagtc accgaaatca gcatgagcgc cgagttctta 3240
gaaggaatcc caactttggt accctcggat gagaagagaa agctgtacat gggcaccggg 3300
aggaatgaca cgttcacata cgctggatgc caggggctaa ctaagccgaa ggtacaaata 3360
gtgttggacc acaacaccca agtgtgtagc gcgaatgtga tgtacacggc actttctaga 3420
gccaccgata ggattcactt cgtgaacaca agtgcaaatt cctctgcctt ctgggaaaag 3480
ttggacagca ccccttacct caagactttc ctatcagtgg tgagagaaca agcactcagg 3540
gagtacgagc cggcagaggc agagccaatt caagagcctg agccccagac acacatgtgt 3600
gtcgagaatg aggagtccgt gctagaagag tacaaagagg aactcttgga aaagtttgac 3660
agagagatcc actctgaatc ccatggtcat tcaaactgtg tccaaactga agacacaacc 3720
attcagttgt tttcgcatca acaagcaaaa gatgagactc tcctctgggc gactatagat 3780
gcgcggctca agaccagcaa tcaagaaaca aacttccgag aattcctgag caagaaggac 3840
attggggacg ttctgttttt aaactaccaa aaagctatgg gtttacccaa agagcgtatt 3900
cctttttccc aagaggtctg ggaagcttgt gcccacgaag tacaaagcaa gtacctcagc 3960
aagtcaaagt gcaacttgat caatgggact gtgagacaga gcccagactt cgatgaaaat 4020
aagattatgg tattcctcaa gtcgcagtgg gtcacaaagg tggaaaaact aggtctaccc 4080
aagattaagc caggtcaaac catagcagcc ttttaccagc agactgtgat gcttttttgga 4140
actatggcta ggtacatgcg atggttcaga caggctttcc agccaaaaga agtcttcata 4200
aactgtgaga cgacgccaga tgacatgtct gcatgggcct tgaacaactg gaatttcagc 4260
agacctagct tggctaatga ctacacagct ttcgaccagt ctcaggatgg agccatgttg 4320
caatttgagg tgctcaaagc caaacaccac tgcataccag aggaaatcat tcaggcatac 4380
atagatatta agactaatgc acagattttc ctaggcacgt tatcaattat gcgcctgact 4440
ggtgaaggtc ccacttttga tgcaaacact gagtgcaaca tagcttacac ccatacaaag 4500
tttgacatcc cagccggaac tgctcaagtt tatgcaggag acgactccgc actggactgt 4560
gttccagaag tgaagcatag tttccacagg cttgaggaca aattactcct aaagtcaaag 4620

cctgtaatca cgcagcaaaa gaagggcagt tggcctgagt tttgtggttg gctgatcaca 4680
ccaaaagggg tgatgaaaga cccaattaag ctccatgtta gcttaaaatt ggctgaagct 4740
aagggtgaac tcaagaaatg tcaagattcc tatgaaattg atctgagtta tgcctatgac 4800
cacaaggact ctctgcatga cttgttcgat gagaaacagt gtcaggcaca cacactcact 4860
tgcagaacac taatcaagtc agggagaggc actgtctcac tttcccgcct cagaaacttt 4920
ctttaaccgt taagttacct tagagatttg aataagatgt cagcaccagc tagtacaaca 4980
cagcccatag ggtcaactac ctcaactacc acaaaaactg caggcgcaac tcctgccaca 5040
gcttcaggcc tgttcactat cccggatggg gatttcttta gtacagcccg tgccatagta 5100
gccagcaatg ctgtcgcaac aaatgaggac ctcagcaaga ttgaggctat ttggaaggac 5160
atgaaggtgc ccacagacac tatggcacag gctgcttggg acttagtcag acactgtgct 5220
gatgtaggat catccgctca aacagaaatg atagatacag gtccctattc caacggcatc 5280
agcagagcta gactggcagc agcaattaaa gaggtgtgca cacttaggca attttgcatg 5340
aagtatgccc cagtggtatg gaactggatg ttaactaaca acagtccacc tgctaactgg 5400
caagcacaag gtttcaagcc tgagcacaaa ttcgctgcat tcgacttctt caatggagtc 5460
accaacccag ctgccatcat gcccaaagag gggctcatcc ggccaccgtc tgaagctgaa 5520
atgaatgctg cccaaactgc tgcctttgtg aagattacaa aggccagggc acaatccaac 5580
gactttgcca gcctagatgc agctgtcact cgaggaagga tcaccggaac gaccacagca 5640
gaggcagtcg ttactctgcc tcctccataa cagaaacttt ctttaaccgt taagttacct 5700
tagagatttg aataagatgg atattctcat cagtagtttg aaaagtttag gttattctag 5760
gacttccaaa tctttagatt caggaccttt ggtagtacat gcagtagccg gagccggtaa 5820
gtccacagcc ctaaggaagt tgatcctcag acacccaaca ttcaccgtgc atacactcgg 5880
tgtccctgac aaggtgagta tcagaactag aggcatacag aagccaggac ctattcctga 5940
gggcaacttc gcaatcctcg atgagtatac tttggacaac accacaagga actcatacca 6000
ggcacttttt gctgaccctt atcaggcacc ggagtttagc ctagagcccc acttctactt 6060
ggaaacatca tttcgagttc cgaggaaagt ggcagatttg atagctggct gtggcttcga 6120
tttcgagacg aactcaccgg aagaagggca cttagagatc actggcatat tcaaagggcc 6180
cctactcgga aaggtgatag ccattgatga ggagtctgag acaacactgt ccaggcatgg 6240
tgttgagttt gttaagccct gccaagtgac gggacttgag ttcaaagtag tcactattgt 6300
gtctgccgca ccaatagagg aaattggcca gtccacagct ttctacaacg ctatcaccag 6360
gtcaaaggga ttgacatatg tccgcgcagg gccataggct gaccgctccg gtcaattctg 6420
aaaaagtgta catagtatta ggtctatcat ttgctttagt ttcaattacc tttctgcttt 6480
ctagaaatag cttaccccac gtcggtgaca acattcacag cttgccacac ggaggagctt 6540

acagagacgg caccaaagca atcttgtaca actccccaaa tctagggtca cgagtgagtc 6600
tacacaacgg aaagaacgca gcatttgctg ccgttttgct actgactttg ctgatctatg 6660
gaagtaaata catatctcaa cgcaatcata cttgtgcttg tggtaacaat catagcagtc 6720
attagcactt ccttagtgag gactgaacct tgtgtcatca agattactgg ggaatcaatc 6780
acagtgttgg cttgcaaact agatgcagaa accataaggg ccattgccga tctcaagcca 6840
ctctccgttg aacggttaag tttccattga tactcgaaag aggtcagcac cagctagcaa 6900
caaacaagaa catgagagac ctcgcgattt aaatcgatgg tctcagatcg gtcgtatcac 6960
tggaacaaca accgctgagg ctgttgtcac tctaccacca ccataactac gtctacataa 7020
ccgacgccta ccccagtttc atagtatttt ctggtttgat tgtatgaata atataaataa 7080
aaaaaaaaaa aaaaaaaaaa aactagtgag ctcttctgtc agcgggccca ctgcatccac 7140
cccagtacat taaaaacgtc cgcaatgtgt tattaagttg tctaagcgtc aatttgttta 7200
caccacaata tatcctgcca ccagccagcc aacagctccc cgaccggcag ctcggcacaa 7260
aatcaccact cgatacaggc agcccatcag tcagatcagg atctcctttg cgacgctcac 7320
cgggctggtt gccctcgccg ctgggctggc ggccgtctat ggccctgcaa acgcgccaga 7380
aacgccgtcg aagccgtgtg cgagacaccg cggccgccgg cgttgtggat acctcgcgga 7440
aaacttggcc ctcactgaca gatgaggggc ggacgttgac acttgagggg ccgactcacc 7500
cggcgcggcg ttgacagatg aggggcaggc tcgatttcgg ccggcgacgt ggagctggcc 7560
agcctcgcaa atcggcgaaa acgcctgatt ttacgcgagt ttcccacaga tgatgtggac 7620
aagcctgggg ataagtgccc tgcggtattg acacttgagg ggcgcgacta ctgacagatg 7680
aggggcgcga tccttgacac ttgaggggca gagtgctgac agatgagggg cgcacctatt 7740
gacatttgag gggctgtcca caggcagaaa atccagcatt tgcaagggtt tccgcccgtt 7800
tttcggccac cgctaacctg tcttttaacc tgcttttaaa ccaatattta taaaccttgt 7860
ttttaaccag ggctgcgccc tgtgcgcgtg accgcgcacg ccgaaggggg gtgccccccc 7920
ttctcgaacc ctcccggccc gctaacgcgg gcctcccatc cccccagggg ctgcgcccct 7980
cggccgcgaa cggcctcacc ccaaaaatgg cagcctgtcg atcagatctg gctcgcggcg 8040
gacgcacgac gccggggcga gaccataggc gatctcctaa atcaatagta gctgtaacct 8100
cgaagcgttt cacttgtaac aacgattgag aatttttgtc ataaaattga aatacttggt 8160
tcgcattttt gtcatccgcg gtcagccgca attctgacga actgcccatt tagctggaga 8220
tgattgtaca tccttcacgt gaaaatttct caagtgctgt gaacaagggt tcagatttta 8280
gattgaaagg tgagccgttg aaacacgttc ttcttgtcga tgacgacgtc gctatgcggc 8340
atcttattat tgaatacctt acgatccacg ccttcaaagt gaccgcggta gccgacagca 8400

cccagttcac aagagtactc tcttccgcga cggtcgatgt cgtggttgtt gatctagatt 8460
taggtcgtga agatgggctc gagatcgttc gtaatctggc ggcaaagtct gatattccaa 8520
tcataattat cagtggcgac cgccttgagg agacggataa agttgttgca ctcgagctag 8580
gagcaagtga ttttatcgct aagccgttca gtatcagaga gtttctagca cgcattcggg 8640
ttgccttgcg cgtgcgcccc aacgttgtcc gctccaaaga ccgacggtct tttgtttta 8700
ctgactggac acttaatctc aggcaacgtc gcttgatgtc cgaagctggc ggtgaggtga 8760
aacttacggc aggtgagttc aatcttctcc tcgcgttttt agagaaaccc cgcgacgttc 8820
tatcgcgcga gcaacttctc attgccagtc gagtacgcga cgaggaggtt tatgacagga 8880
gtatagatgt tctcattttg aggctgcgcc gcaaacttga ggcggatccg tcaagccctc 8940
aactgataaa aacagcaaga ggtgccggtt atttctttga cgcggacgtg caggtttcgc 9000
acggggggac gatggcagcc taagatcgac aggctggcca attcgtgcgc ggaaccccta 9060
tttgtttatt tttctaaata cattcaaata tgtatccgct catgagacaa taaccctgat 9120
aaatgcttca ataatattga aaaaggaaga gtatggctaa aatgagaata tcaccggaat 9180
tgaaaaaact gatcgaaaaa taccgctgcg taaaagatac ggaaggaatg tctcctgcta 9240
aggtatataa gctggtggga gaaaatgaaa acctatattt aaaaatgacg gacagccggt 9300
ataaagggac cacctatgat gtggaacggg aaaaggacat gatgctatgg ctggaaggaa 9360
agctgcctgt tccaaaggtc ctgcactttg aacggcatga tggctggagc aatctgctca 9420
tgagtgaggc cgatggcgtc ctttgctcgg aagagtatga agatgaacaa agccctgaaa 9480
agattatcga gctgtatgcg gagtgcatca ggctctttca ctccatcgac atatcggatt 9540
gtccctatac gaatagctta gacagccgct tagccgaatt ggattactta ctgaataacg 9600
atctggccga tgtggattgc gaaaactggg aagaagacac tccatttaaa gatccgcgcg 9660
agctgtatga tttttaaag acggaaaagc ccgaagagga acttgtcttt tcccacggcg 9720
acctgggaga cagcaacatc tttgtgaaag atggcaaagt aagtggcttt attgatcttg 9780
ggagaagcgg cagggcggac aagtggtatg acattgcctt ctgcgtccgg tcgatcaggg 9840
aggatatcgg ggaagaacag tatgtcgagc tatttttga cttactgggg atcaagcctg 9900
attgggagaa aataaaatat tatatttac tggatgaatt gttttagctg tcagaccaag 9960
tttactcata tatactttag attgatttaa aacttcattt ttaatttaaa aggatctagg 10020
tgaagatcct ttttgataat ctcatgacca aaatccctta acgtgagttt tcgttccact 10080
gagcgtcaga ccccgtagaa aagatcaaag gatcttcttg agatcctttt tttctgcgcg 10140
taatctgctg cttgcaaaca aaaaaaccac cgctaccagc ggtggtttgt ttgccggatc 10200
aagagctacc aactcttttt ccgaaggtaa ctggcttcag cagagcgcag ataccaaata 10260
ctgtccttct agtgtagccg tagttaggcc accacttcaa gaactctgta gcaccgccta 10320

catacctcgc tctgctaatc ctgttaccag tggctgctgc cagtggcgat aagtcgtgtc 10380
ttaccgggtt ggactcaaga cgatagttac cggataaggc gcagcggtcg ggctgaacgg 10440
ggggttcgtg cacacagccc agcttggagc gaacgaccta caccgaactg agatacctac 10500
agcgtgagct atgagaaagc gccacgcttc ccgaagggag aaaggcggac aggtatccgg 10560
taagcggcag ggtcggaaca ggagagcgca cgagggagct tccaggggga aacgcctggt 10620
atctttatag tcctgtcggg tttcgccacc tctgacttga gcgtcgattt ttgtgatgct 10680
cgtcaggggg gcggagccta tggaaaaacg ccagcaacgc ggccttttta cggttcctgg 10740
cagatcctag atgtggcgca acgatgccgg cgacaagcag gagcgcaccg acttcttccg 10800
catcaagtgt tttggctctc aggccgaggc ccacggcaag tatttgggca aggggtcgct 10860
ggtattcgtg cagggcaaga ttcggaatac caagtacgag aaggacggcc agacggtcta 10920
cgggaccgac ttcattgccg ataaggtgga ttatctggac accaaggcac caggcgggtc 10980
aaatcaggaa taagggcaca ttgccccggc gtgagtcggg gcaatcccgc aaggagggtg 11040
aatgaatcgg acgtttgacc ggaaggcata caggcaagaa ctgatcgacg cggggttttc 11100
cgccgaggat gccgaaacca tcgcaagccg caccgtcatg cgtgcgcccc gcgaaacctt 11160
ccagtccgtc ggctcgatgg tccagcaagc tacggccaag atcgagcgcg acagcgtgca 11220
actggctccc cctgccctgc ccgcgccatc ggccgccgtg gagcgttcgc gtcgtctcga 11280
acaggaggcg gcaggtttgg cgaagtcgat gaccatcgac acgcgaggaa ctatgacgac 11340
caagaagcga aaaaccgccg gcgaggacct ggcaaaacag gtcagcgagg ccaagcaggc 11400
cgcgttgctg aaacacacga agcagcagat caaggaaatg cagctttcct tgttcgatat 11460
tgcgccgtgg ccggacacga tgcgagcgat gccaaacgac acggcccgct ctgccctgtt 11520
caccacgcgc aacaagaaaa tcccgcgcga ggcgctgcaa aacaaggtca ttttccacgt 11580
caacaaggac gtgaagatca cctacaccgg cgtcgagctg cgggccgacg atgacgaact 11640
ggtgtggcag caggtgttgg agtacgcgaa gcgcacccct atcggcgagc cgatcacctt 11700
cacgttctac gagctttgcc aggacctggg ctggtcgatc aatggccggt attacacgaa 11760
ggccgaggaa tgcctgtcgc gcctacaggc gacggcgatg ggcttcacgt ccgaccgcgt 11820
tgggcacctg gaatcggtgt cgctgctgca ccgcttccgc gtcctggacc gtggcaagaa 11880
aacgtcccgt tgccaggtcc tgatcgacga ggaaatcgtc gtgctgtttg ctggcgacca 11940
ctacacgaaa ttcatatggg agaagtaccg caagctgtcg ccgacggccc gacggatgtt 12000
cgactatttc agctcgcacc gggagccgta cccgctcaag ctggaaacct tccgcctcat 12060
gtgcggatcg gattccaccc gcgtgaagaa gtggcgcgag caggtcggcg aagcctgcga 12120
agagttgcga ggcagcggcc tggtggaaca cgcctgggtc aatgatgacc tggtgcattg 12180

caaacgctag ggccttgtgg ggtcagttcc ggctgggggt tcagcagcca gcgcctgatc 12240

tggggaaccc tgtggttggc acatacaaat ggacgaacgg ataaaccttt tcacgccctt 12300

ttaaatatcc gattattcta ataaacgctc ttttctctta ggtttacccg ccaatatatc 12360

ctgtcaaaca ctgatagttt 12380

<210> 25
<211> 33
<212> DNA
<213> Artificial Sequence

<220>
<223> oligonucleotide

<400> 25
tttgaagaca tctcaacgca atcatacttg tgc 33

<210> 26
<211> 38
<212> DNA
<213> Artificial Sequence

<220>
<223> oligonucleotide

<400> 26
tttgaagact tctcggttat gtagacgtag ttatggtg 38

<210> 27
<211> 11
<212> DNA
<213> Artificial Sequence

<220>
<223> BsaI cleavage site

<220>
<221> misc_feature
<222> (7)..(11)
<223> n is a, c, g, or t

<400> 27
ggtctcnnnn n 11

<210> 28
<211> 13
<212> DNA
<213> Artificial Sequence

<220>
<223> donor/acceptor splicing site

<400> 28
aggtraggca ggt 13

<210> 29
<211> 681
<212> DNA
<213> Potato virus X

<400> 29
atggatattc tcatcagtag tttgaaaagt ttaggttatt ctaggacttc caaatcttta 60

gattcaggac ctttggtagt acatgcagta gccggagccg gtaagtccac agccctaagg 120

aagttgatcc tcagacaccc aacattcacc gtgcatacac tcggtgtccc tgacaaggtg 180

agtatcagaa ctagaggcat acagaagcca ggacctattc ctgagggcaa cttcgcaatc 240

ctcgatgagt atactttgga caacaccaca aggaactcat accaggcact ttttgctgac 300

ccttatcagg caccggagtt tagcctagag ccccacttct acttggaaac atcatttcga 360

gttccgagga aagtggcaga tttgatagct ggctgtggct tcgatttcga gacgaactca 420

ccggaagaag ggcacttaga gatcactggc atattcaaag ggcccctact cggaaaggtg 480

atagccattg atgaggagtc tgagacaaca ctgtccaggc atggtgttga gtttgttaag 540

ccctgccaag tgacgggact tgagttcaaa gtagtcacta ttgtgtctgc cgcaccaata 600

gaggaaattg gccagtccac agctttctac aacgctatca ccaggtcaaa gggattgaca 660

tatgtccgcg cagggccata g 681

<210> 30
<211> 226
<212> PRT
<213> Potato virus X

<400> 30

Met Asp Ile Leu Ile Ser Ser Leu Lys Ser Leu Gly Tyr Ser Arg Thr
1 5 10 15

Ser Lys Ser Leu Asp Ser Gly Pro Leu Val Val His Ala Val Ala Gly
20 25 30

Ala Gly Lys Ser Thr Ala Leu Arg Lys Leu Ile Leu Arg His Pro Thr
35 40 45

Phe Thr Val His Thr Leu Gly Val Pro Asp Lys Val Ser Ile Arg Thr
50 55 60

Arg Gly Ile Gln Lys Pro Gly Pro Ile Pro Glu Gly Asn Phe Ala Ile
65 70 75 80

Leu Asp Glu Tyr Thr Leu Asp Asn Thr Thr Arg Asn Ser Tyr Gln Ala
85 90 95

Leu Phe Ala Asp Pro Tyr Gln Ala Pro Glu Phe Ser Leu Glu Pro His

100 105 110

Phe Tyr Leu Glu Thr Ser Phe Arg Val Pro Arg Lys Val Ala Asp Leu
115 120 125

Ile Ala Gly Cys Gly Phe Asp Phe Glu Thr Asn Ser Pro Glu Glu Gly
130 135 140

His Leu Glu Ile Thr Gly Ile Phe Lys Gly Pro Leu Leu Gly Lys Val
145 150 155 160

Ile Ala Ile Asp Glu Glu Ser Glu Thr Thr Leu Ser Arg His Gly Val
165 170 175

Glu Phe Val Lys Pro Cys Gln Val Thr Gly Leu Glu Phe Lys Val Val
180 185 190

Thr Ile Val Ser Ala Ala Pro Ile Glu Glu Ile Gly Gln Ser Thr Ala
195 200 205

Phe Tyr Asn Ala Ile Thr Arg Ser Lys Gly Leu Thr Tyr Val Arg Ala
210 215 220

Gly Pro
225

<210> 31
<211> 345
<212> DNA
<213> Potato virus X

<400> 31
atgtccgcgc agggccatag gctgaccgct ccggtcaatt ctgaaaaagt gtacatagta 60

ttaggtctat catttgcttt agtttcaatt acctttctgc tttctagaaa tagcttaccc 120

cacgtcggtg acaacattca cagcttgcca cacggaggag cttacagaga cggcaccaaa 180

gcaatcttgt acaactcccc aaatctaggg tcacgagtga gtctacacaa cggaaagaac 240

gcagcatttg ctgccgtttt gctactgact ttgctgatct atggaagtaa atacatatct 300

caacgcaatc atacttgtgc ttgtggtaac aatcatagca gtcat 345

<210> 32
<211> 115
<212> PRT
<213> Potato virus X

<400> 32

Met Ser Ala Gln Gly His Arg Leu Thr Ala Pro Val Asn Ser Glu Lys
1 5 10 15

```
Val Tyr Ile Val Leu Gly Leu Ser Phe Ala Leu Val Ser Ile Thr Phe
            20                  25                  30

Leu Leu Ser Arg Asn Ser Leu Pro His Val Gly Asp Asn Ile His Ser
        35                  40                  45

Leu Pro His Gly Gly Ala Tyr Arg Asp Gly Thr Lys Ala Ile Leu Tyr
    50                  55                  60

Asn Ser Pro Asn Leu Gly Ser Arg Val Ser Leu His Asn Gly Lys Asn
65                  70                  75                  80

Ala Ala Phe Ala Ala Val Leu Leu Leu Thr Leu Leu Ile Tyr Gly Ser
                85                  90                  95

Lys Tyr Ile Ser Gln Arg Asn His Thr Cys Ala Cys Gly Asn Asn His
            100                 105                 110

Ser Ser His
        115
```

<210> 33
<211> 210
<212> DNA
<213> Potato virus X

<400> 33

```
atggaagtaa atacatatct caacgcaatc atacttgtgc ttgtggtaac aatcatagca        60

gtcattagca cttccttagt gaggactgaa ccttgtgtca tcaagattac tggggaatca       120

atcacagtgt tggcttgcaa actagatgca gaaaccataa gggccattgc cgatctcaag       180

ccactctccg ttgaacggtt aagtttccat                                        210
```

<210> 34
<211> 70
<212> PRT
<213> Potato virus X

<400> 34

```
Met Glu Val Asn Thr Tyr Leu Asn Ala Ile Ile Leu Val Leu Val Val
1               5                   10                  15

Thr Ile Ile Ala Val Ile Ser Thr Ser Leu Val Arg Thr Glu Pro Cys
            20                  25                  30

Val Ile Lys Ile Thr Gly Glu Ser Ile Thr Val Leu Ala Cys Lys Leu
        35                  40                  45
```

Asp Ala Glu Thr Ile Arg Ala Ile Ala Asp Leu Lys Pro Leu Ser Val
50 55 60

Glu Arg Leu Ser Phe His
65 70

<210> 35
<211> 714
<212> DNA
<213> Potato virus X

<400> 35
atgtcagcac cagctagcac aacacagccc atagggtcaa ctacctcaac taccacaaaa 60

actgcaggcg caactcctgc cacagcttca ggcctgttca ctatcccgga tggggatttc 120

tttagtacag cccgtgccat agtagccagc aatgctgtcg caacaaatga ggacctcagc 180

aagattgagg ctatttggaa ggacatgaag gtgcccacag acactatggc acaggctgct 240

tgggacttag tcagacactg tgctgatgta ggatcatccg ctcaaacaga aatgatagat 300

acaggtccct attccaacgg catcagcaga gctagactgg cagcagcaat taaagaggtg 360

tgcacactta ggcaattttg catgaagtat gccccagtgg tatggaactg gatgttaact 420

aacaacagtc cacctgctaa ctggcaagca caaggtttca agcctgagca caaattcgct 480

gcattcgact tcttcaatgg agtcaccaac ccagctgcca tcatgcccaa agaggggctc 540

atccggccac cgtctgaagc tgaaatgaat gctgcccaaa ctgctgcctt tgtgaagatt 600

acaaaggcca gggcacaatc caacgacttt gccagcctag atgcagctgt cactcgaggt 660

cgtatcactg gaacaacaac cgctgaggct gttgtcactc taccaccacc ataa 714

<210> 36
<211> 237
<212> PRT
<213> Potato virus X

<400> 36

Met Ser Ala Pro Ala Ser Thr Thr Gln Pro Ile Gly Ser Thr Thr Ser
1 5 10 15

Thr Thr Thr Lys Thr Ala Gly Ala Thr Pro Ala Thr Ala Ser Gly Leu
20 25 30

Phe Thr Ile Pro Asp Gly Asp Phe Phe Ser Thr Ala Arg Ala Ile Val
35 40 45

Ala Ser Asn Ala Val Ala Thr Asn Glu Asp Leu Ser Lys Ile Glu Ala
50 55 60

Ile Trp Lys Asp Met Lys Val Pro Thr Asp Thr Met Ala Gln Ala Ala
65 70 75 80

Trp Asp Leu Val Arg His Cys Ala Asp Val Gly Ser Ser Ala Gln Thr
85 90 95

Glu Met Ile Asp Thr Gly Pro Tyr Ser Asn Gly Ile Ser Arg Ala Arg
100 105 110

Leu Ala Ala Ala Ile Lys Glu Val Cys Thr Leu Arg Gln Phe Cys Met
115 120 125

Lys Tyr Ala Pro Val Val Trp Asn Trp Met Leu Thr Asn Asn Ser Pro
130 135 140

Pro Ala Asn Trp Gln Ala Gln Gly Phe Lys Pro Glu His Lys Phe Ala
145 150 155 160

Ala Phe Asp Phe Phe Asn Gly Val Thr Asn Pro Ala Ala Ile Met Pro
165 170 175

Lys Glu Gly Leu Ile Arg Pro Pro Ser Glu Ala Glu Met Asn Ala Ala
180 185 190

Gln Thr Ala Ala Phe Val Lys Ile Thr Lys Ala Arg Ala Gln Ser Asn
195 200 205

Asp Phe Ala Ser Leu Asp Ala Ala Val Thr Arg Gly Arg Ile Thr Gly
210 215 220

Thr Thr Thr Ala Glu Ala Val Val Thr Leu Pro Pro Pro
225 230 235

<210> 37
<211> 4371
<212> DNA
<213> Potato virus X

<400> 37
atggccaagg tgcgcgaggt ttaccaatct tttacagact ccaccacaaa aactctcatc 60

caagatgagg cttatagaaa cattcgcccc atcatggaaa aacacaaact agctaaccct 120

tacgctcaaa cggttgaagc ggctaatgat ctagaggggt tcggcatagc caccaatccc 180

tatagcattg aattgcatac acatgcagcc gctaagacca tagagaataa acttctagag 240

gtgcttggtt ccatcctacc acaagaacct gttacattta tgtttcttaa acccagaaag 300

ctaaactaca tgagaagaaa cccgcggatc aaggacattt tccaaaatgt tgccattgaa 360

ccaagagacg tagccaggta ccccaaggaa acaataattg acaaactcac agagatcaca 420

acggaaacag catacattag tgacactctg cacttcttgg atccgagcta catagtggag 480
acattccaaa actgcccaaa attgcaaaca ttgtatgcga ccttagttct ccccgttgag 540
gcagccttta aaatggaaag cactcacccg aacatataca gcctcaaata cttcggagat 600
ggtttccagt atataccagg caaccatggt ggcggggcat accatcatga attcgctcat 660
ctacaatggc tcaaagtggg aaagatcaag tggagggacc ccaaggatag ctttctcgga 720
catctcaatt acacgactga gcaggttgag atgcacacag tgacagtaca gttgcaggaa 780
tcgttcgcgg caaaccactt gtactgcatc aggagaggag acttgctcac accggaggtg 840
cgcactttcg gccaacctga caggtacgtg attccaccac agatcttcct cccaaaagtt 900
cacaactgca agaagccgat tctcaagaaa actatgatgc agctcttctt gtatgttagg 960
acagtcaagg tcgcaaaaaa ttgtgacatt tttgccaaag tcagacaatt aattaaatca 1020
tctgacttgg acaaatactc tgctgtggaa ctggtttact tagtaagcta catggagttc 1080
cttgccgatt tacaagctac cacctgcttc tcagacacac tttctggtgg cttgctaaca 1140
aagacccttg caccggtgag ggcttggata caagagaaaa agatgcagct gtttggtctt 1200
gaggactacg cgaagttagt caaagcagtt gatttccacc cggtggattt ttctttcaaa 1260
gtggaaactt gggacttcag attccacccc ttgcaagcgt ggaaagcctt ccgaccaagg 1320
gaagtgtcgg atgtagagga aatggaaagt ttgttctcag atggggacct gcttgattgc 1380
ttcacaagaa tgccagctta tgcggtaaac gcagaggaag atttagctgc aatcaggaaa 1440
acgcccgaga tggatgtcgg tcaagaagtt aaagagcctg caggagacag aaatcaatac 1500
tcaaaccctg cagaaacttt cctcaacaag ctccacagga aacacagtag ggaggtgaaa 1560
caccaggccg caaagaaagc taaacgccta gctgaaatcc aggagtcaat gagagctgaa 1620
ggtgatgccg aaccaaatga aataagcggg acgatggggg caatacccag caacgccgaa 1680
cttcctggca cgaatgatgc cagacaagaa ctcacactcc caaccactaa acctgtccct 1740
gcaaggtggg aagatgcttc attcacagat tctagtgtgg aagaggagca ggttaaactc 1800
cttggaaaag aaaccgttga aacagcgacg caacaagtca tcgaaggact tccttggaaa 1860
cactggattc ctcaattaaa tgctgttgga ttcaaggcgc tggaaattca gagggatagg 1920
agtggaacaa tgatcatgcc catcacagaa atggtgtccg ggctggaaaa agaggacttc 1980
cctgaaggaa ctccaaaaga gttggcacga gaattgttcg ctatgaacag aagccctgcc 2040
accatccctt tggacctgct tagagccaga gactacggca gtgatgtaaa gaacaagaga 2100
attggtgcca tcacaaagac acaggcaacg agttggggcg aatacttgac aggaaagata 2160
gaaagcttaa ctgagaggaa agttgcgact tgtgtcattc atggagctgg aggttctgga 2220
aaaagtcatg ccatccagaa ggcattgaga gaaattggca agggctcgga catcactgta 2280

gtcctgccga ccaatgaact gcggctagat tggagtaaga aagtgcctaa cactgagccc 2340
tatatgttca agacctctga aaaggcgtta attgggggaa caggcagcat agtcatcttt 2400
gacgattact caaaacttcc tcccggttac atagaagcct tagtctgttt ctactctaaa 2460
atcaagctaa tcattctaac aggagatagc agacaaagcg tctaccatga aactgctgag 2520
gacgcctcca tcaggcattt gggaccagca acagagtact tctcaaaata ctgccgatac 2580
tatctcaatg ccacacaccg caacaagaaa gatcttgcga acatgcttgg tgtctacagt 2640
gagagaacgg gagtcaccga aatcagcatg agcgccgagt tcttagaagg aatcccaact 2700
ttggtaccct cggatgagaa gagaaagctg tacatgggca ccgggaggaa tgacacgttc 2760
acatacgctg gatgccaggg gctaactaag ccgaaggtac aaatagtgtt ggaccacaac 2820
acccaagtgt gtagcgcgaa tgtgatgtac acggcacttt ctagagccac cgataggatt 2880
cacttcgtga acacaagtgc aaattcctct gccttctggg aaaagttgga cagcacccct 2940
tacctcaaga ctttcctatc agtggtgaga gaacaagcac tcagggagta cgagccggca 3000
gaggcagagc caattcaaga gcctgagccc cagacacaca tgtgtgtcga gaatgaggag 3060
tccgtgctag aagagtacaa agaggaactc ttggaaaagt ttgacagaga gatccactct 3120
gaatcccatg gtcattcaaa ctgtgtccaa actgaagaca caaccattca gttgttttcg 3180
catcaacaag caaaagatga gactctcctc tgggcgacta tagatgcgcg gctcaagacc 3240
agcaatcaag aaacaaactt ccgagaattc ctgagcaaga aggacattgg ggacgttctg 3300
tttttaaact accaaaaagc tatgggttta cccaaagagc gtattccttt ttcccaagag 3360
gtctgggaag cttgtgccca cgaagtacaa agcaagtacc tcagcaagtc aaagtgcaac 3420
ttgatcaatg ggactgtgag acagagccca gacttcgatg aaaataagat tatggtattc 3480
ctcaagtcgc agtgggtcac aaaggtggaa aaactaggtc tacccaagat taagccaggt 3540
caaaccatag cagcctttta ccagcagact gtgatgcttt ttggaactat ggctaggtac 3600
atgcgatggt tcagacaggc tttccagcca aaagaagtct tcataaactg tgagacgacg 3660
ccagatgaca tgtctgcatg ggccttgaac aactggaatt tcagcagacc tagcttggct 3720
aatgactaca cagctttcga ccagtctcag gatggagcca tgttgcaatt tgaggtgctc 3780
aaagccaaac accactgcat accagaggaa atcattcagg catacataga tattaagact 3840
aatgcacaga ttttcctagg cacgttatca attatgcgcc tgactggtga aggtcccact 3900
tttgatgcaa acactgagtg caacatagct tacacccata caaagtttga catcccagcc 3960
ggaactgctc aagtttatgc aggagacgac tccgcactgg actgtgttcc agaagtgaag 4020
catagtttcc acaggcttga ggacaaatta ctcctaaagt caaagcctgt aatcacgcag 4080
caaaagaagg gcagttggcc tgagttttgt ggttggctga tcacaccaaa aggggtgatg 4140
aaagacccaa ttaagctcca tgttagctta aaattggctg aagctaaggg tgaactcaag 4200

```
aaatgtcaag attcctatga aattgatctg agttatgcct atgaccacaa ggactctctg     4260
catgacttgt tcgatgagaa acagtgtcag gcacacacac tcacttgcag aacactaatc     4320
aagtcaggga gaggcactgt ctcactttcc cgcctcagaa actttcttta a              4371
```